# EUROPEAN PATENT SPECIFICATION

(11) **EP 2 244 701 B1**
(45) Date of publication and mention of the grant of the patent: **09.05.2012**
(21) Application number: 09705986.9
(22) Date of filing: 01.02.2009
(51) Int. Cl.: A61K 31/409, A61K 31/555, C07D 487/22, A61P 25/28

(54) **CORROLES FOR NEUROPROTECTION AND NEURORESCUE**
CORROLE FÜR NEUROPROTEKTION UND NEURO-RESCUE
CORROLES POUR LA NEUROPROTECTION ET LA SAUVEGARDE NEUROLOGIQUE

(30) Priority: 31.01.2008 US 25043
(43) Date of publication of application: 03.11.2010
(73) Proprietor: Technion Research and Development Foundation, Ltd., 32000 Haifa (IL)
(72) Inventor: GROSS, Zeev, 49213 Petach Tikva (IL); OKUN, Zoya, 36832 Nesher (IL); MAHAMMED, Atif, 30017 Muawya Village (IL); MANDEL, Silvia, 34403 Haifa (IL); YOUDIM, Moussa B.H., 32763 Haifa (IL); AMIT, Tamar, 34678 Haifa (IL); BAR-AM, Orit, 36073 Kyriat Tivon (IL); KUPERSHMIDT, Lana, 27053 Kyriat Bialik (IL); SALTSMAN, Irena, 33071 Haifa (IL)
(74) Representative: Lederer, Franz
(86) International application number: PCT/IL2009/000121
(87) International publication number: WO 2009/095923

(56) References cited:
- WO-A1-03/004021
- MAHAMMED A ET AL: "Albumin-Conjugated Corrole Metal Complexes: Extremely Simple Yet Very Efficient Biomimetic Oxidation Systems" JOURNAL OF THE AMERICAN CHEMICAL SOCIETY, AMERICAN CHEMICAL SOCIETY, NEW YORK, USA, vol. 127, no. 9, 1 January 2005 (2005-01-01), pages 2883-2887, XP008115839 ISSN: 0002-7863 [retrieved on 2005-02-11]

## Description

### FIELD OF THE INVENTION

The present invention relates to neuroprotection and neurorescue and, particularly, to transition metal complexes of corroles for use as neuroprotective and neurorescuing agents for use in treatment of diabetes and neurodegenerative diseases and disorders, and to some new metal complexes of corroles.

### BACKGROUND OF THE INVENTION

Research into the causes of neurodegenerative disorders such as Parkinson's disease (PD), Alzheimer's disease (AD), Huntington disease (HD) and amyotrophic lateral sclerosis (ALS, a motor neuron disease), as well as of diabetes, have been fueled in part by frustration over the shortcomings of the drugs available for treatment. The true aim of therapy must ultimately be to identify the disease process before symptoms are recognized and to develop new drugs to prevent the progress of cell death. Novel therapeutic strategies for neurodegenerative diseases are clearly needed that should focus on either neuroprotection or neurorestoration as to slow down the death process or cause neurorestoration via neurogenesis, respectively. Neuropathological studies converge into a common concept, viewing neurodegenerative diseases as multifactorial, whereby several mechanisms are implicated in a cascade of events involving many biochemical and signaling pathways.

Ongoing research has clearly indicated that therapies aimed at blocking oxidative processes involving reactive oxygen species (ROS), nitric oxide (NO) production, diet supplied antioxidant polyphenols, antiapototic drugs, such as calcium channel and caspases inhibitors, bioenergetic drugs inhibitors of glutamate transmission and iron chelators would be a prominent approach in monotherapy or as part of antioxidant cocktail formulation for the treatment of these diseases (Youdim et al., 2005; Mandel et al., 2007a). The rationale is that the simultaneous manipulation of multiple desired targets in the central nervous system (CNS) will exert higher therapeutic effectiveness (Mandel et al., 2007b). Iron accumulation and deposition in the brain can cause a vast range of disorders of the CNS including PD, AD, Lewy body disease, HD, ALS, multiple sclerosis (MS), aceruloplasminemia, Friedreich's ataxia, and neurodegeneration with brain iron accumulation. Furthermore, iron is known to accumulate in the brain as a function of age, in a cell-specific manner, particularly in brain regions that are susceptible to neuron damage. It is well established that iron participates in the Fenton chemistry, reacting with hydrogen peroxide (H₂O₂) to produce the most reactive of all ROS, the hydroxyl radical. The formation of the latter, combined with the depletion of endogenous antioxidants, particularly tissue reduced glutathione (GSH), the most common pathway of iron deposition in the brain, leads to oxidative stress (OS). Iron also facilitates the decomposition of lipid peroxides to produce highly cytotoxic oxygen-related free radicals. Free radicals-related OS causes damage to DNA, lipids, proteins and ultimately cell death associated with neurodegenerative diseases.

It is widely accepted that ROS participate in the development and progression of Diabetes Mellitus (DM) and its complications. Beta cells have a reduced capacity to scavenge free radicals and are very sensitive to ROS and reactive nitrogen species (RNS) action because of their poor antioxidant system (low levels of GSH and of the H₂O₂ decomposing enzyme catalase). These facts are believed to be responsible for the high sensitivity of insulin producing cells to various insults leading to their destruction and resulting in diabetes.

Intriguingly, several lines of evidence indicate that the developmental causes of PD, AD and DM share common etiologies, particularly regarding their common neuroendocrine origin and the role of oxidative/nitrosative stress in the process of cell death. Thus, it can be speculated that neurodegenerative diseases and DM, can be prevented, and/or the damaging effects of ROS reduced, by administration of free radical scavengers and antioxidant compounds, as part of a polypharmacology approach or as a multimodal acting cytoprotective drug intended to treat the several etiologies of these diseases.

In addition to ROS, prominent role of RNS in the early stages of numerous diseases is indicated, even though the final outcome in terms of the specific malfunction or illness differs substantially in each case (Shah et al., 2007). Accordingly, endogenous and exogenous (diet-derived) antioxidants are expected to play a major role in preventing oxidative/nitrosative damage to vital biomolecules, but many compounds that display potent anti-oxidant properties *in vitro* fail in displaying beneficial *in vivo* effects. One clue for a possible resolution of this apparent conflict comes from increasing evidence indicating a crucial role for *nitrated* protein residues (mainly nitrotyrosine) in all these diseases (Mohiuddin et al., 2006). This suggests that excessive RNS are not neutralized by the natural anti-oxidants as efficiently as the ROS.

One particularly detrimental species is peroxynitrite (HOONO) (PN), formed via the ultra-fast interaction between superoxide anion (O₂⁻) and NO, whose homolytic cleavage leads to hydroxyl radical (or CO₃⁻ in CO₂-rich environment) and ·NO₂, the most reactive ROS and RNS (Goldstein et al., 2005). These species (and secondary radicals derived from them) are considered to be involved in the damage of a very large variety of molecules (Szabo et al., 2007). In contrast to other ROS and RNS precursors, peroxynitrite (PN) is particularly toxic, since there is no intrinsic biological defense system against it; and all natural catalytic anti-oxidants (including SOD and catalase enzymes) do not react with PN faster than the vital biomolecules (Olmos et al., 2007). This calls for the development of synthetic reagents that could act on and neutralize PN by one or more of the following ways: a) interfering with its formation by eliminating its precursors (O₂⁻ and NO); b) rapidly decomposing it to biologically benign products; c) repairing the damage caused by it. The major advances in this field are with metalloporphyrin-based decomposition catalysts of peroxynitrite, which appear to be efficient for treating certain diseases that are related to oxidative/nitrosative stress (Wu et al., 2003; Liang et al., 2007).

A note of caution must, however, be taken when translating *in vitro* findings into any particular *in vivo* system (Schlieve et al, 2006). For example, the prominent anti-cancer drug cisplatin becomes therapeutically inactive very fast because of its strong binding to serum albumin. This kind of limitation is even more severe for treating neurodegenerative diseases because the potential drug must be able to cross the blood-brain-barrier (BBB).

Corroles are tetrapyrrole macrocycles closely related to porphyrins, with one carbon atom less in the outer periphery and one NH proton more in their inner core. The corroles are much less known than porphyrins and their synthesis was considered to be very complex, until a simple procedure for corrole synthesis has been disclosed in US 6,541,628.

US 6,730,666 discloses porphyrins and corroles useful for inhibition of cell proliferation mediated by growth factor receptor tyrosine kinase activity, for example, for inhibition of angiogenesis, or vascular smooth muscle cell proliferation in disorders including atherosclerosis, hyperthrophic heart failure and postsurfical restenosis, and of cell proliferation and migration in the treatment of primary tumors and metastasis. The corroles defined in this patent are metal-free and positively charged and the sole corrole disclosed therein, 5,10,15-tris[2,3,5,6-tetrafluorophenyl-4-(N-methyl-2-pyridylium)]-21H,23H-corrole triiodide, was shown to inhibit the appearance of lung metastasis in an animal model.

Corroles can chelate a variety of metal ions and, once bound, the mettalocorroles are much less prone to dissociation of potentially toxic metal than analogous metalloporphyrins. Co-inventors in the present application have demonstrated in two recent publications demonstrated that iron(III) and manganese(III) complexes of 5,10,15-tris(pentafluorophenyl)-2,17-bis(sulfonic acid)-corrole (disclosed in US 6,939,963) are excellent catalysts for decomposition of two important reactive molecules, hydrogen peroxide and peroxynitrite (Mahammed et al., 2005; Mahammed and Gross, 2006, Aviv et al., 2007). Firm evidence in favor of a disproportionation mechanism were provided for both H₂O₂ and peroxynitrite (HOONO): they first serve as oxidants for transferring the manganese(III) corrole into the (oxo)manganese(V) complex, which then utilizes the same molecules as reductants for returning to manganese(III). Less detailed mechanistic insight was obtained for the iron complex, but its catalytic rates were found to be faster than those of the Mn complex and it apparently induced isomerization rather than disproportionation of peroxynitrite. The very fast action of the iron complex and the unique mechanism adopted by the manganese corrole (no other manganese complexes are capable of decomposing ROS and RNS catalytically in the absence of co-reductants, Gershman et al., 2007) suggest a significant added value of these complexes in the continuous efforts devoted to the development of synthetic catalysts that may either neutralize or avoid the formation of reactive oxygen and nitrogen species.

### SUMMARY OF THE INVENTION

In one aspect, the present invention relates to a transition metal complex of an amphiphilic corrole, an optical isomer or a pharmaceutically acceptable salt thereof, for use in neuroprotection and neurorescue, particularly for the treatment of diabetes or of a neurodegenerative disease, disorder or condition.

The amphiphilic corrole is preferably a 5,10,15-tris-aryl- or 5,10,15- tris-CF₃-corrole, and said transition metal complex of the amphiphilic corrole more preferably has the formula I as defined hereinafter in the specification.

The present invention further provides a propargyl-containing corrole carrying one or more radicals substituted by a propargylamino group or one or more nitrogen-containing heteroaryl radicals substituted by propargyl at the ring N atom, and pharmaceutical compositions comprising them.

The propargyl-containing corrole is preferably a transition metal complex of an amphiphilic corrole of the formula I as defined hereinafter in the specification.

### BRIEF DESCRIPTION OF THE FIGURES

The corrole metal complexes are presented in the legends of the figures below by a short abbreviation in bold as used herein in the specification. The full names of the compounds are shown in the beginning of the section Examples and their structural formulas are depicted in Schemes 1 and 2, at the end of the description, just before the References.

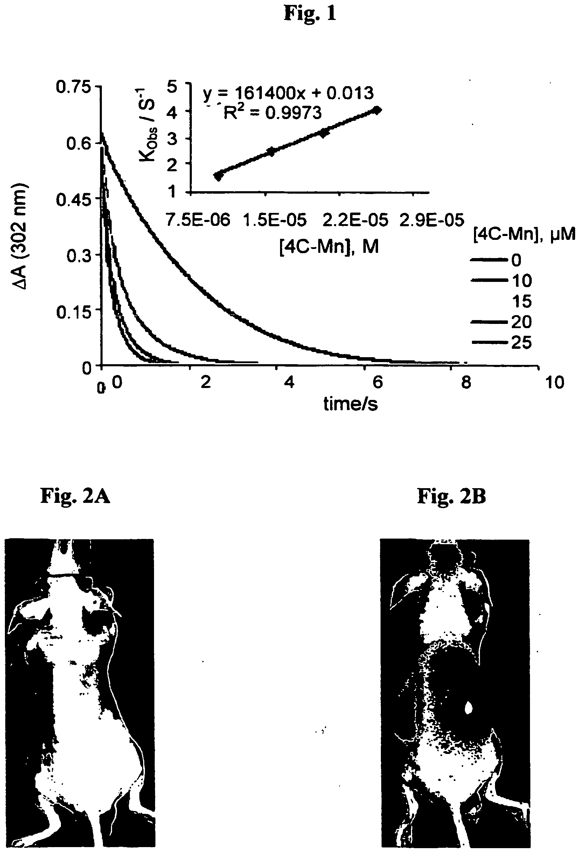

**Fig. 1** shows decomposition of 385 µm peroxynitrite at pH 7.4 and 25 °C monitored at λ = 302 nm as a function of the concentration of manganese(III) corrole **4C-Mn.** Inset: Plot used for the determination of the catalytic rate constant.
**Figs. 2A-2B** show *in-vivo* fluorescence-based imaging of a live mouse before **(****Fig. 2A****)** and 30 minutes after **(****Fig. 2B****)** intraperitoneal injection of 19 mg/kg of the gallium corrole complex **1C-Ga.** The results are indicative of the crossing of the blood brain barrier (BBB) by **1C-Ga,** the fluorescent analog of **1C-Fe** and **1C-Mn.**

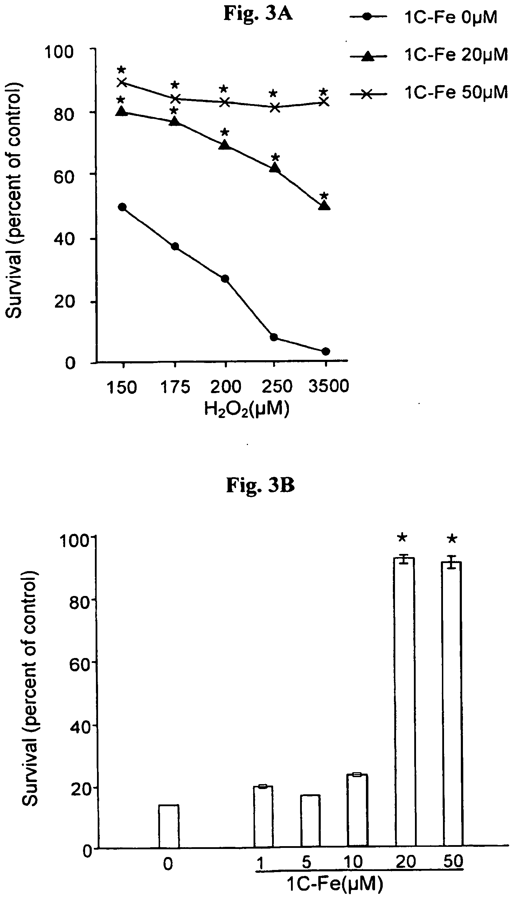

**Figs. 3A-3C** show protective effect of corrole **1C-Fe,** on clonal beta cell lines against hydrogen peroxide toxicity. **(3A)** protective effect of corrole **1C-Fe** on INS-1E cells treated with increasing hydrogen peroxide (H₂O₂) concentrations. **(3B)** dose- dependent protective effect of corrole **1C-Fe** on RIN-m cells treated with H₂O₂ (35 µM). **(3C)** corrole **1C-Fe** shows no toxicity when applied alone (without H₂O₂) at concentrations up to 50 µM. The results represent the mean ± SEM of three independent experiments performed under the same conditions. *p <0.05 versus vehicle-treated cells.

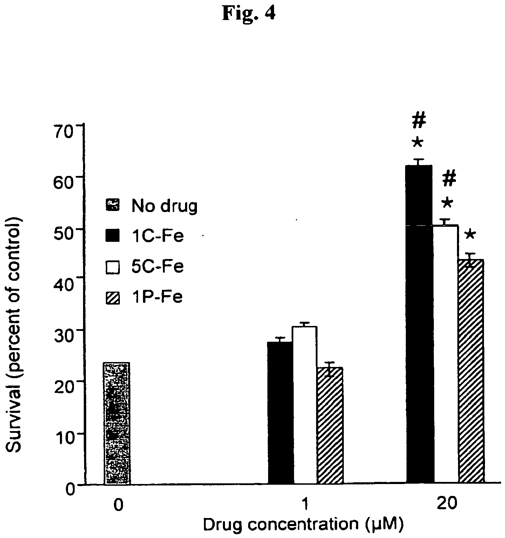

**Fig. 4** displays protective effect of iron(III) corroles **1C-Fe** and **5C-Fe** (in concentrations of 1 and 20 µM) relative to their structurally related porphyrin **1P-Fe,** against toxicity induced by H₂O₂ (35 µM) in RIN-m cells. Cell injury was evaluated by the MTT test. The results represent the mean ± SEM of a representative experiment that was repeated twice with similar results performed under the same conditions. *p <0.001 vs vehicle-treated cells, #p <0.01 vs corresponding porphyrin.

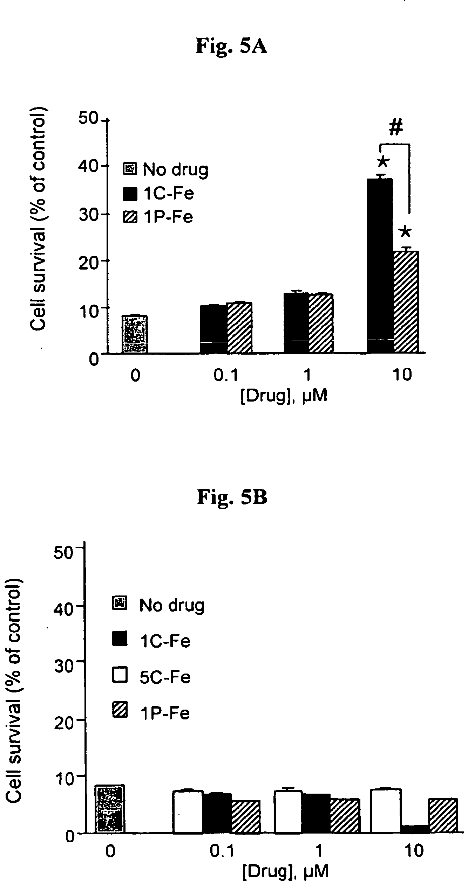

**Figs. 5A-5B** show cytoprotective effect of corroles and porphyrins on RIN-m cell line exposed to H₂O₂. **(5A)** protective effect of iron(III) corrole **1C-Fe** and structurally related porphyrin **1P-Fe. (5B)** inefficiency of manganese(III) complexes **1C-Mn, 2C-Mn** and structurally related porphyrin**2P-Mn** to protect against H₂O₂ induced damage. All concentrations are in µM. *p<0.004, #p<0.008.

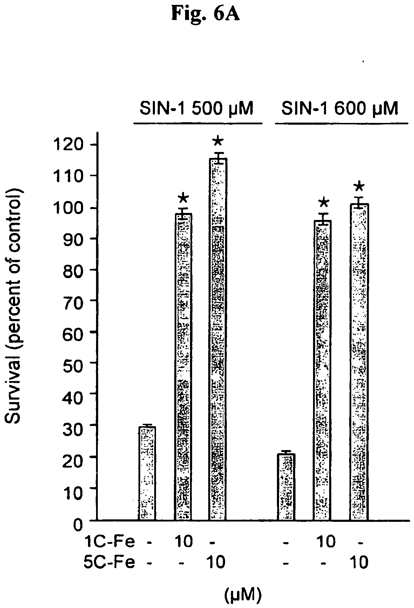

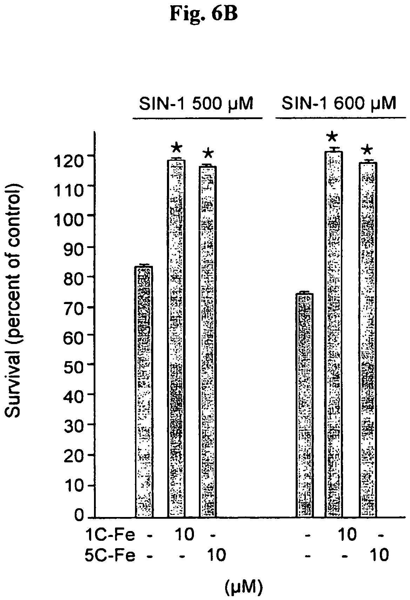

**Figs. 6A-6B** show the protective effect of iron(**III**) corroles **1C-Fe** and **5C-Fe** against toxicity induced by the nitric oxide donor linsidomine (SIN-1), in INS-1E cells and RIN-m cells, respectively. The results represent the mean ± SEM of three independent experiments performed under the same conditions. *p <0.05 versus vehicle-treated cells.

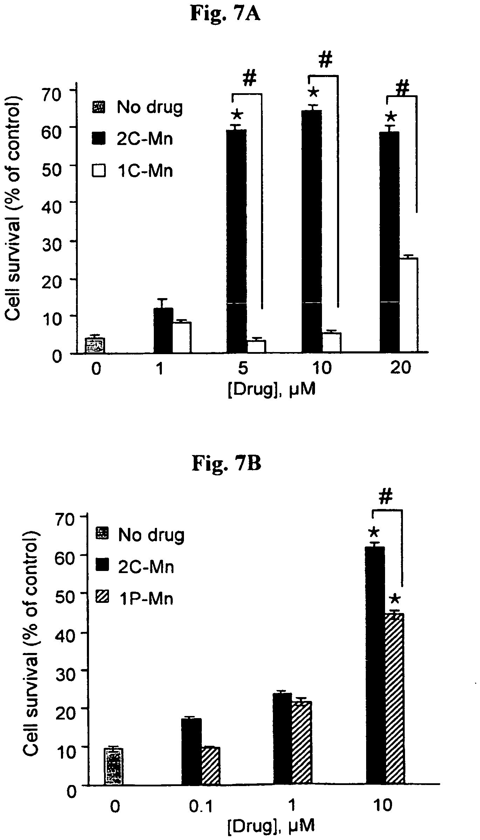

**Figs. 7A-7C** display comparative effects between **(7A)** the positively charged manganese(III) corrole **2C-Mn** and its negatively charged analog **1C-Mn; (7B)** the manganese(III) positively-charged corrole **2C-Mn** and the structurally related porphyrin **2P-Mn;** (7C) the negatively-charged iron(III) corrole **1C-Fe** and the structurally related porphyrin **1P-Fe** in RIN-m cells exposed to SIN-1. The results are the mean ± standard error; experiments were repeated twice with similar results performed under the same conditions. *p<0.005, #p<0.01

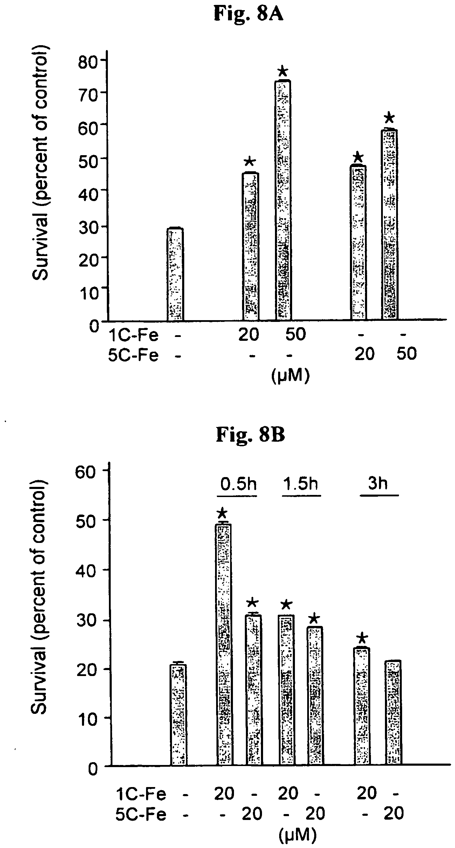

**Figs. 8A-8B** display neuroprotective and neurorescue effects of iron(III) corroles **1C-Fe** and **5C-Fe** against hydrogen peroxide-induced cell death in human neuroblastoma SH-SY5Y cell line. **(8A)** dose dependent neuroprotective effect of corroles **1C-Fe** and **5C-Fe** on SH-SY5Y cells. **(8B)** neurorescue of SH-SY5Y cells with corroles **1C-Fe** and **5C-Fe,** added 0.5, 1.5 and 3 hours after insult with H₂O₂. *p <0.01 vs. untreated cells.

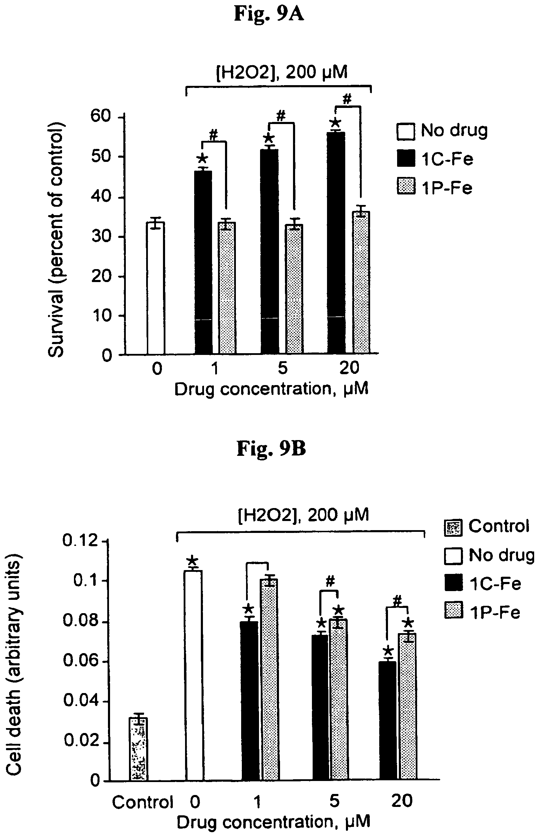

**Figs. 9A-9D** show comparative effects of iron(III) corrole **1C-Fe** versus structurally related porphyrin **1P-Fe** in SH-SY5Y cells exposed to H₂O₂. compounds **1C-Fe** and **1P-Fe** (1-50 µM) were added 30 min before insult with H₂O₂ (200 µM) for a subsequent 24 h period. **(9A)** cell viability was evaluated by MTT test, and **(9B)** cell death was assessed using the apoptotic cell death detection ELISA kit. **(9C)** the drug **(1C-Fe)** shows no toxicity when given alone (without H₂O₂) at concentrations up to 50 µM. The graphs present results expressed as percentage of untreated control. Data are expressed as mean ± SEM (n=6) of a representative experiment that was repeated twice with similar results.$p <0.001 vs control; ***p** <0.001 vs vehicle -treated cells, # p<0.001 vs corresponding porphyrin. **(9D)** Neurorescue effects of corrole **1C-Fe** against H₂O₂-induced cell death in human neuroblastoma SH-SY5Y cell line. SH-SY5Y cells were plated in microtiter plates (96 wells) in DMEM-Eagle/F-12(HAM) (1:1), containing 10% FCS. SH-SY5Y cells were first exposed to 20 mM H₂O₂, followed by the addition of corrole **1C-Fe** after 0.5-1.5 h and incubated for further 24 h. *p <0.005 vs untreated cells.

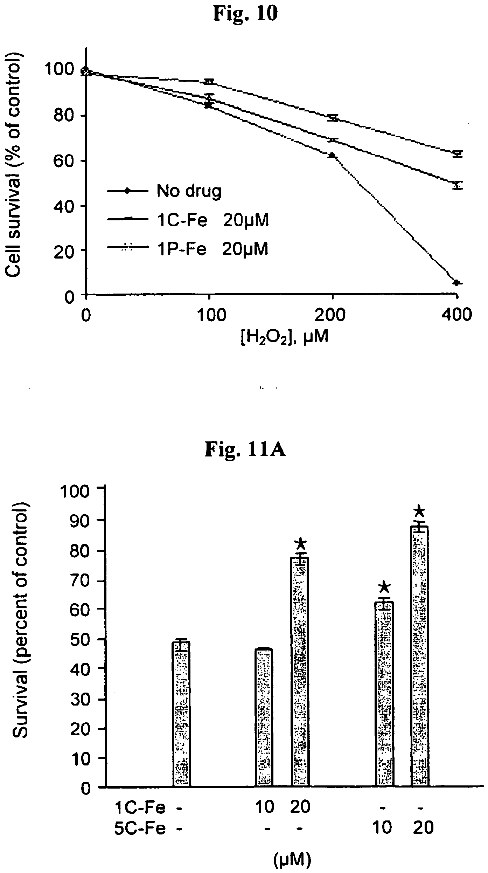

**Fig. 10** shows comparative effect of iron(III) corrole **1C-Fe** and porphyrin **1P-Fe** complexes in SH-SY5Y cells exposed to different H₂O₂ concentrations.

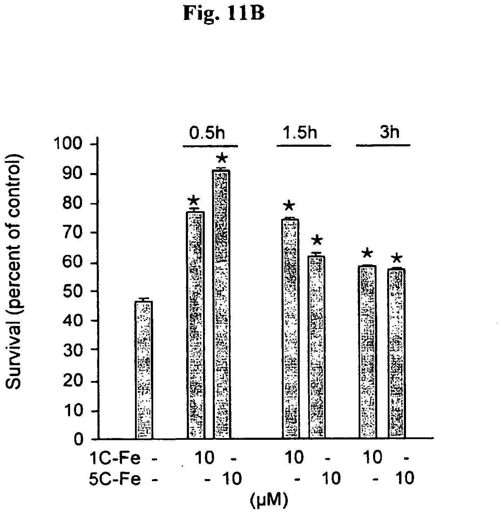

**Figs. 11A-11B** display neuroprotective and neurorescue effects of corroles **1C-Fe** and **5C-Fe** against SIN-1-induced cell death in SH-SYSY cell line. **(11A)** a dose dependent neuroprotective effect of corroles **1C-Fe** and **5C-Fe** on SH-SY5Y cells. **(11B)** neurorescue of SH-SY5Y cells with corroles **IC-Fe** and **5C-Fe** added at the indicated time after the insult with SIN-1. *p <0.01 vs. untreated cells.

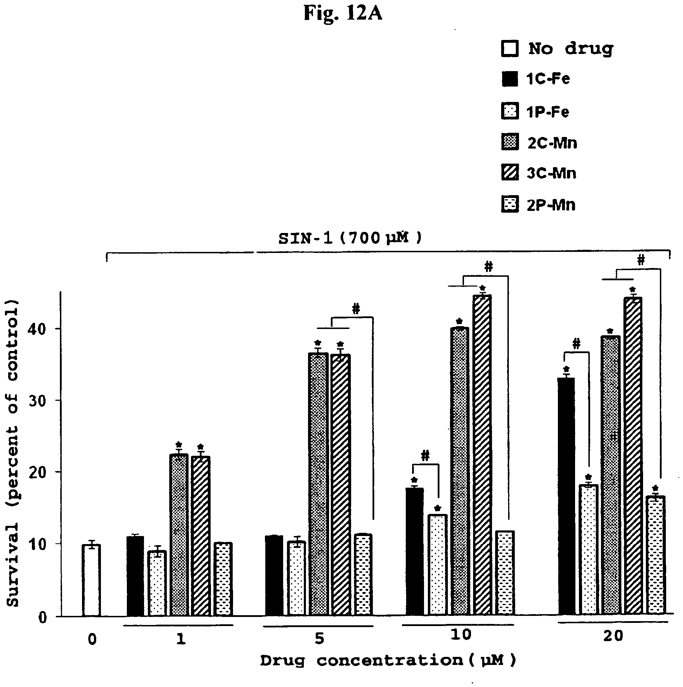

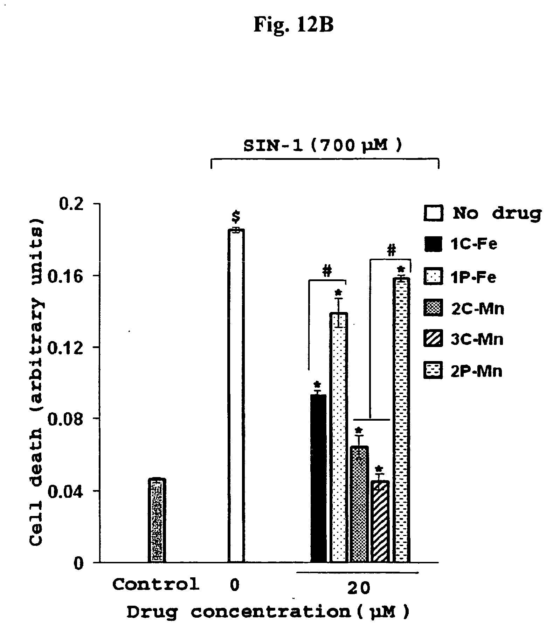

**Figs. 12A-12C** show comparative effects of corroles **1C-Fe, 2C-Mn** and **3C-Mn,** and porphyrins **1P-Fe** and **2P-Mn,** against SIN-1-induced toxicity in SH-SY5Y cells. SH-SY5Y cells were pretreated with or without one of the complexes 30 min before exposure to SIN-1 and incubated for a subsequent 24 h period. **(12A)** cell viability was evaluated by MTT test; and **(12B)** cell death was assessed using the apoptotic cell death detection ELISA kit. The graphs present results expressed as percentage of untreated control. Data are expressed as mean ± SEM (n=6) of a representative experiment that was repeated twice with similar results. $p <0.001 vs control; *p<0.001 vs. vehicle-treated cells; # p<0.001 vs. corresponding porphyrin. **(12C)** cytoprotection by iron(III) corrole **1C-Fe** and manganese(III) corrole **3C-Mn** in SH-SY5Y cells exposure to different SIN-1 concentrations. *p<0.005, #p<0.005

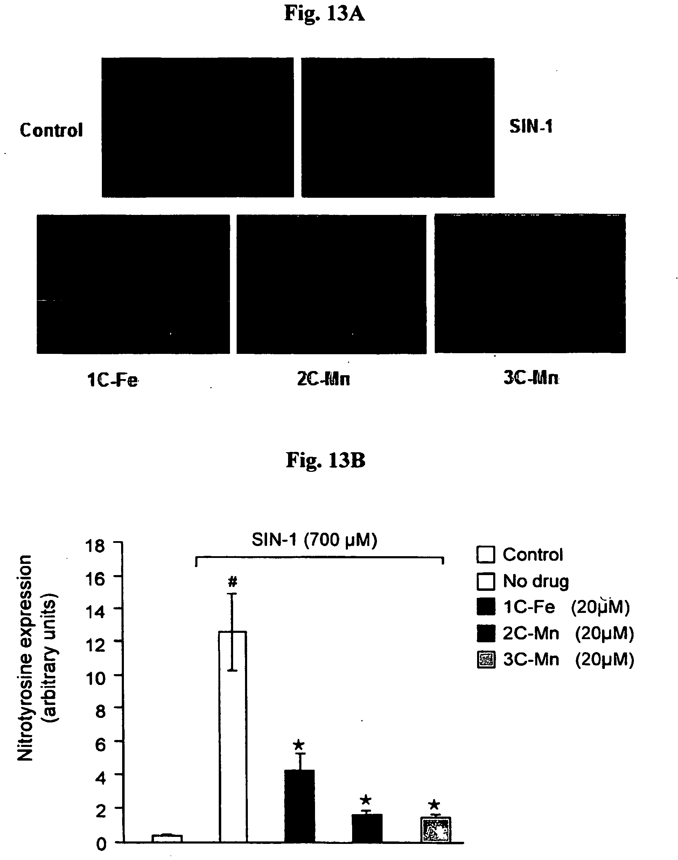

**Figs. 13A-13B** show effects of corroles **1C-Fe, 2C-Mn** and **3C-Mn** on nitrotyrosine level in SIN-1-induced neurotoxicity model. SH-SY5Y cells were pretreated with or without the corroles (20 µM) for 30 min before exposure to SIN-1 (700 µM) and analyzed after a subsequent 24 h period. Nitrotyrosine was detected by fluorescence microscopy using a specific primary antibody. **(13A)** The images show representative fields obtained by the combination of DAPI nuclear staining (reflected in blue color) and nitrotyrosine-antibody (reflected in red color). **(13B)** graph that represents absolute values of six to eight separate fields expressed as mean ± SEM. #p <0.001 vs control; *p <0.001 vs vehicle -treated cells.

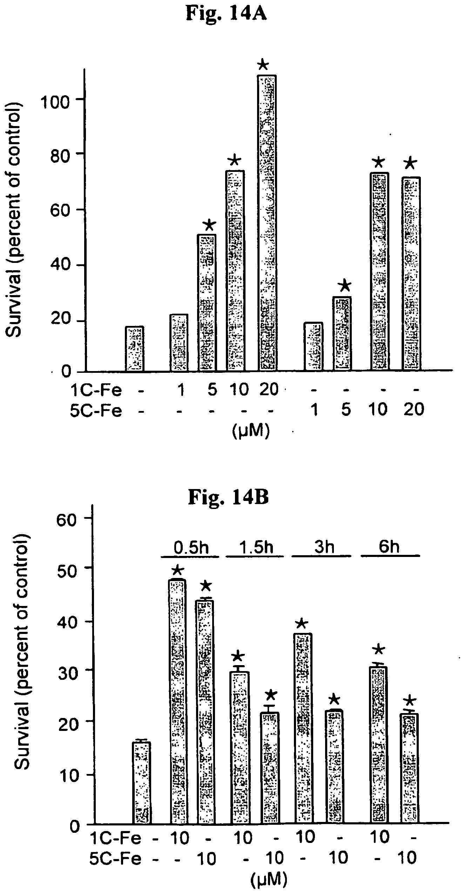

**Figs. 14A-14B** display neuroprotective and neurorescue effects of iron(III) corroles 1C-Fe and **5C-Fe** against 6-hydroxydopamine (OHDA)-induced cell death in SH-SY5Y cell line. **(14A)** dose-dependent neuroprotective effect of corroles **1C-Fe** and **5C-Fe** on SH-SY5Y cells. **(14B)** neurorescue of SH-SY5Y cells with corroles **1C-Fe** and **5C-Fe** added 0.5, 1.5, 3 and 6 h after the insult with 6-OHDA. *p <0.01 vs. untreated cells.

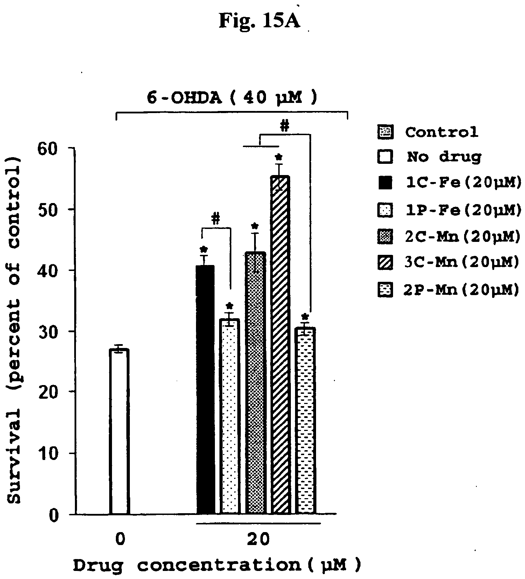

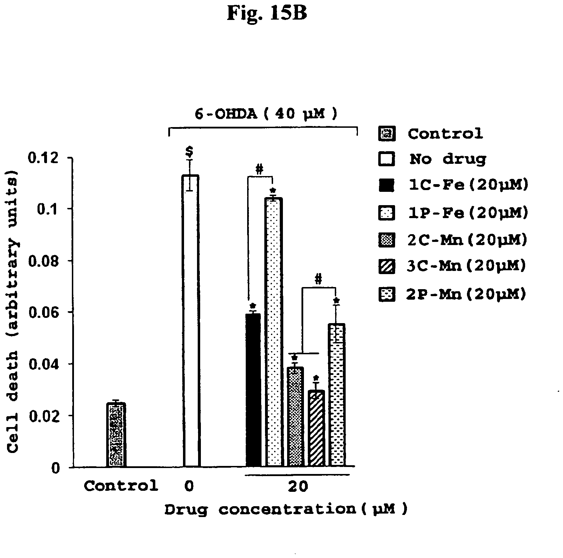

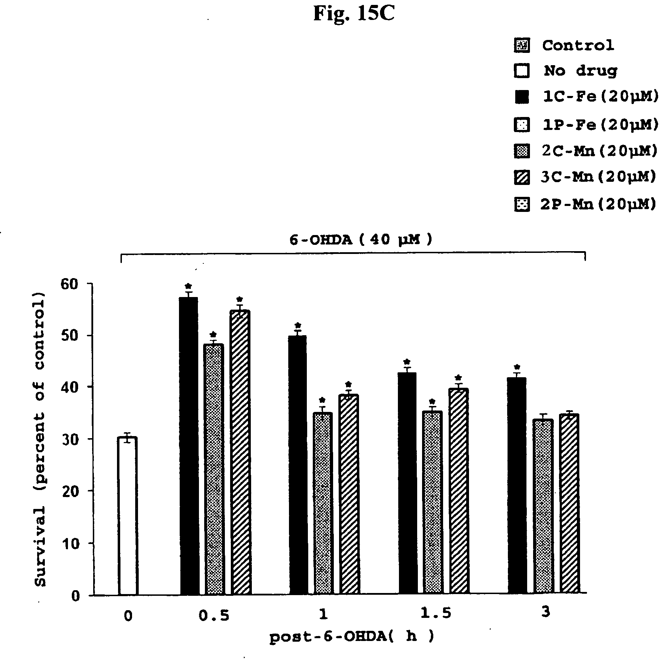

**Figs. 15A-15C** show comparative effects of corroles **1C-Fe, 2C-Mn** and **3C-Mn** and porphyrins **1P-Fe, 2P-Mn** against 6-OHDA-induced toxicity. SH-SY5Y cells were pretreated with or without the complexes (20 µM) 30 min before exposure to 6-OHDA (40 µM) for a subsequent 24 h period. **(15A)** cell viability was evaluated by MTT test and **(15B)** cell death was assessed using the apoptotic cell death detection ELISA kit. **(15C)** for neurorescue, SH-SY5Y cells were first exposed to 40 µM 6-OHDA for 0.5-3 h, followed by the addition of the corroles **1C-Fe, 2C-Mn** and **3C-Mn** (20 µM) and incubated for further 24 h. The graphs present results expressed as percentage of untreated control. Data are expressed as mean ± SEM (n=6) of a representative experiment that was repeated twice with similar results. $p <0.001 vs control;*p <0.001 vs vehicle-treated cells; # p<0.001 vs corresponding porphyrin.

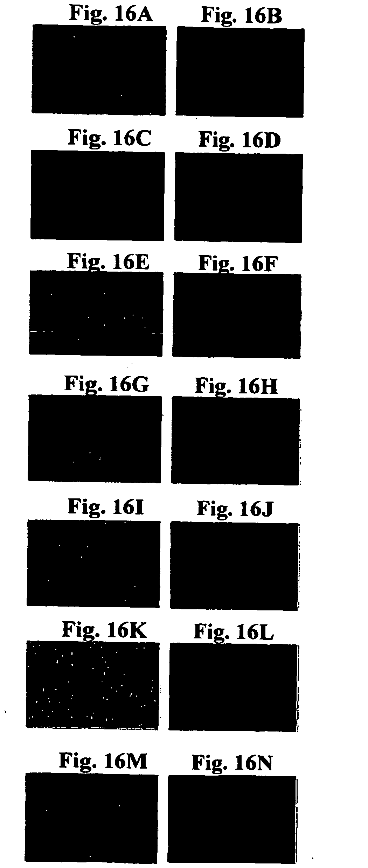

**Figs. 16 A-N** show effects of corroles **1C-Fe (E,F), 2C-Mn (I,J),** and **3C-Mn (K,L),**and porphyrins **1P-Fe (G,H)** and **2P-Mn (M,N)** against 6-OHDA-induced toxicity. SH-SY5Y cells were pretreated with or without the corroles (20 µM) 30 min before exposure to 6-OHDA (40 µM) for a subsequent 24 h period. Growth-associated protein (GAP-43) was detected by fluorescence microscopy using a specific primary antibody **(****Figs. 16A****,C,E,G,I,K** and **M** for control, 40 µM 6-OHDA, **1C-Fe, 1P-Fe, 2C-Mn, 3C-Mn** and **2P-Mn,** respectively). Apoptotic nuclei were determined by terminal deoxynucleotidyl transferase-mediated UTP-digoxigenin nick end labeling analysis and DAPI-staining was applied for nuclear labeling **(****Figs. 16B****,D,F,H,J,L** and **N** for control, 40 µM 6-OHDA, **1C-Fe, 1P-Fe, 2C-Mn, 3C-Mn** and **2P-Mn,** respectively). The images are representative fields from two independent experiments.

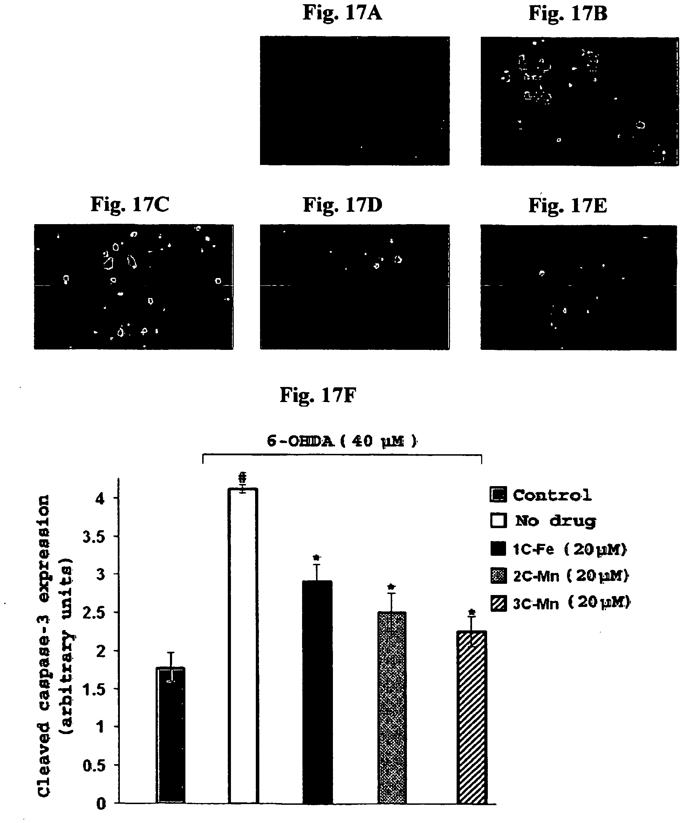

**Figs. 17A-17F** show effects of corroles **1C-Fe, 2C-Mn,** and **3C-Mn,** on cleaved caspase-3 level in 6-OHDA-induced neurotoxicity model. SH-SY5Y cells were pretreated with or without the corroles (20 µM) 30 min before exposure to 6-OHDA (40 µM) for a subsequent 24 h period. 6-OHDA was detected by fluorescence microscopy using a specific primary antibody. **(17A-E)** the images are representative fields for control, 6-OHDA (40 µM), **1C-Fe, 2C-Mn** and **3C-Mn,** respectively. **(17F)** the graph represents absolute values of six to eight separate fields expressed as mean ± SEM. #p <0.001 vs. control; *p <0.005 vs. vehicle - treated cells.

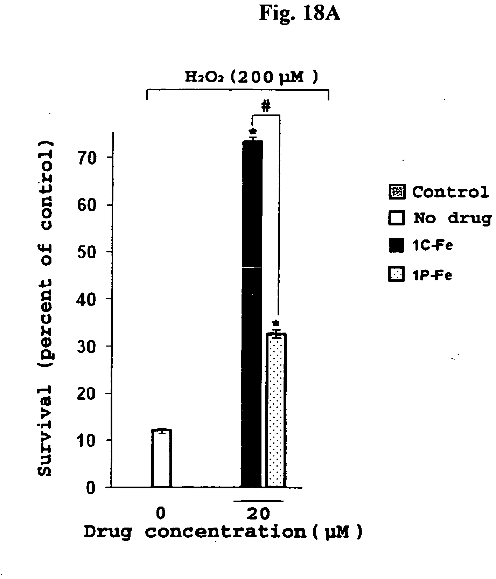

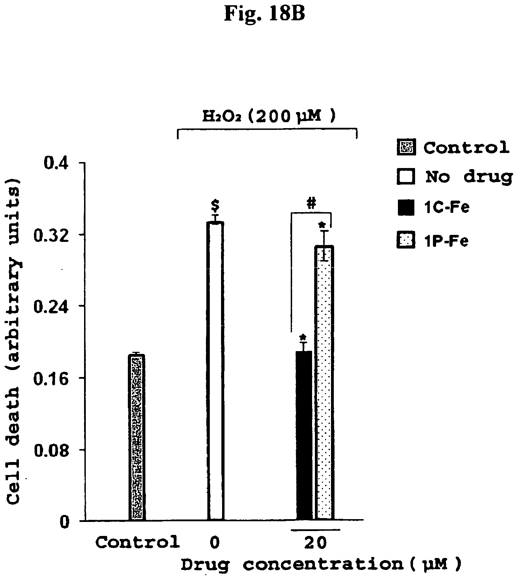

**Figs. 18A-18B** compare protective effects of corrole **1C-Fe** and porphyrin **1P-Fe** against hydrogen peroxide induced toxicity in NSC-34 cell line. Compounds **1C-Fe** and **1P-Fe** (20 µM) were added 30 min before insult with the hydrogen peroxide (300 µM) for a subsequent 24 h period. **(18A)** Cell viability was evaluated by MTT test and **(18B)** cell death was assessed using the apoptotic cell death detection ELISA kit. The graphs present results expressed as percentage of untreated control. Data are expressed as mean ± SEM (n=6) of a representative experiment that was repeated twice with similar results. $p <0.001 vs. control; *p <0.001 vs. vehicle -treated cells; # p<0.001 vs. corresponding porphyrin.

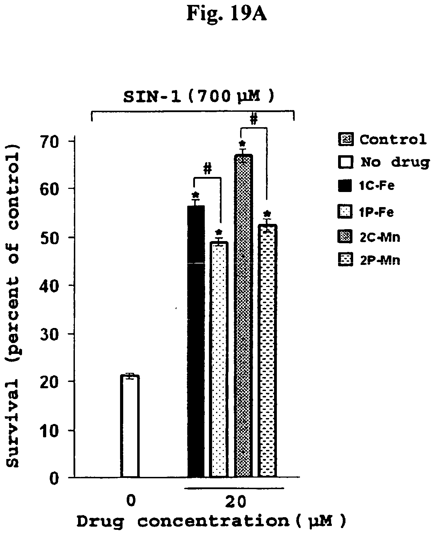

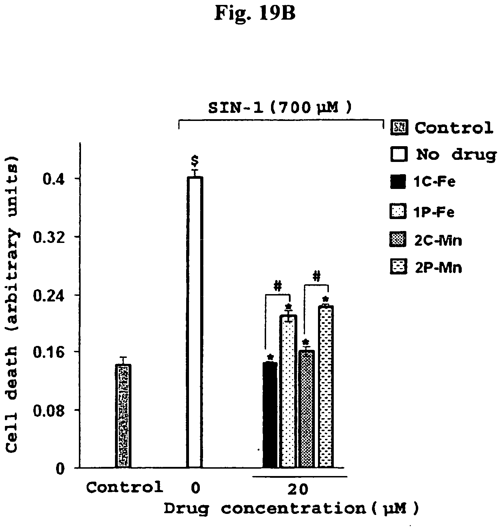

**Figs. 19A-19B** display comparative effects of corroles **1C-Fe** and **2C-Mn** and porphyrins **1P-Fe and 2P-Mn** against SIN-1 induced toxicity in NSC-34 cells. NSC-34 cells were pretreated with or without the corroles **1C-Fe** and **2C-Mn** and porphyrins **1P-Fe** and **2P-Mn,** respectively, 30 min before exposure to SIN-1 for a subsequent 24 h period. **(19A)** Cell viability was evaluated by MTT test; and **(19B)** cell death was assessed using the apoptotic cell death detection ELISA kit. The graphs present results expressed as percentage of untreated control. Data are expressed as mean ± SEM (n=6) of a representative experiment that was repeated twice with similar results. $p <0.001 vs control;*p <0.001 vs. vehicle-treated cells; # p<0.001 vs. corresponding porphyrin.

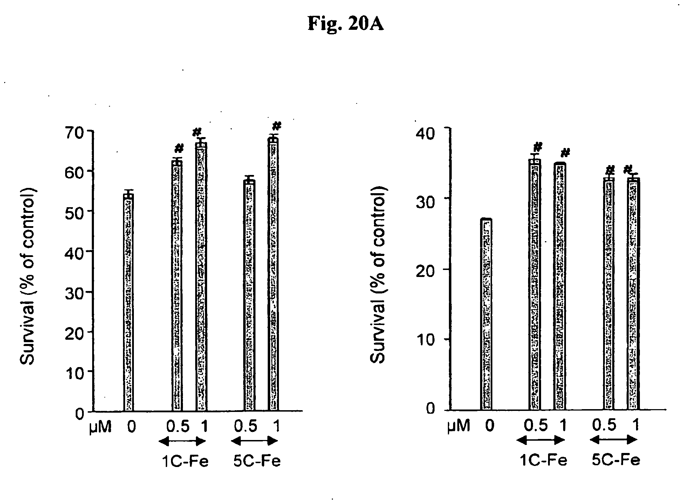

**Figs. 20A-****20B** display neuroprotective effect of corroles **1C-Fe** and **5C-Fe** in a cell culture model of familial **ALS. (20A)** dose-dependent neuroprotective effect of corroles **1C-Fe** and **5C-Fe** on mouse motoneuronal NSC-34 G93A cells expressing mutant G93A-SOD1. **(20B)** neuroprotective effect of corroles **1C-Fe** and **5C-Fe** on SH-SY5Y G93A cells expressing mutant G93A-SOD1. #p <0.001 vs cells that do not express the mutation (control).

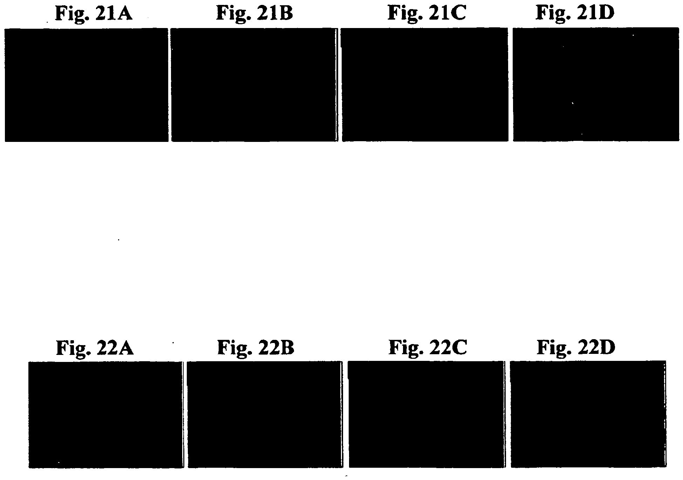

**Figs. 21A-D** shows cellular uptake of the fluorescent anionic corrole complex **1C-Ga** (20 µM) by RIN-m cell line. **(21A)** nuclei staining by DAPI, **(21B)** corrole detection by fluorescence, **(21C)** merged images of Figs. 21 A and 21B, **(21D)** same as **21B,** but in blank and white.
**Fig. 22** shows cellular uptake of the fluorescent anionic corrole complex **1C-**Ga (20 µM) by SH-SY5Y cell line. **(21A)** nuclei staining by DAPI, **(21B)** corrole detection by fluorescence, **(21C)** merged images of Figs. 21A and 21B, **(21D)** same as 21B, but in blank and white.

### DETAILED DESCRIPTION OF THE INVENTION

The present invention relates to complexes as claimed and compositions thereof for use in neuroprotection and neurorescue.

The metalated corrole for use in the invention is preferably an iron- or manganese 5,10,15-tris-aryl or 5,10,15- tris-CF₃ corrole.

In one preferred embodiment, the corrole has the formula I: 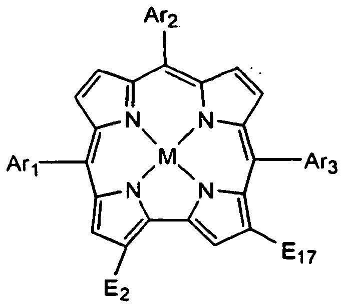 wherein:
Ar₁, Ar₂ and Ar₃, the same or different, each is selected from CF₃ or a carboaryl, heteroaryl or mixed carboaryl-heteroaryl radical;
M is a metal selected from Mn, Fe, Ru, Co, V, Cr, or Cu; and
E₂ and E₁₇, the same or different, each is H, SO₃H, SO₂N-R₁R₂, CO₂H, CO₂R or CON-R₁R₂; R is C₁-C₈ alkyl or C₆-C₁₂ aryl; and R₁ and R₂, the same or different, each is H, C₁-C₈ alkyl optionally substituted by -COOH, C₂-C₈ alkynyl, C₆-C₁₂ aryl or together with the N atom to which they are attached form a saturated 5-6 membered ring optionally containing a further heteroatom selected from O, S and N.

As defined herein, the term "carboaryl", by itself or as part of the mixed carboaryl-heteroaryl radical, refers to a monocyclic or bicyclic aromatic radical having from 6 to 12 carbon atoms, such as phenyl, biphenyl or naphthyl optionally mono- or poly-substituted by one or more halogen atoms, or by radicals including, but not limited to, C₁-C₈ alkyl, C₁-C₈ alkoxy, nitro, hydroxyl, SO₃H, -NR₁R₂, - N⁺R₁R₂R₃, or -N-R₁-NH₂, wherein R₁, R₂ and R₃, the same or different, each is H, C₁-C₈ alkyl, C₂-C₈ alkynyl, C₆-C₁₂ aryl, C₆-C₁₂aryl-C₁-C₈ alkyl or R₁ and R₂ together with the N atom to which they are attached form a saturated 5-6 membered ring optionally containing a further heteroatom selected from O, S and N. The term "aryl" herein refers to the same definitions as the carboaryl.

As used herein, the term "alkyl" alone or as part of a radical such as "aralkyl" refers to a straight or branched C₁-C₈ alkyl radical such as, but not limited to, methyl. ethyl, n-propyl, isopropyl, n-butyl, isobutyl, tert-butyl, n-heptyl, 2,2-dimethylpropyl, pentyl, n-hexyl, n-heptyl and octyl, and preferably has 1-4 carbon atoms, more preferably methyl, ethyl, and propyl. The term "alkynyl" refers to a straight or branched C₂-C₈ alkynyl radical such as, but not limited to, ethynyl, propargyl, butynyl, and the like, in which the triple bond is in the ω-position, and is preferably propargyl. The term "halogen" as used herein refers to fluoro, chloro, bromo or iodo, and is preferably chloro or fluoro, more preferably fluoro.

In one embodiment, the carboaryl radical is preferably a phenyl radical, which may be monosubstituted, for example, by a propargylamino or methoxy group, preferably at position 4, or it is polysubstituted, wherein the substituents are preferably halogen atoms, more preferably chloro or fluoro, sulfo, propargylamino, alkoxy, aminoalkylamino, and trialkylammonium. In one embodiment, the phenyl is disubstituted by chloro or fluoro. In more preferred embodiments, the phenyl is pentasubstituted, wherein the substituents are preferably 5 fluoro atoms, or 4 fluoro atoms and a substituent selected from alkoxy, propargylamino, aminoalkylamino, and trialkylammonium.

As used herein, the term "heteroaryl", by itself or as part of the mixed carboaryl-heteroaryl radical, refers to a 5-6 membered aromatic ring containing 1-3 heteroatoms selected from O, S and N such as, but not limited to, pyrrolyl, furyl, thienyl, imidazolyl, pirazolyl, oxazolyl, thiazolyl, pyridyl, pirazinyl, pyrimidinyl, 1,3,4-triazinyl, or 1,2,3-triazinyl, preferably pyridyl, which may be substituted as defined above for the carboaryl radical. When the heteroaryl has a N atom in the ring, it may be substituted at the ring N atom, preferably by an alkyl or alkynyl group as described above. In preferred embodiments, the heteroaryl radical is a N-(C₁-C₈)alkyl-pyridylium, preferably 2-, 3- or 4-(N-methyl) pyridylium, or N-propargyl- pyridylium..

The term "mixed carboaryl-heteroaryl" refers to a radical derived from a carboaryl and a heteroaryl radical condensed to each other such as. benzofuryl, isobenzofuryl, indolyl, benzimidazolyl, benzthiazolyl, benzoxazolyl, quinoline, isoquinoline and the like, or covalently linked to each other such as pyridilium-phenyl. The mixed carboaryl-heteroaryl radical may be substituted as defined above. It is to be understood that the substitutions may be in any of the carbocyclic and/or heterocyclic rings.

Examples of mixed carboaryl-heteroaryl radical that can be used according to the invention include N-(C₁-C₈)alkyl-pyridylium-tetrafluorophenyl, for example, 4-(N-methyl-2-pyridylium)- 2,3,5,6-tetrafluoro-phenyl and the corresponding 3- and 4-(N-methyl) pyridylium compounds.

The saturated 5-6 membered ring optionally containing a further heteroatom selected from O, S and N may be preferably tetrahydropyrrolyl, piperidinyl, morpholino, thiomorpholino, and piperazino, which may be further substituted at the second N atom by C₁-C₄ alkyl, hydroxyalkyl or benzyl.

In some preferred embodiments, the carboaryl is 2,6-dichlorophenyl, 2,6-difluorophenyl, 4-sulfophenyl, 4-methoxyphenyl, pentafluorophenyl, 4-methoxy-2,3,5,6-tetrafluorophenyl, 4-N-propargylamino-2,3,5,6-tetrafluorophenyl, or 4-N-propargylamino-phenyl; the heteroaryl is 4-(N-methyl)-pyridylium, 2-(N-methyl)-pyridylium, 4-(N-propargyl)-pyridylium, or 2-(N-propargyl)-pyridylium, and the carboaryl-heteroaryl is 4-(pyridyl)-2,3,5,6-tetrafluorophenyl, 4-(N-methyl-pyridylium)-2,3,5,6-tetrafluorophenyl, 4-(N-propargyl-pyridylium)-2,3,5,6-tetra-fluorophenyl, 2-(N-propargyl-pyridylium)-2,3,5,6-tetrafluorophenyl.

In some more preferred embodiments, Ar₁, Ar₂ and Ar₃ are the same and each is CF₃, 4-sulpho-phenyl, pentafluorophenyl, 4-methoxy-2,3,5,6-tetrafluoro-phenyl, 4-(N-methyl)-pyridylium, 2-(N-methyl)-pyridylium, 4-(N-propargyl)-pyridylium, or 2-(N-propargyl)-pyridylium.

In some other more preferred embodiments, Ar₁ and Ar₃ are 4-(N-methyl)-pyridylium and Ar₂ is pentafluorophenyl; or Ar₁ and Ar₃ are 4-N-propargylamino-2,3,5,6-tetrafluorophenyl and Ar₂ is 4-methoxyphenyl; or Ar₁ and Ar₃ are 4-(N-propargyl)-pyridylium and Ar₂ is pentafluorophenyl; or Ar₁ and Ar₃ are 2-(N-propargyl)-pyridylium and Ar₂ is pentafluorophenyl; or Ar₁ and Ar₃ are 2-(N-methyl)-pyridylium and Ar₂ is 4-N-propargylaminophenyl.

In some preferred embodiments, E₂ and E₁₇ are the same and are H, SO₃H, SO₂NH-propargyl or SO₂NH-CH₂-COOH.

The present invention further provides new corroles carrying one or more radicals substituted by a propargylamino group or one or more nitrogen-containing heteroaryl radicals substituted by propargyl at the ring N atom.

In particular, the invention relates to metal-chelated corroles of the formula I above carrying a propargyl group, wherein either: (i) at least one of Ar₁, Ar₂ and Ar₃ is a carboaryl radical substituted by a -NR₁R2 group, wherein R1 is H and R2 is propargyl, or a N-heteroaryl radical substituted at the ring N atom by propargyl; or (ii) at least one of E2 and E17 is -CONR1R2 or -SO2N-R1R2, wherein R1 is H and R2 is propargyl.

In some preferred embodiments, in the new corroles of formula I, E₂ and E₁₇ each is SO₂N-R₁R₂, wherein R₁ is H and R₂ is propargyl, or at position 5, 10 and/or 15, Ar₁, Ar₂ and/or Ar₃ is a phenyl radical solely substituted by a propargylamino group, preferably at position 4, or by a propargylamino group and other substituents such as halogen, preferably fluoro, or Ar₁, Ar₂ and/or Ar₃ is a pyridyl radical substituted by propargyl at the ring N atom.

Examples of propargyl-containing groups according to the invention include, but are not limited to, 4-(N-propargyl)-pyridylium, 2-(N-propargyl)-pyridylium, 4-N-propargylaminophenyl and 4-N-propargylamino-2,3,5,6-tetrafluorophenyl at positions 5, 10 and/ or 15 of the corrole or -SO₂-NH-propargyl at positions 2 and 17 of the corrole.

In preferred embodiments, the propargyl-containing corrole is metalated and the central atom is more preferably Fe or Mn.

In some preferred embodiments, the corrole for use in the present invention is selected from:
(i) the corroles in which E₂ and E₁₇ are both SO₃H, Ar₁, Ar₂ and Ar₃ each is pentafluorophenyl, and M is Fe (herein designated corrole **1C-Fe),** Mn (herein designated corrole **1C- Mn),** or Cu (herein designated corrole **1C-Cu);** or Ar₁, Ar₂ and Ar₃ each is CF₃ and M is Fe or Mn;
(ii) the corrole in which E₂ and E₁₇ are both SO₃H, Ar₁, Ar₂ and Ar₃ each is 4-methoxy-2,3,5,6,-tetrafluorophenyl, and M is Fe (herein designated corrole **6C-Fe);**
(iii) the corrole in which E₂ and E₁₇ are both SO₂NH-CH₂-COOH, Ar₁, Ar₂ and Ar₃ each is pentafluorophenyl, and M is Fe (herein designated corrole **5C-Fe)**;
(iv) the corrole in which E₂ and E₁₇ are both H, Ar₁ and Ar₃ each is 4-(N-methyl)-pyridylium, Ar₂ is pentafluorophenyl, and M is Mn (herein designated corrole **2C-Mn**);
(v) the corrole in which E₂ and E₁₇ are both H, Ar₁, Ar₂ and Ar₃ each is 2-(N-methyl)-pyridylium, and M is Mn (herein designated corrole **3C-Mn);**
(vi) the corrole in which E₂ and E₁₇ are both H, Ar₁ and Ar₃ each is 2-(N-methyl)-pyridylium, Ar₂ is pentafluorophenyl, and M is Mn (herein designated corrole **4C-Mn);** and
(vii) the corroles in which E₂ and E₁₇ are both SO₃H, Ar₁, Ar₂ and Ar₃ each is CF₃, and M is Mn or Fe (herein designated corrole **M-Mn** or **H-Fe,** respectively).,
(viii) the corroles in which E₂ and E₁₇ are both H, Ar₁ and Ar₃ each is 4-(N-propargyl)-pyridylium, Ar₂ is pentafluorophenyl and M is Mn or Fe (herein designated corrole **E-pr-Mn** or **E-pr-Fe,** respectively);
(ix) the corrole in which E₂ and E₁₇ are both H, Ar₁ and Ar₃ each is 2-(N-propargyl)-pyridylium, Ar₂ is pentafluorophenyl, and M is Mn or Fe (herein designated corrole **H-Mn** or **H-Fe,** respectively);
(x) the corroles in which E₂ and E₁₇ are both SO₃H, Ar₁ and Ar₃ each is 4-N-propargylamino-2,3,5,6-tetrafluorophenyl and Ar₂ is 4-methoxyphenyl, and M is Mn or Fe (herein designated corrole **I-Mn** or **I-Fe,** respectively);
(xi) the corrole in which E₂ and E₁₇ are both H, Ar₁ and Ar₃ each is 2-(N-methyl)-pyridylium, Ar₂ is 4-propargylamino-phenyl, and M is Mn or Fe (herein designated corrole **J-Mn** or **J-Fe,** respectively);
(xii) the corroles in which E₂ and E₁₇ are both -SO₂-NH-propargyl, Ar₁, Ar₂ and Ar₃ each is pentafluorophenyl, and M is Mn or Fe (herein designated corrole **K-Mn** or **K-Fe,** respectively); and
(xiii) the corroles in which E₂ and E₁₇ are both -SO₂-NH-propargyl, Ar₁, Ar₂ and Ar₃ each is CF₃, and M is Mn or Fe (herein designated corrole **M-Mn** or **M-Fe,** respectively).

The structural formulas of the above-mentioned corroles (i) - (vii) and propargyl-containing corroles (viii) - (xiii) are depicted in Schemes 1 and 2, respectively, herein at the end of the description, just before the references.

In more preferred embodiments, the corroles for use in the present invention are the corroles **1C-Fe, 1C-Mn, 2C-Mn, 3C-Mn, 4C-Mn, 5C-Fe, 5C-Fe, and E-pr-Mn.**

Some of the corroles not containing a propargyl group have been disclosed in applications WO 03/004021 and PCT/IL2008/001066 of the same applicant, which are herewith incorporated by reference as if fully disclosed herein.

Also contemplated by the present invention are pharmaceutically acceptable salts of the corrole of formula I.

Pharmaceutically acceptable salts are formed with metals or amines, such as alkali and alkaline earth metals or organic amines. Examples of metals used as cations are sodium, potassium, magnesium, calcium, and the like. Examples of suitable amines are N,N'-dibenzylethylenediamine, chloroprocaine, choline, diethanolamine, ethylenediamine, N-methylglucamine, and procaine (see, for example, Berge S. M., et al., "Pharmaceutical Salts," (1977) J. of Pharmaceutical Science, 66:1-19). The salts can also be pharmaceutically acceptable quaternary salts such as a quaternary salt of the formula -NRR'R" + Z' wherein R, R' and R" each is independently hydrogen, alkyl or benzyl and Z is a counterion, including chloride, bromide, iodide, O-alkyl, toluenesulfonate, methylsulfonate, sulfonate, phosphate, or carboxylate.

Pharmaceutically acceptable acid addition salts of the compounds include salts derived from inorganic acids such as hydrochloric, nitric, phosphoric, sulfuric, hydrobromic, hydriodic, phosphorous, and the like, as well as salts derived from organic acids such as aliphatic mono- and dicarboxylic acids, phenyl-substituted alkanoic acids, hydroxy alkanoic acids, alkanedioic acids, aromatic acids, aliphatic and aromatic sulfonic acids, etc. Such salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, trifluoromethyl sulfonate or tosylate and the like. Also contemplated are salts of amino acids such as arginate and the like and gluconate or galacturonate (see, for example, Berge S. M., et al., "Pharmaceutical Salts," (1977) J. of Pharmaceutical Science, 66:1-19).

The present invention further provides a pharmaceutical composition comprising a new propargylamino-substituted corrole, a pharmaceutically acceptable salt or an optical isomer thereof and a pharmaceutically acceptable carrier.

The invention still further relates to a pharmaceutical composition for neuroprotection and neurorescue, particularly for treatment of diabetes and neurodegenerative diseases, disorders or conditions, comprising a pharmaceutically acceptable carrier and a metal complex of an amphiphilic corrole, an optical isomer or a pharmaceutically acceptable salt thereof, preferably of formula I herein.

As used in the present specification, the term "treatment" means alleviating or ameliorating the symptoms or complications of diabetes or of the neurodegenerative disease, disorder or consition, preventing their progress or curing. The corrole according to the patient is therefore administered to the patient in an effective amount as many times as necessary to achieve one or more of these goals.

The pharmaceutical compositions of the present invention comprising metal complexes of corroles are formulated for administration to the patient using techniques well-known in the art, for example, as summarized in Remington's Pharmaceutical Sciences, Mack Publishing Co., Easton, Penna., latest edition.

In a preferred embodiment, the pharmaceutical composition for use in the present invention is administered parenterally, for example, by intravenous or intramuscular injection, or preferably orally. The doses will depend on the type of disease or disorder and condition and age of the patient and may vary between 0.1 to 10 mg/kg/day.

The corroles according to the invention, including the novel propargyl-containing corroles, are useful as neuroprotective and neurorescuing agents for treatment and/or prevention of diabetes and neurodegenerative diseases, disorders or conditions and any other disease, disorder or condition that can benefit from the neuroprotection and neurorescue activity provided by the corroles.

In one embodiment, the disease, disorder or condition that can be treated by these corroles is diabetes type II and complications thereof.

In another embodiment, the disease, disorder or condition is a neurodegenerative disease, disorder or condition including Parkinson's disease (PD), Alzheimer's disease (AD), Huntington disease (HD), amyotrophic lateral sclerosis (ALS), multiple sclerosis, Friedreich's ataxia, Hallervorden-Spatz disease, a dementia, and psychiatric diseases, disorders and conditions;

The dementia may be an AD or a non-AD dementia such as Lewy body dementia, vascular dementia and a dementia caused by Parkinson's disease, Huntington's disease, Creutzfeldt-Jakob disease, head trauma or HIV infection. The psychiatric disease, disorder or condition may be an affective or mood disorder including depression, a dysthymic disorder, a bipolar disorder, a cyclothymic disorder, schizophrenia or a schizophrenia-related disorder such as brief psychotic disorder, a schizophreniform disorder, a schizoaffective disorder and delusional disorder, or it may be drug use and dependence such as alcoholism, opiate dependence, cocaine dependence, amphetamine dependence, hallucinogen dependence, or phencyclidine use and withdrawal symptoms related thereto.

In another embodiment, the disease, disorder or condition is a memory loss disorder such as amnesia or memory loss associated with Alzheimer's type dementia, with a non-Alzheimer's type dementia, or with a disease or disorder selected from Parkinson's disease, Huntington's disease, Creutzfeldt-Jakob disease, head trauma, HIV infection, hypothyroidism and vitamin B12 deficiency;

In further embodiments, the disease, disorder or condition is an acute neurological traumatic disorder or neurotrauma such as head trauma injury or spinal cord trauma injury; a) demyelinating disease such as multiple sclerosis; a seizure disorder such as epilepsy; a cerebrovascular disorder such as brain ischemia or stroke; a behavior disorder of neurological origin that may be a hyperactive syndrome or an attention deficit disorder; and a neurotoxic injury which is caused by a neurotoxin such as a nerve gas or the toxin delivery system of poisonous snakes, fish or animals.

In some preferred embodiments, the neurodegenerative disease is Parkinson's disease, Huntington's disease, Alzheimer's disease or amyotrophic lateral sclerosis.

As shown in the examples herein, the corroles metal complexes, preferably the Fe and Mn complexes, are highly potent in protecting the activity of insulin-secreting cell lines against the toxic effects of additives that mimic naturally occurring oxidative/nitrosative stress. A comparison with structurally related metalloporphyrins reveals that the corrole derivatives are significantly superior. Even more remarkable is the observed neurorescue/neurorestorative effect in the models of neurodegenerative diseases (AD, PD and ALS) against oxidative/nitrosative stress and deprivation of neurotrophic factor support.

It is also shown herein in the examples that the corroles inhibit the monoamine oxidase enzymes MAO-A and MAO-B.

The corroles used herein display a consistent superiority in all monitored neuroprotection and neurorescue parameters. It is also shown that the corroles have a positive impact in cell lines model for diabetes and neurodegenerative diseases to retard or perhaps even reverse the accelerated rate of cytodegeneration. It is also shown that some of the corroles used herein may cross the BBB, likely due to the strong and spontaneous association to proteins that may facilitate the process.

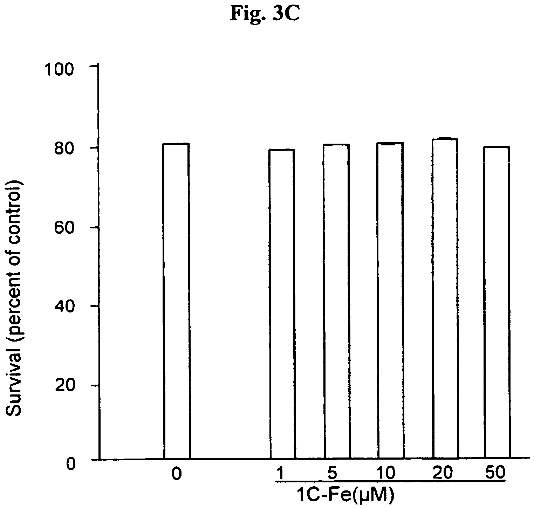

The results herein indicate that the corroles are highly potent regarding the protective activity of insulin-secreting cells against the toxic effects of additives that mimic naturally occurring oxidative/nitrosative stress **(****Figs. 3-7****).** Even more remarkable is the observed neurorescue/neurorestorative effect in the models of neurodegenerative diseases (AD, PD and ALS) against oxidative/nitrosative stress and deprivation of neurotrophic factor support **(****Figs. 8-20****).** Consistently, iron corroles **1C-Fe** and **5C-Fe** and manganese corroles **2C-Mn and 3C-Mn** displayed superiority over other corroles.

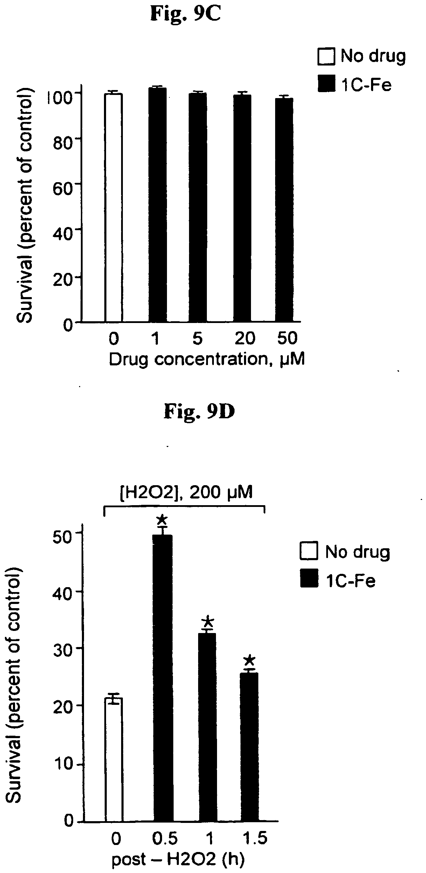

The present findings suggest that corroles **1C-Fe, 5C-Fe, 2C-Mn and 3C-Mn** may have a positive impact on diabetes, aging and neurodegenerative diseases as to retard, or perhaps, even reverse the accelerated rate of cytodegeneration. This may suggest a potential disease modifying activity. It is further important to emphasize the observation that the corrole complexes perform significantly better than structurally analogous porphyrins **(1C-Fe vs. 1P-Fe** and **2C-Mn** vs. **2P-Mn)** in many cases (see **Figs. 4****,** **7****,** **9****,** **12****,** **15** and **18****).** Notably, the iron corrole **1C-Fe** and the pyridinium-substituted manganese corrole **2C-Mn** were successful against SIN-induced toxicity in RIN-m cells, while the sulfonato-substituted manganese corrole **1C-Mn** was ineffective. This correlates very well with the results of Table 3, suggesting that the rate of detoxification by **1C-Mn** and its SOD activity are apparently too low. In addition, the observation that corroles **1C-Fe** and **5C-Fe** were able not only to protect but also to rescue neurons when administered after the oxidative damage was induced **(****Figs 8B****,** **9D****,** **11B****,** **14B** and **15C****),** suggests: a) that the compounds have the ability to enter the cells and b) that mechanisms other than a direct antioxidant effect contribute to their neuroprotective action. A detailed toxicity study has not been performed, nevertheless the corrole compounds tested so far do not display *in vitro* cytotoxicity up to 50 µM (see: **Figs. 3** and **9C****),** and mice that received 10 mg/kg amounts of corroles **1C-Fe** and **1C-Mn** for 10 weeks did not display any observable abnormal behaviour or weight loss.

The invention will now be illustrated by the following non-limiting examples.

### EXAMPLES

In the examples, the following compounds will be identified by abbreviated names as detailed below. The chemical structure of these compounds is presented in Schemes I and 2, just before the References.
**Corrole 1C-Fe:** 2,17-bis(sulfonato)-5,10,15-tris(pentafluorophenyl)-corrolato iron(III)
**Corrole 1C-Mn:** 2,17-bis(sulfonato)-5,10,15-tris(pentafluorophenyl)-corrolato manganese(III)
**Corrole 1C-Cu:** 2,17-bis(sulfonato)-5,10,15-tris(pentafluorophenyl)-corrolato copper(III)
**Corrole 1C-Ga:** 2,17-bis(sulfonato)-5,10,15-tris(pentafluorophenyl)-corrolato galium(III)
**Corrole 2C-Mn:** 5,15-bis(4-N-methyl-pyridilium)-10-pentafluorophenyl-corrolato manganese(III) diiodide salt
**Corrole 3C-Mn:** 5,10,15- tris(N-methyl-2-pyridilium) corrolato manganese(III)
**Corrole 4C-Mn:** 5,15- bis(N-methyl-2-pyridilium) 10-pentafluorophenyl corrolato manganese(III)
**Corrole 5C-Fe:** 2,17-bis(N-sulfonylglycine)-5,10,15-tris(pentafluorophenyl)-corrolato iron(III)
**Corrole 6C-|Fe:** 5,10,15 tris(4-methoxy-tetrafluorophenyl)-corrolato iron (III).
**Corrole E-pr-Mn:** 5,15-bis(4-N-propargyl-pyridilium)-10-pentafluorophenyl corrolato manganese (**III**)
**Porphyrin 1P-Fe:** 5,10,15,20-tetra (4-sulfonatophenyl)-porphyrinato iron(III)
**Porphyrin 2P-Mn:** 5,15-bis(N-methyl-4-pyridilium)-10,20-bis-(pentafluorophenyl) porphyrinato manganese(III) diiodide salt

The corroles **1C-Fe, 1C-Mn, 1C-Cu** and **1C-Ga** were prepared according to procedures previously disclosed by the inventors (WO 03/004021, US 6,939,963; Mahammed and Gross, 2006; Saltsman et al., 2002). The corrole **2C-Mn** was prepared as described previously by the inventors (Gershman et al., 2007). The corroles **3C-Mn, 4C-Mn, 5C-Fe and 6C-Fe** as well the propargyl-substituted corroles are new and their synthesis is described hereinbelow in the Examples. In the syntheses, the solvents and standard chemicals were purchased from reliable sources and used as received.

### Example 1: Catalytic decomposition of ROS/RNS by the metal corroles

SOD activity (enzyme-like catalytic decomposition of superoxide anion radical) of the corroles **1C-Fe, 6C-Fe, 1C-Mn, 1C-Cu, 2C-Mn** and **3C-Mn** was examined via the cytochrome C assay. The catalytic rate constants for decomposition of peroxynitrite (PN) by the newly prepared iron corrole **6C-Fe** and manganese corrole **3C-Mn** were determined via stopped-flow kinetics as previously described for corroles **1C-Fe, 1C-Mn,** and **2C-Mn** (Mahammed and Gross, 2006; Gershman et al., 2007). These tests are performed in order to estimate how electronic and structural variables affect reactivity, which is a crucial factor for design of new complexes.

**Table 1. SOD activity and catalytic rate constants for decomposition of PN.^{a}**

| Complex | **1C-Fe** | **6C-Fe** | **1C-Mn** | **1C-Cu** | **2C-Mn** | **3C-Mn** |
|---|---|---|---|---|---|---|
| IC₅₀ (µM) | 1.64 | 1.18 | 5.9 | 12 | 1.52 | 3.27 |
| k_{cat} (M⁻¹s⁻¹) | 2.0×10⁶ | n.d. | 4.0×10⁴ | n.d. | 4.0×10⁵ | 2.5×10⁴ |

| | | | | | | |
|---|---|---|---|---|---|---|
| n.d. = not yet determined. ^{a} Note how IC₅₀ and *k*_{cat} are affected by the kind of metal ion (1C-Fe < 1C-Mn << 1C-Cu) and by the corrole substituents in both the Mn and iron complexes. | | | | | | |

The results presented in Table 1 show that corroles display substantial SOD-like activity in the cytochrome C assay and also suggest that the IC₅₀ values may be further reduced by increasing the electron-richness of the complexes (compare 6C-**Fe** with **1C-Fe** for example). This can be done, for example, by using corroles with much more electron-donating aryl groups and determination of their electrochemical redox potential. We have already confirmed that in a series of corroles that are not water-soluble (work performed in water/DMSO mixtures), shifting of redox potentials and SOD activities are strongly correlated indeed (not shown).

The catalase-like activity (catalytic decomposition of hydrogcn peroxide into molecular oxygen and water) of the metalated corroles was also examined (by an oxygen-measuring electrode), revealing that the iron corroles decompose H₂O₂ much faster than the corresponding manganese complexes.

In all these investigations, even for very large numbers of catalytic turnovers, there was no oxidative degradation of the corrole complexes.

### Example 2. Determination of peroxynitrite decomposition rates by new manganese corroles

The investigations were performed by examining the decay of 385 µM peroxynitrite at pH 7.4 and 25 °C in the presence and absence of various amounts of Mn corrole catalysts **2C-Mn, 2C'-Mn, 2C"-Mn, 3C-Mn** and **4C-Mn.** The differences between **2C-Mn, 2C'-Mn,** and **2C"-Mn** is in the identity of the 10-aryl group, which is C₆F₅, *para-anisyl,* and phenyl, respectively. The results (one example using **4C-Mn** is shown in **Fig. 1****)** were obtained by monitoring changes in absorbance at 302 nm, the λₘₐₓ of peroxynitrite. The catalytic performances of five of the above compounds were tested; and the thus elucidated k_{cat} values are presented in Table 2. Importantly, in sharp contrast with the corroles, none of the structurally similar manganese (III) porphyrin complexes affected the decomposition of peroxynitrite by any extent (not shown).

**Table 2. Peroxynitrite decomposition rate by various manganese corroles.**

| **Complex** | **2C-Mn** | **2C'-Mn** | **2C"-Mn** | **3C-Mn** | **4C-Mn** |
|---|---|---|---|---|---|
| k_{cat}(M⁻¹s⁻¹) | 1.5×10⁵ | 7.7×10⁴ | 8.1×10⁴ | 2.5×10⁴ | 1.6×10⁵ |

### Example 3. Rescuing molecules from damage attributable to the hydroxyl radical with Fe and Mn corroles

Corrole complexes were examined with respect to their efficiency of rescuing DMSO from damage attributed to the hydroxyl radical, formed via the Fenton reaction that also involves superoxide anion radical and hydrogen peroxidedifferent ROS and/or RNS. It also mimics reaction occurring in biological systems that are exposed to metal toxicity, the situation that occurs in diseases caused by brain iron accumulation. The results summarized in Table 3 demonstrate that the iron(III) corrole **1C-Fe** is very potent in preventing the oxidative damage and that the newly prepared manganese corroles **2C-Mn** and especially **3C-Mn** apparently protect better than corrole **1C-Mn** against metal-induced oxidation of substrates.

**Table 3. Inhibitory effect of Fe and Mn corroles on oxidation/nitration of selected molecules**

| ROS/RNS, reactive species | Substrate | Product | **Yield (%) or amount of product^{a}** | | | | |
|---|---|---|---|---|---|---|---|
| | | | No additive | **1C-Fe** | **1C-Mn** | **2C-Mn** | **3C-Mn** |
| CuSO₄ / ascorbate | DMSO | Formaldehyde | 44 µM | 0 | 20 µM | 7 µM | 0 |
| CuSO₄ / glutathione | DMSO | Formaldehyde | 11 µM | 0 | 7 µM | 5 µM | 0 |

### Example 4. Biodistribution of corroles

For obtaining information about in-vivo localization and distribution of the various corroles that have sulfonic acid head groups, *in*-*vivo* imaging of corroles in whole animals was performed. Of particular interest was to examine whether the leading corrole compounds unlike most porphyrins are able to cross the blood brain barrier (BBB).

The corrole 1C-Fe is shown herein to be a neurorescue and neuroprotective compound. The corrole **1C-Ga** (Scheme 1) is a fluorescent analogue of **1C-Fe** that does not exhibit those abilities of **1C-Fe,** but can be used as a material that can be easily detected in cells and tissues due to its fluorescence. Because **1C-Fe** and **1C-Ga** differ only by the metal center, it is assumed that their biodistribution is the same.

The in vivo imaging experiments were performed as follows. Nude mice received i.p. injections of the fluorescent gallium(III) corrole **1C-Ga** and were examined by IVIS 200 imaging system [Xenogen]. The result shown in **Fig. 2B** (30 min after injection of 19 mg/kg **1C-Ga)** demonstrates possible BBB penetration and accumulation of the corrole complex in brain area.

### Example 5. Protective effect of iron corrole 1C-Fe on clonal beta cell lines exposed to hydrogen peroxide (H₂O₂) - Neurorescue/neurorestorative effects

Islets and especially β cells contain among the lowest levels of antioxidant enzyme activities compared to other tissues. These facts are believed to be responsible for the high sensitivity of insulin-producing cells to various insults, leading to destruction of β cells and consequently resulting in diabetes. This triggered our study on examining protective properties of corroles against oxidative stress in insulin-producing β cells.

Two insulin-secreting beta cell lines, rat insulinoma (INS-1E) and RIN-m cells, were used to examine the protective effect of corrole **1C-Fe**. The cells were cultured at 37°C, in a humid 5% C02, 95% air environment in regular RPMI-1640 medium (Invitrogen) supplemented with 5% fetal calf serum (FCS).

After 2 days of culture, INS-1E cells (5 × 10⁴ cells/well in 96-well plates) were treated with corrole **1C-Fe** (20 and 50 µM) added 30 minutes prior to treatment with increasing concentrations of H₂O₂ and then further incubated for 24h. The results are shown in **Fig. 3A****.**

After 2 days of culture, RIN-m cells (5 x 10⁴ cells/well in 96-well plates) were treated with corrole **1C-Fe** (1-50 µM) before addition of 35 µM of H₂O₂. Cell viability was evaluated by a colorimetric assay for mitochondrial function estimation using the MTT test as previously described (Gassen et al., 1998) and expressed as percentage of control. The results are shown in **Fig. 3****B.**

Data presented in Figs. **3A** and **3B** show a significant protective effect of corrole **1C-Fe** on beta cells against a wide range of hydrogen peroxide concentrations. Corrole **1C-Fe** shows no toxicity when given alone (without H₂O₂) at concentrations up to 50 µM **(****Fig. 3C****).**

### Example 6: Comparative effects of corroles 1C-Fe, 1C-Mn, 5C-Fe and 2C-Mn and porphyrins 1P-Fe and 1P-Mn against toxicity induced by H₂O₂ in RIN-m cells

RIN-m cells were grown as described in Example 5. After 3 days of culture, RIN-m cells (2.5 x10⁴ cells/well in 96-well plates) were treated with corroles **1C-Fe** or **5C-Fe** or porphyrin **1P-Fe** (1 and 20 µM), added 30 min prior to treatment with 35 µM H₂O₂ and further incubated for 24h. Cell injury was evaluated by MTT test. The results in **Fig. 4** represent the mean ± SEM of a representative experiment repeated twice with similar results performed under the same conditions. Corroles **1C-Fe** and **5C-Fe** (in a concentration of 20µM) exhibit a greater protective effect against toxicity induced by H₂O₂ in RIN-m cells, relative to their structurally analogous porphyrin **1P-Fe.**

Protective effect of iron(III) corrole complexes against H₂O₂-induced toxicity (150µM) was compared to that of manganese(III) corroles and related porphyrin. While the iron(III) complexes of corrole **1C-Fe** and the structurally related porphyrin **1P-Fe** showed cytoprotective effects against H₂O₂, revealing some advantages of the former **(****Fig. 5A****).** The Mn(III) complexes **1C-Mn** and **2C-Mn** and the structurally analogous porphyrin **2P-Mn** were totally inefficient (**Figs**. **5A-5B****).**

### Example 7: Protective effect of iron corroles 1C-Fe and 5C-Fe against toxicity induced by the nitric oxide donor, SIN-1 (a precursor of peroxynitrite), in INS-1E and RIN-m cells

INS-1E cells or RIN-m cells were cultured in RPMI with low serum content (2% FCS), placed in microtiter plates (96 wells) at a density of 2.5 x10⁴ cells/well and allowed to attach for 24h before treatment. Corroles 1C-Fe and 5C-Fe (10 µM) were added 30 min prior to insult with the nitric oxide donor SIN-1 (500 or 600 µM) for a subsequent 24h period. Cell viability was evaluated by a colorimetric assay for mitochondrial function using 3-(4,5-dimethylthiazol-2-yl)-2,5-diphenyltetrazolium bromide (MTT test), and expressed as percentage of untreated control. Values higher than 100% reflect increased mitochondrial activity induced by the drug. The results represent the mean ± SEM of three independent experiments performed under the same conditions.

The results depicted in **Figs. 6A-6B** demonstrate a marked protective effect of corrole **1C-Fe** and **5C-Fe** against SIN-1 induced toxicity on INS-1E cells and RIN-m beta cells, respectively. The damage is more pronounced in INS-1E cells, while the protection is extremely efficient in both cases.

### Example 8. Comparative effects of iron corroles 1C-Fe and manganese corroles 1C-Mn and 2C-Mn relative to their respective porphyrins 1P-Fe and 2C-Mn in RIN-m beta cells exposed to SIN-1.

Insulin-secreting RIN-m cells were incubated in RPMI with low serum content (2% FCS) and placed in microtiter plates (96 wells) at a density of 2.5 x10⁴ cells/well and allowed to attach for 24 h before treatment. Cells were treated with corroles **1C-Fe, 1C-Mn** or **2C-Mn** or porphyrins **1P-Fe** or **2P-Mn,** in various concentrations (0.1-20µM), for 30 min prior to insult with SIN-1 (800 µM) for a subsequent 24 h period. Cell viability was evaluated by MTT test and expressed as percentage of untreated control.

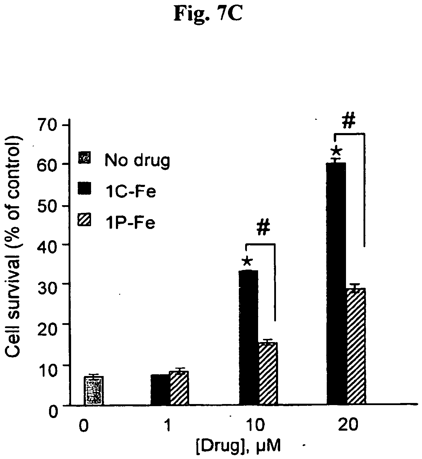

The first comparison concerned the cytoprotective properties of positively charged manganese corrole **2C-Mn** vs. its negatively charged analog **1C-Mn (****Fig. 7A****).** The results were unequivocally in favor of the former and **2C-Mn** was chosen as leading compound for manganese complexes. For revealing possible corrole's advantages, a structurally analogous porphyrin **2P-Mn** was also synthesized and was investigated in the same experiments **(****Fig. 7C****).** Iron corrole **1C-Fe** and related porphyrin **1P-Fe** were also tested under the same conditions, revealing advantages of the former **(****Fig. 7B****).**

At each drug concentration, the iron corrole **1C-Fe** exhibits a greater protective effect on RIN-m beta cells, against toxicity induced by SIN-1, relative to its respective porphyrin **1P-Fe (****Fig.7B****).** The Mn-Corrole **2C-Mn** also shows improved protective effect on RIN-m cells against toxicity induced by SIN-1, relative to its corresponding porphyrin **2P-Mn (****Fig. 7C****).**

### Example 9. Neuroprotective and neurorescue effects of iron(III) corroles IC-Fe and 5C-Fe on H₂O₂-induced cell death in human neuroblastoma SH-SY5Y cell line.

Human neuroblastoma cell line was also examined under conditions similar to RIN-m cells to reveal the corrole's ability to prevent neuronal death caused by oxidative stress. SH-SY5Y cells were plated in microtiter plates (96 wells) in DMEM-Eagle/F-12(HAM) (1:1), containing 10% FCS. To examine neuroprotective effect, SH-SY5Y cells were treated for 30 min with corroles **1C-Fe** and **5C-Fe** (20 and 50 µM) before addition of 20 µM H₂O₂ for 24 h. To examine neurorescue, SH-SY5Y cells were first exposed to 20 µM H₂O₂ for 0.5h, 1.5h or 3h, followed by the addition of corroles **1C-Fe** and **5C-Fe** (20 µM) and incubation for 24h.

The results are shown in **Figs. 8A-8B****.** Both corrole **1C-Fe** and **5C-Fe** show a dose-dependent neuroprotective effect against H₂O₂-induced toxicity in SH-SY5Y cells **(****Fig. 8A****).** Moreover, corroles **1C-Fe** and **5C-Fe** instigated neurorescue of SH-SY5Y cells following insult with H₂O₂ for various time periods **(****Fig. 8B****).**

### Example 10. Comparative effects of corrole 1C-Fe and porphyrin 1P-Fe against toxicity induced by H₂O₂ in SH-SY5Y cells

Compounds **1C-Fe** and **1P-Fe** (1-50 µM) were added 30 min before insult with H₂O₂ (200 µM), followed by a subsequent 24 h period. The neuroprotective effects of the iron corrole **1C-Fe** and its structurally analogous iron porphyrin **1P-Fe**, against H₂O₂-induced neurotoxicity were investigated using MTT reduction assay and cell death ELISA, based on the use of mouse monoclonal antibodies to detect free histones and fragmented DNA (F. Hollderieder et al, Biochemica 2002, 1, 25). The results show (**Figs**. **9A**-**9C**) that H₂O₂ at 200 µM for 24h markedly decreased cultured human neuroblastoma SH-SYSY cell viability when compared with untreated control cells. Pretreating the cells with corrole **1C-Fe** (1-20 µM) for 30 min prior to the incubation with 200 µM H₂O₂ for 24 h, dose-dependently increased cell viability, as determined by MTT reduction when compared with H₂O₂-treated cells (**Fig. 9A**). Corrole **IC-Fe** added in the absence of H₂O₂ (up to 50 µM) had no significant effect on the viability of SH-SY5Y (**Fig**. **9C**). Similar results were obtained when neuroprotection was evaluated by ELISA detection assay, demonstrating that corrole **1C-Fe** significantly and dose-dependently protected SH-SY5Y cells against H₂O₂ neurotoxicity (**Fig. 9B**). Consistently, at all concentrations studied, corrole **1C-Fe** displayed superiority over porphyrin **1P-Fe** in protecting SH-SY5Y cells from H₂O₂-induced neurotoxicity. In light of the observed neuroprotective effect of corrole **1C-Fe,** we attempted to determine the length of the delay between H₂O₂ exposure and corrole **1C-Fe** treatment that would still afford neuroprotection. H₂O₂ was administered to SH-SY5Y cells and corrole **1C-Fe** was then added for 0.5-1.5 h thereafter. The neuroprotective effect of corrole **1C-Fe** remained unaltered at 0.5 h post-H₂O₂ insult (**Figs. 9A** **and** **9D**). At 1 and 1.5 h post- H₂O₂ insult, corrole **1C-Fe** significantly protected SH-SY5Y cells, but the extent of protection gradually decreased (**Fig. 9D**). Notably, both pyridinium-substituted manganese corroles **2C-Mn** and **3C-Mn** (up to 50 mM) were not effective at preventing H₂O₂-mediated cytotoxicity (data not shown).

Cytoprotection by iron complexes against different H₂O₂ concentrations is shown in **Fig. 10****.** As in previous cases, the corrole showed superiority over the analogous porphyrin regarding cell survival in conditions of induced oxidative damage by different H₂O₂ concentrations.

### Example 11. Neuroprotective and neurorescue effects of iron corroles 1C-Fe and 5C-Fe against SIN-1-induced cell death in human neuroblastoma SH-SY5Y cell line.

SH-SY5Y cells were cultured in DMEM-Eagle/F-12(HAM) (1:1) containing 10% FCS. Cells were resuspended in medium containing 2% FCS, seeded in microtiter plates (96 wells) and allowed to attach for 24 h before initiation of treatment. To examine neuroprotective effects, SH-SY5Y cells were treated for 30 min with corroles **1C-Fe** and **5C-Fe** (10 and 20 µM) before addition of 20 µM H₂O₂ for 24 h. To examine neurorescue, SH-SY5Y cells were first exposed to 20 µM H₂O₂ for 0.5h, 1.5h or 3h followed by the addition of corroles **1C-Fe** and **5C-Fe** (20 µM) and incubation for 24h.

The results are shown in **Figs. 11A-11B****.** Both corroles **1C-Fe** and **5C-Fe** showed a dose-dependent neuroprotective effect against SIN-1 induced toxicity in SH-SY5Y cells (**Fig**. **11A**). Moreover, corroles **1C-Fe** and **5C-Fe** instigated neurorescue of SH-SY5Y cells following insult with SIN-1 for various time periods (**Fig**. **11B**).

### Example 12: Comparative effects of corroles 1C-Fe, 2C-Mn and 3C-Mn and porphyrins 1P-Fe and 2P-Mn against toxicity induced by SIN-1 in SH-SY5Y cells

SH-SY5Y cells were pretreated with or without corroles **1C-Fe, 2C-Mn** and **3C-Mn** and structurally related porphyrins **1P-Fe** and **2P-Mn,** respectively, 30 min before exposure to SIN-1 for a subsequent 24 h period. **Figs. 12A-12B** demonstrate the ability of iron corrole **1C-Fe** and manganese corroles **2C-Mn** and **3C-Mn** to protect, in a dose-dependent manner, SH-SY5Y cells against SIN-1, as determined by both the MTT reduction analysis and ELISA assay. The iron corrole **1C-Fe** at concentrations of 10 and 20 µM was able to inhibit neuronal death, but was less potent than the manganese corroles **2C-Mn** and **3C-Mn.** In addition, both the iron and manganese corroles displayed significant superiority over their respective analogous porphyrins.

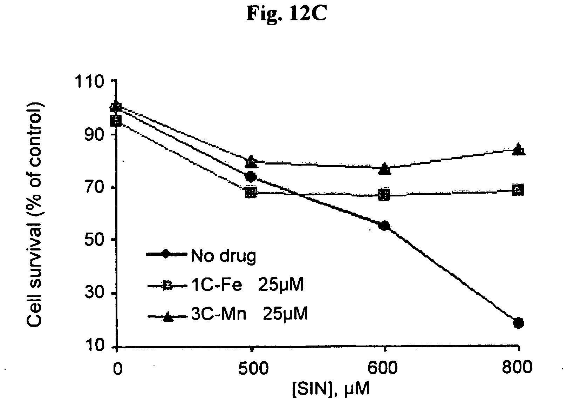

In addition to **2C-Mn** with its positively-charged para-pyridinium moieties, **3C-Mn** with *ortho*-pyridinium moieties has been examined as well. The manganese(III) corrole **3C-Mn** appears to be more effective not only than **2C-Mn,** but also shows better cytoprotective properties than the iron(III) corrole complex **1C-Fe.** **Fig. 12C** reveals the ability of both iron(III) and manganese(III) corroles 1C-Fe and 3C-Mn (25 µM) to protect cells against different SIN-1 concentrations, with the cytoprotective properties of **3C-Mn** apparently being better than that of **1C-Fe** at all toxin concentrations.

To determine the effect of the corroles on SIN-1-induced cellular protein nitration, SH-SY5Y neuroblastoma cells were treated with SIN-1 (700 µM) in the absence or presence of 20 µM of the corroles **1C-Fe, 2C-Mn,** and **3C-Mn.** Protein nitration was detected by immunofluorescence analysis using a monoclonal antibody against nitrotyrosine (Ntyr), a biological marker of peroxynitrite. **Figs. 13A-13B** illustrate that immunoreactivity to Ntyr was markedly induced by exposure to SIN-1 compared with control cells. Administration of iron corrole resulted in partial (albeit quite remarkable) reduction in Ntyr staining, while both manganese corroles **2C-Mn** and **3C-Mn** totally abolished SIN-1-induced formation of nitrotyrosine.

### Example 13: Neuroprotective and neurorescue effects of iron corroles 1C-Fe and 5C-Fe against 6-hydroxydopamine (OHDA)-induced cell death in human neuroblastoma SH-SY5Y cell line.

In this series of experiments, we induced cellular damage by 6-OHDA - as a model for Parkinson's disease. 6-Hydroxydopamine (6-OHDA) is a dopaminergic neurotoxin putatively involved in the pathogenesis of Parkinson's disease (PD). Its neurotoxicity has been related to the production of reactive oxygen species. Under physiological conditions, 6-OHDA is rapidly and nonenzymatically oxidized by molecular oxygen to form hydrogen peroxide and the corresponding p-quinone. The latter then undergoes an intramolecular cyclization followed by a cascade of oxidative reactions resulting in the formation of an insoluble polymeric pigment related to neuromelanin. Although the precise molecular mechanism of cytotoxicity for 6-OHDA remains uncertain, it has been often linked to the production of ROS. The H₂O₂ resulting from the autoxidation of 6-OHDA can easily be reduced in the presence of Fe²⁺ by the Fenton reaction to give the hydroxyl radical, which as mentioned before is considered the most damaging free radical for living cells.

SH-SY5Y cells were cultured in DMEM-Eagle/F-12(HAM) (1:1) containing 10% FCS. Cells were resuspended in medium containing 2% FCS, seeded in microtiter plates (96 wells) and allowed to attach for 24h. To examine the neuroprotective effect, cells were pretreated for 30 min with corroles **1C-Fe** and **5C-Fe** (1-20 µM), and were then exposed to 6-OHDA (60 µM) for further 24h. For neurorescue assessment, SH-SY5Y cells were first treated with 6-OHDA (60 µM) for 0.5h, 1.5h, 3h and 6h followed by the addition of compounds **1C-Fe** and **5C-Fe** (10 µM) for further 24h.

The results are depicted in **Figs. 14A-14B****.** Both corrole **1C-Fe** and **5C-Fe** show a dose-dependent neuroprotective effect against 6-OHDA-induced toxicity in SH-SY5Y cells with corrole **1C-Fe** showing a greater effect (**Fig**. **14A**). Moreover, corroles **1C-Fe** and **5C-Fe** instigated neurorescue of SH-SY5Y cells following insult with 6-OHDA for various time periods (**Fig**. **14B**).

### Example 14. Comparative effects of corroles 1C-Fe, 2C-Mn and 3C-Mn and porphyrins 1P-Fe and 2P-Mn against 6-OHDA-induced neurotoxicity in SH-SY5Y cells

SH-SY5Y cells were pretreated with or without corroles **1C-Fe, 2C-Mn** and **3C-Mn** and porphyrins **1P-Fe and 2P-Mn** (20 µM), 30 min before exposure to 6-OHDA (40 µM) for a subsequent 24 h period. Cell viability was evaluated by MTT test and cell death was assessed using the apoptotic cell death detection ELISA kit. For neurorescue, SH-SY5Y cells were first exposed to 40 µM 6-OHDA for 0.5. 1, 1.5 and 3 h, followed by the addition of the compounds **1C-Fe, 2C-Mn and 3C-Mn** (20 µM) and incubated for further 24 h. Growth-associated protein (GAP-43) was detected by fluorescence microscopy using a specific primary antibody. Apoptotic nuclei were determined by terminal deoxynucleotidyl transferase-mediated UTP-digoxigenin nick end labeling analysis. 4',6-Diamidino-2-phenylnidole (DAPI) staining was applied for nuclear labeling. 6-OHDA was detected by fluorescence microscopy using a specific primary antibody.

As can be seen in **Figs. 15A-15B****,** the iron corrole **1C-Fe,** as well as the manganese corroles **2C-Mn** and **3C-Mn,** significantly attenuated 6-OHDA-induced cytotoxicity in SH-SY5Y cell cultures. The magnitude of the neuroprotective effect was more pronounced for the manganese corroles. Additional neurorescue experiments (**Fig**. **15C**) revealed that the iron and manganese corroles confer pronounced neuroprotective effect at 0.5 h post-administration of 6-OHDA, that becomes gradually reduced up to 3 h after exposure to the insult. To further confirm the neuroprotective effect of the corroles in SH-SY5Y cells exposed to 6-OHDA, we employed an immunofluorescence analysis, using a specific primary antibody against the axonal marker, growth-associated protein (GAP-43), and DAPI- staining for nuclear labeling.

**Figs. 16A-N** show that treatment with the corroles **1C-Fe, 2C-Mn** and **3C-Mn** (20 µM) significantly attenuated 6-OHDA-induced cell mortality, improved cell morphology and reduced the number of apoptotic nuclei, as compared with vehicle-treated cells (**16A**-**D**). Furthermore, all three corroles (**1C**-**Fe**, **2C-Mn** and **3C-Mn**) exhibited a remarkable superiority over the porphyrins **1P-Fe** and **2P-Mn,** in agreement with the results obtained by MTT test and cell death ELISA analysis.

Since 6-OHDA is also known to induce apoptosis in various cell types, including SH-SY5Y (von Coelln et al. 2001; Nie et al. 2002; Shimizu et al. 2002; Jordan et al. 2004), we further tested effect of corroles on the levels of the apoptotic marker, cleaved caspase-3, a major executer of the mitochondrial intrinsic pathway of apoptosis. **Figs. 17A-E** show that SH-SY5Y cells incubated with vehicle and exposed to 6-OHDA (40 µM) exhibited a significant increase in the levels of cleaved caspase-3 versus control cells. This effect on cleaved caspase-3 was abolished partially by iron corrole **1C-Fe** administration or totally by administration of manganese corroles **2C-Mn** or **3C-Mn,** as indicated by immunofluorescence analysis (**Fig**. **17F**).

### Example 15: Comparative effects of corroles 1C-Fe, 2C-Mn and porphyrins I P-Fe, 2P-Mn against toxicity induced by SIN-1 or by H₂O₂ in NSC-34 cells.

The neuroprotective effects of the iron corrole **1C-Fe** and analogous porphyrin **1P-Fe** against H₂O₂ insult were further examined in the motor neuron cell line, NSC-34. NSC-34 cell is a hybrid neuroblastoma x spinal cord (NSC) cell line that resembles motor neurons, displaying a multipolar neuron-like phenotype. Mouse motoneuronal NSC-34 cells were pretreated with or without the corroles **1C-Fe** or **2C-Mn** or the structurally-related porphyrins **1P-Fe, 2P-Mn,** respectively, 30 min before exposure to H₂O₂ (200 µM) or SIN-1 (700 µM) for a subsequent 24 h period. Cell viability (**Figs**. **18A**, **19A**) was evaluated by MTT test and cell death (**Figs**. **18B**, **19B**) was assessed using the apoptotic cell death detection ELISA kit. [The graphs in **Fig. 18A-18B** and **19A-19B** present results expressed as percentage of untreated control. Data are expressed as mean ± SEM (n=6) of a representative experiment that was repeated twice with similar results.

As shown in **Figs. 18A-18B** for corrole **1C-Fe** (20 µM) and porphyrin **1P-Fe,** the corrole **1C-Fe** was found to confer significant protection against H₂O₂-mediated cytotoxicity in NSC-34 cells, in both MTT (**Fig**. **18A**) and ELISA (**Fig**. **18B**) assays. Consistent with the results in SH-SY5Y cells, it was found that the porphyrin **1P-Fe** was not markedly effective in preventing H₂O₂-mediated cytotoxicity in NSC-34 cells. As shown in **Figs. 19A-19B** for corroles **1C-Fe** and **2C-Mn** (20 µM) and porphyrins **1P-Fe** and **2P-Mn,** both the iron corrole **1C-Fe** (20 µM) and the manganese corrole **2C-Mn** (20 µM) significantly attenuated SIN-1 (700 µM)-induced cytotoxicity in NSC-34 motor neuron cells, revealing higher cytoprotective activity than their respective analogous porphyrins **1P-Fe** and **2P-Mn.**

### Example 16: Neuroprotective effect of iron corroles 1C-Fe and 5C-Fe in cell culture model of familial ALS.

Mutant-superoxide dismutase (G93A-SOD1) is associated with familial amyotrophic lateral sclerosis (FALS). A cell culture model of FALS was implemented by stably transfecting mouse motoneuronal NSC-34 cells or SH-SY5Y cells, to express mutant G93A-SOD1 at levels approximating those seen in the human disease. NSC-34 cells or SH-SY5Y cells were incubated in Dulbecco's modified Eagle's/F-12 medium supplemented with 10% FCS tetracycline-free (FCS Tet-free; Clontech), at 37° C in an atmosphere of 5% CO2 in air. These cell lines are stably transfected with the pTet-ON plasmid (Clontech, Palo Alto, CA) coding for the reverse tetracycline controlled transactivator (rtTA). Induction of mutant SOD1 expression was obtained by shifting nonconfluent cultures into growth medium containing 1% N₂ supplement (Invitrogen, Carlsbad, CA) and 1 mg/ml doxocyclin for 48 h in the presence or absence of corrole **1C-Fe** and **5C-Fe.**

The results are shown in **Figs. 20A****-20B.** In the induced mutant cells, survival was decreased by more than 40% while treatment with either corrole **1C-Fe** or **5C-Fe** significantly improved cell viability of NSC-34 G93A cells even at a concentration as low as 0.5 µM (**Fig**. **20A**). Similar results were obtained when the G93A mutation was expressed in SH-SY5Y cells (**Fig**. **20B**).

### Example 17: Cellular uptake of the corroles

For testing cellular membrane penetration, as well as uptake and accumulation of corroles in the cells, we took advantage of the fluorescence property of an appropriate corrole. For this purpose we used a fluorescent corrole complex (**1C**-**Ga**) that is structurally identical to the catalytic compounds utilized in the cytoprotection/cytorescue experiments, except that the chelated metal is gallium(III) rather than iron(III) in **1C-Fe** or manganese(III) in **1C-Mn.** The results are shown in **Figs. 21A-D** for RIN-m (insulinoma) cell line and **Figs. 22A-D** for SH-SY5Y (neuroblastoma) cell line. Both figures demonstrate corrole's accumulation inside the cells originated from different cell lines.

To visualize intracellular corrole we used fluorescence microscopy. Neuroblastoma SH-SY5Y cells and insulinoma RIN-m cells were plated on coverslips. At 24 h after plating, corrole **1C-Ga** was added directly to the cell media and incubated for half an hour at 37 °C. The cells were then aspirated from the media and washed with phosphate buffer to eliminate background signal due to extra cellular corrole. Fixation with formaldehyde was performed. Coverslips with mounting medium (with DAPI) were directly placed on slides and viewed by fluorescence microscopy; the results are shown in **Figs. 21, 22****.**

From these results we observe that the corrole probably accumulates in the cytoplasm of neuroblastoma and insulinoma cells, but remains excluded from the nucleus. For more accurate and specific analysis of corrole distribution between cellular organelles, specific staining and confocal microscopy will be utilized in the future.

### Example 18. Preparation of 2,17-bis-sulfonato-5,10,15-tris(p-methoxy-tetrafluorophenyl)iron(III) corrole (Corrole 6C-Fe, Scheme 1):

### 18.1 Preparation of 5,10,15-tris(p-methoxy-tetrafluorophenyl)corrole

200 mg of 5,10,15-tris(pentafluorophenyl)corrole was disolved in 100 mL of sodium methoxide solution (0.5 M in methanol). The solution was heated to reflux for 6 hr under argon, followed by evaporation of the solvent. The product was purified by two subsequent silica gel columns (the eluent was ethanol for the first column and CH₂Cl₂/n-hexane 2:1 for the second column), affording 160 mg (77% yield) of 5,10,15-tris(paramethoxytetrafluorophenyl)corrole. ¹H NMR (300 MHz, CDCl₃) δ= 9.02 (d, J= 4.0 Hz, 2H), 8.73 (d, J= 4.8 Hz, 2H), 8.54 (d, J= 4.8 Hz, 2H), 8.51 (d, J= 4.0 Hz, 2H), 4.31 (s, 9H). ¹⁹F NMR (282.4 MHz, CDCl₃) δ= -139.6 (dd, J¹= 22 Hz, J² = 7.0 Hz, 2F), -140.1 (dd, J¹= 22 Hz, J² = 7.0 Hz, 4F), -158.2 (dd, J¹= 22 Hz, J²= 7.0 Hz, 4F), -158.6 (dd, J¹= 22 Hz, J²= 7.0 Hz, 2F).

### 18.1 Preparation of 2,17-bis-sulfonato-5,10,15-tris(p-methoxy-tetrafluorophenyl)corrole (Corrole 6C, Scheme 1).:

100 mg of 5,10,15-tris(p-methoxy-tetrafluorophenyl)corrole and 10 ml of sulfuric acid was stirred at 25 C for 4 hr, after which the reaction mixture was cooled by an ice bath and treated with small ice chips (5-10 g). The acid was neutralized by sodium carbonate, and the product was separated from the sodium sulfate via adding ethanol, filtration and evaporation. The product was purified by silica gel column (the eluent was CH₂Cl₂/ethanol 2:1), affording 80 mg (67% yield) of 2,17-bis-sulfonato-5,10,15-tris(p-methoxytetrafluorophenyl)corrole. ¹H NMR (300 MHz, CD₃OD) δ= 9.67 (s, 1H), 8.57 (s, 1H), 8.38 (d, J= 4.8 Hz, 1H), 8.22 (d, J= 4.5 Hz, 1H), 8.15 (d, J= 4.8 Hz, 1H), 8.14 (d, J= 4.5 Hz, 2H), 4.24 (s, 3H), 4.23 (s, 3H), 4.21 (s, 3H). ¹⁹F NMR (282.4 MHz, CD₃OD) δ= -140.9 (dd, J¹= 24Hz, J²= 8.0 Hz, 2F), -141.9 (dd, J¹= 24 Hz, J²= 8.0 Hz, 2F), -142.1 (dd, J¹= 24 Hz, J²= 8.0 Hz, 2F), -161.6 (dd, J¹= 24 Hz, J²= 8.0 Hz, 2F), -162.1 (dd, J¹= 24 Hz, J²= 8.0 Hz, 2F), -164.3 (dd, J¹= 24 Hz, J²= 8.0 Hz, 2F). MS (TOF LD-) *m*/*z* (%) 1011.9 (100%) [M²⁻ + Na⁺).

### 18.3 Preparation of 2,17-bis-sulfonato-5,10,15-tris(p-methoxytetrafluorophenyl)iron(III) corrole (6C-Fe).

One portion of FeCl₂.4H₂O (100 mg) was added at once to pyridine solution (10 ml) of 2,17-bis-sulfonato-5,10,15-tris(paramethoxytetrafluorophenyl)corrole (100 mg), and the mixture was heated immediately to reflux for 10 min. The product was purified by silica gel column ( the eluent was ether/ethanol 3:1 at the beginning then ether/ethanol 1:2), affording 75 mg (71% yield) of the title product 2,17-bis-sulfonato-5,10,15-tris(paramethoxytetrafluorophenyl)iron(III) corrole. ¹⁹F NMR (282.4 MHz, CD₃OD) δ=-109.2 (1F), -119.3 (2F), -153.4 (1F), -154.8 (1F), - 157.4 (1F). MS (TOF LD-) *m*/*z* (%)1065.9 (100%) [M²⁻ + Na⁺).

### Example 19. Preparation of 2,17-bis-sulfonato glycine-5,10,15-tris(pentafluorophenyl)iron(III) corrole (corrole 5C-Fe, Scheme 1):

### 19.1 Preparation of 2,17-bis-sulfonato glycine ethyl ester-5,10,15-tris(pentafluorophenyl) corrole

A solution of 2,17-bis-sulfonylchloride-5,10,15-tris(pentafluoro-phenyl) corrole (70 mg) and 200 µl glycine ethyl ester in CH₂Cl₂ (20 ml) was stirred for 1 hr. The solution was washed twice with a solution of HCl (2 M) and then with distilled water. The solvent was evaporated and 2,17-bis-sulfonato glycineethylester-5,10,15-tris(pentafluorophenyl) corrole was obtained in quantitative yield. ¹⁹F NMR (188 MHz, CDCl₃) δ= -136.8 (broad peak, 4F), -139.0 (broad peak, 2F), -150.7 (broad peak, 1F), -151.1 (broad peak, 2F), -160.7 (broad peak, 4F), -162.5 (broad peak, 2F),. MS (TOF LD+) *m*/*z* (%) 1149.2 (100%) [M + Na⁺).

### 19.2 Preparation of 2,17-bis-sulfonato glycine ethyl ester-5,10,15-tris(pentafluorophenyl)iron(III) corrole

One portion of FeCl₂.4H₂O (70 mg) was added at once to pyridine solution (10 ml) of 2,17-bis-sulfonato glycineethylester-5,10,15-tris(pentafluorophenyl) corrole (70 mg), and the mixture was heated immediately to reflux for 10 min. The product was purified by silica gel column (the eluent was CH₂Cl₂/n-hexane 1:1 at the beginning then CH₂Cl₂/THF 100:1), affording 60 mg (82% yield) of 2,17-bis-sulfonato glycine ethyl ester-5,10,15-tris(pentafluoro-phenyl)iron(III) corrole. ¹⁹F NMR (282.4 MHz, CDCl₃) δ= -118.1 (2F), -123.0 (2F), -124.9 (2F), -153.7 (2F), - 156.1 (1F), -160.9 (2F), -161.2 (2F), -163.2 (2F). MS (TOF LD-) *m*/*z* (%) 1179.242 (80%) [M), 1202.244 (100%) [M + Na]).

### 19.3 Preparation of 2,17-bis-sulfonato glycine -5,10,15-tris(pentafluoro-phenyl) iron(III) corrole:

60 mg of 2,17-bis-sulfonato glycine ethyl ester-5,10,15-tris(pentafluorophenyl)iron(III) corrole and 100 ml of water that contain 500 mg sodium carbonate was refluxed for 1 hr, after which the basic solution was neutralized by HCl and washed with CH₂Cl₂. The product transferred to the organic phase. The CH₂Cl₂ washed twice with water and dried by sodium sulfate. 45 mg of the end product 2,17-bis-sulfonato glycine -5,10,15-tris(pentafluorophenyl)iron(III) corrole were obtained (79% yield) after filtration and evaporation of the CH₂Cl₂ solution. ¹⁹F NMR (282.4 MHz, CDCl₃) δ= -118.2 (2F), -123.1 (2F), -125.0 (2F), -153.8 (2F), - 156.1 (1F), -161.1 (4F), -163.3 (2F). MS (TOF LD-) *m*/*z* (%) 1122.468 (100%) [M]).

### Example 20. Preparation of corroles 3C-Mn and 4C-Mn (Scheme 1).

The preparation of the metal-free and nonalkylated corroles **3C** and **4C**, from which the metal complexes **3C-Mn** and **4C-Mn,** respectively, were prepared, is described in Saltsman et al., 2008.

### 20.1 Preparation of 5, 10, 15- tris(o-pyridyl)corrole (3C, the precursor of 3C-M, Scheme 1).

For the preparation of title compound in Example 20.1, 2-pyridinium dipyrromethane and appropriate aldehyde (2-pyridine carboxaldehyde/ pentafluorobenzaldehyde) were reacted as described by Gryko and Piechota, 2002. Shortly, samples of dipyrromethane (0.4mmol) and the appropriate aldehyde (0.2 mmol) were dissolved in CH₂Cl₂ (12 mL) and trifluoroacetic acid (62 µL, 0.8 mmol) was added to this stirring mixture at room temperature. After 1h, triethylamine (112 µL, 0.8 mmol) was added and the reaction mixture was diluted with CH₂Cl₂ (308 mL). DDQ (90 mg, 0.4 mmol) was added and stirring was continued for a further 10 min. The reaction mixture was evaporated to dryness and purified by column chromatography on silica, with the purification details that are described for each case.

The reaction mixture was purified via column chromatography on silica. The second blue-green band was eluted with ethyl acetate: *n*-hexane (at first, 3: 1 and then gradually up to 100% ethyl acetate, then 10% methanol in ethyl acetate). Second chromatographic treatment (ethyl acetate: n-hexane, 3: 1, then 3% n-hexane in ethyl acetate, then 5% methanol in ethyl acetate) provided pure corrole (25 mg, 24% yield), R_{f} (silica, ethyl acetate) = 0.24 ¹H 500 MHz NMR (C₆D₆) δ = 8.81 (br s, 2H), 8.79 (d, ³*J* (H,H) = 4.12 Hz, 2H), 8.67 (br s 1H), 8.65 (d, ³*J* (H,H) = 4.35 Hz, 2H), 8.44 (d, ³*J* (H,H) = 4.12 Hz, 2H), 8.23 (d, ³*J* (4.58 Hz, 2H), 8.04 (d, ³*J* (H,H) = 7.56 Hz, 2H), 7.91 (d, ³*J* (H,H) = 7.33 Hz, 1H), 7.34 (m, 3H), 6.92 (m, 3H). UV-vis (ethylacetate): λₘₐₓ, nm (ε x 10⁻³) 418 (36.3), 582 (6.7), 614 (4.4).
MS (MALDI-TOF): *m*/*z* (%): 528.3 [M⁻, 100%]; 530.5 [M⁺, 100%].

### 20.2 Preparation of 10-(pentafluorophenyl)-5, 15-bis(o-pyridyl)corrole (4C).

The reaction mixture from the reaction with pentafluorobenzadehyde was purified via column chromatography on silica. The second green-blue band that was eluted with ethyl acetate: n-hexane (1: 4, then 1: 2) provided a fraction that contained corrole **4C.** Final purification of **4C** was achieved by preparative thin-layer chromatography (silica plate, ethyl acetate: *n*-hexane, 3:4) as to afford pure **4C** (27 mg, 22% yield), R_{f} (silica, ethyl acetate: *n*-hexane, 2: 3) = 0.81. ¹H 300 MHz NMR (C₆D₆): δ = 8.74 (br s, 2H), 8.61 (d, ³*J* (H,H) = 4.12 Hz, 2H), 8.22 (d, ³*J* (H,H) = 4.67 Hz, 2H), 8.09 (d, ³*J* (H,H) = 7.96 Hz, 2H), 7.71 (d, ³*J* (H,H) = 4.94 Hz, 2H), 7.06 (m, 4H), 6.42 (m, 2H), -1.47 (br s, 3H). ¹⁹F 282 MHz (C₆D₆): δ = 138.36 (dd, ³*J* (F,F) = 25.3 Hz, ⁴*J* (F,F) = 5.6 Hz, 2F), -154.16 (t, ³*J* (F,F) = 22.6 Hz, 1F), -162.96 (td, ³*J* (F,F) = 25.4 Hz, *⁴J* (F,F) = 8.5 Hz, 2F). UV-vis (EtOAc): λₘₐₓ, nm (ε x 10⁻³) 416 (48.5), 578 (10.3).
MS (MALDI-TOF): *m*/*z* (%): 617.0 [M-, 100%]; 619.2 [M⁺, 100%].
MS (MALDI-TOF): *m*/*z* (%): 619.2 [M⁺, 100%].

### 20.3 Preparation of N-methylated 3C and 4C.

**3C** and **4C** were dissolved in the minimum volume of DMF and excess methyl iodide (100eqv) was added to the solutions. Reaction mixtures were stirred at room temperature overnight. Small amount of methanol and three-fold excess of diethyl ether were added the reaction mixtures. Precipitated products were collected and washed with addition portion of diethyl ether.
**5,10,15-tris(N-methyl-*o*-pyridiniumyl)corrole (3C³⁺):** was obtained as a mixture of three atropoisomeric structures, which were separated by reversed-phase HPLC. R_{f} (silica, KNO₃ sat : H₂O : acetonitrile, 1:1:8)= 0.46. UV-vis (acetonitrile): λ_{λmax}, nm (ε x 10⁻³) 408 (8.0), 436 (8.2), 626 (3.3).
MS (MALDI-TOF) ES⁺ (CH₃CN): *m*/*z* (%): 575 (10) [M⁺], 560 (40) [M-15(CH₃)], 545 (100) [M-30(2xCH₃)], 287 (100) [M⁺]/2, 196 (25) [M+16(O)]/3.

**10-(pentafluorophenyl)-5,15-bis(N-methyl-*o*-pyridyniuml)corrole (4C²⁺):** was obtained as a mixture of two atropoisomers, which were separated by reversed-phase HPLC. R_{f} (silica, KNO₃ sat: H₂O : acetonitrile, 1 : 1 : 8) = 0.55. ¹H 300 MHz NMR CD₃CN): δ = 9.15 (d, ³*J* (H,H) =4.16 Hz, 2H), 9.12 (d, ³*J* (H,H) = 6.46 Hz, 2H), 8.69 (m, 4H), 8.56 (d, ³*J* (H,H) =4.74 Hz, 2H), 8.52 (d, ³*J* (H,H) = 4.22 Hz, 2H), 8.49 (d, ³*J* (H,H) = 4.67 Hz, 2H), 8.31 (t, ³*J* (H,H) = 6.59 Hz, 2H), 4.15 (s, 3H), 4.13 (s, 3H). ¹⁹F 282 MHz (CD₃CN): δ = -141.01 (m, 2F), -158.21 (t, ³*J* (F,F) =19.4 Hz, 1F), -165.01 (m, 2F). UV-vis (acetonitrile): λₘₐₓ, nm (ε x 10⁻³)
MS (MALDI-TOF) ES⁺ (CH₃CN): *m*/*z* (%): 648.2 (100) [M⁺], 633.2 (85) [M-15(CH₃)], 324.1 (100) [M⁺]/2.

### 20.3 Preparation of Mn(III) complex of 5, 10, 15-tris(N-methyl-o-pyridynium)corrole (3C-Mn); Mn(III) complex of 10-(pentafluorophenyl)-5, 15-bis(N-methyl-o- pyridynium)corrole (4C-Mn):

The title compounds were prepared by heating DMF solution of the methylated **3C³⁺** or **4C²⁺** to reflux with 15 eqv of Mn(OAC)₂·4H₂O. UV-vis and TLC examinations revealed no starting material. Solvent evaporation and carefully separation by chromatographic column of the residue (silica gel, KNO₃sat: H₂O: acetonitrile, 1: 1: 8 for **3C-Mn** and KCl sat: H₂O: acetonitrile, 1: 1: 8) for **4C-Mn**) afforded 73 % and 85 % yield, respectively, as dark-green solids.
**3C-Mn:** R_{f} (silica, KNO₃sat: H₂O: acetonitrile, 1:1:8)= 0.24. MS (MALDI-TOF) LD⁺ (CH₃CN): *m*/*z* (%): 626.1 (10) [M⁺], 611.1 (30) [M⁺-CH₃], 596.1 (100) [M⁺- (2xCH₃)], 581.1 (95) [M⁺-(3xCH₃)]; ES⁺: 279.0 (100)[M⁺/3 + (2xCl⁻)].
**4C-Mn**: R_{f} (silica, KNO₃sat: H₂O: acetonitrile, 1: 1: 8) = 0.78. ¹⁹F 282 MHz (CD₃CN): δ = -119.29 (br s, 2F), - -154.06 (m, 1F), -157.51 (m, 2F). MS (MALDI-TOF) ES⁺ (CH₃CN): *m*/*z* (%): 350.1 (100) [M⁺]/2.

### Example 21: Synthesis of 5,15-bis(4-N-propargyl-pyridilium)-10-pentafluorophenyl corrolato manganese(III) (E-pr-Mn, Scheme 2)

Corrole bearing pyridine substituents was synthesized as follows: Pentafluorobenzaldehyde (50 µL, 0,4 mmol) was added to a 10 mL solution of 4-pyridyl-dipyrromethane (178 mg, 0.8 mmol) in propionic acid and the mixture was heated to reflux for 50 min. The residue obtained after solvent evaporation was washed with hot water, neutralized with ammonium hydroxide (25 %), and washed again with hot water. The solid material was dissolved in methanol, basic alumina was added, and the solvent was evaporated. Separation between corrole and the analogous porphyrin was achieved by column chromatography (silica, CH₂Cl₂ followed by 0.5 % methanol) followed by separation by preparative thin-layer chromatography (silica plate, CHCl₃/MeOH 50:1) affording pure corrole (18 mg, 8%).

*R*_{f}=0.15 (CH₂Cl₂/ethyl acetate 1:1). UV/Vis (CH₂Cl₂/MeOH (2:1)): λₘₐₓ (ε10⁻³)=416 (104.99), 576 (16.14), 610 (9.40), 640 (5.34). MS (MALDI-TOF): *m*/*z* (%): 619 (100) [*M*⁺]. ¹H NMR (200 MHz, C₆D₆): δ=8.93 (br s, 4 H), 8.67 (d, *J*=4 Hz, 4 H), 8.26 (m, 4 H), 7.90 ppm (br. s, 4 H). ¹⁹F (188 MHz): δ=-138.79 (d, *J*=23.5 Hz, 2 F), -153.22 (t, *J*=21.9 Hz, 1 F), -162.37 ppm (t, *J*=22.5 Hz, 1 F).

The corresponding manganese complex was prepared by heating the corrole solution in pyridine at reflux with 15 equivalents of Mn(OAc)₂·4 H₂O followed by chromatographic separation (silica, starting with CH₂Cl₂ and gradually adding methanol), affording 81 % yield. UV/Vis (MeOH): λₘₐₓ (ε10⁻³)=368 (16.7), 402 (26.7), 420 (4.8), 458 (18.4), 484 (16.2), 634 (9.4). MS (MALDI-TOF LD⁺): *m*/*z* (%): 670 (100) [*M*⁺]. ¹⁹F (C₅D₄N) (188 MHz): δ=-136.58 (br s, 2 F), -155.16 (s, 1 F), -161.23 ppm (s, 2 F)

N-alkynylation: The manganese complex was dissolved in hot THF and excess propargyl bromide was added to the solution, which were then left at 50 °C until complete precipitation. The solid material was collected by centrifugation and washed with THF and diethyl ether until the solvent was colorless. UV/Vis (methanol): λₘₐₓ 462, 600, 638; MS (MALDI-TOF LD⁺): *m*/*z* (%): 711 (10) [*M*⁺], 671 (80) [M-80]. ¹⁹F (MeOD) (188 MHz): δ=-132.1 (br s, 2 F), -144.71 (s, I F), - 161.70 ppm (s, 2 F). The title product was crystallized by diffusion of diethyl ether into concentrated methanol solution, affording 58% yield.

### Example 22. Neuroprotection by corroles in vivo in the MPTP model of PD

The mouse model of Parkinson's disease (PD) using the neurotoxin 1-methyl-4-phenyl-1,2,3,6-tetrahydropyridine (MPTP) recapitulates many of the pathological manifestations encountered in human PD including increased generation of ROS and nitration of tyrosine residues on proteins in the substantia nigra (SN) of the ventral midbrain, a region containing the vulnerable DA neurons. Employing this model it was recently shown that rasagiline and M-30 (a propargyl-containing iron chelator disclosed in WO 2004/041151) are neuroprotective and also have the ability to restore nigral dopaminergic neurons when given post-MPTP (Gal et al., 2005). Based on our preliminary results (see above, **Figs. 3-20**) and on the somewhat larger lipophylicity of corrole **5C-Fe** (higher probability to penetrate the BBB), this was the first corrole tested. Mice are treated with corrole **5C-Fe** (1, 2, 10 and 20 mg/kg, once a day, by oral or intraperitoneal (i.p.) administration) for 5 consecutive days and followed by the combination with MPTP (24 mg/kg, i.p.) for 4 additional days. Monoamine oxidase (MAO) A/B activity is assessed in the striatum and hippocampus, as well as tyrosine hydroxylase (TH) activity and catecholamine levels in the striatum, hypothalamus and dorsal raphe. In addition, parkinsonism-associated proteins, such as α-synuclein, and synphilin-1, and iron regulatory proteins, such as ferritin, transferrin receptor, and hypoxia inducible factor (HIF)-1 are monitored for changes in striatal expression. To link the possible neuroprotective effects of the corrole with antioxidant actions, parameters of ROS and RNS including reduced glutathione (GSH), lipid peroxidation and 3-nitrosylated proteins are tested in the mouse ventral midbrain region, as described (Liang et al., 2007).

### Example 23. Neurorescue by corroles in the MPTP model

Our preliminary results have shown that corroles **5C-Fe** and **1C-Fe** possess not only preventive but also potent neurorescue activity (indicated by their post-damage administration) in cell cultures (see **Figs. 8**, 11, **14** and **15**). Therefore, corrole **5C-Fe** is the one initially investigated for its potential neurorescue/neurorestorative effect in post-MPTP-induced nigrostriatal dopamine neurodegeneration model of PD in mice. MPTP (20 mg/kg, i.p, per day) administration for 4 days is followed by a further 4 days resting period (day 8) to allow for the full conversion of MPTP to its active metabolite, MPP+ (Sagi et al., 2007). At day 8, corrole **5C-Fe** (doses are according to the neuroprotective paradigm results) is administered (i.p. or orally) for 14 days. The same parameters described for the neuroprotective studies in Example 22 are determined here as well.

### Example 24 Molecular effects of corroles in vitro on apoptosis, cell signaling, and OS related processes:

The molecular mechanism of cyotoprotective/cytorescue action of the various corroles is assessed in the following cell lines: clonal mouse-derived SN dopaminergic neuronal cell line, SN4741 (PD model), mouse motor neuronal cell line, NSC-34 (ALS model) and in rat insulinoma INS-1E and RIN-m cell lines (DM model). Apoptosis-associated protein markers (e.g. the Bcl-2 family proteins Bax, Bad, Bcl-2; cytochrome c; phospho-H2AX; cleaved caspase-3 and -8; cleaved poly ADP-ribose (PARP)), signaling cascades (e.g. MAPK, PKC, P13K/AKT) and OS parameters (catalase, GSH, glutathione peroxidase, endogenous ROS levels), are determined as described in earlier publications of the M. Youdim group (Weinreb et al., 2004; Bar-Am et al., 2005). Specifically for the insulinoma cell lines, the effect of the corroles against alloxan, a diabetogenic toxin which selectively kills the insulin-producing beta-cells of the pancreas, are investigated and both cellular and secreted insulin levels, are determined. Also, the levels of thioredoxin-interacting protein, a pro-apoptotic factor recently found to be increased in beta cell subjected to glucose toxicity, is measured (Chen et al., 2006).

### Example 25. Effect of corroles on mitochondrial function

Various corroles are examined with respect to their ability to prevent the collapsing of the mitochondria and consequent decrease in proteasomal activity (because of impairment in ATP supply) following exposure to 6-OHDA, a neurotoxin known to inhibit mitochondrial complexes I and IV. Mitochondrial membrane potential (ΔΨm) is determined by the JC-1 test. Briefly, the corroles are added 30 min before exposure to 6-OHDA (35 µM), for a period of 4 h. Changes in ΔΨm are determined by JC-1 mitochondrial membrane potential detection kit (Biotium, Inc) for 20 min at 37°C. Dual emission images (530 and 590 nm) represent the signal from monomeric (green) and J-aggregate (red) JC-1 fluorescence by confocal microscopy. At high-membrane potentials characteristic of energized mitochondria, JC-1 accumulates sufficiently to aggregate, resulting in large, orange (590 nm) shifts in the emission maximum. At lower potentials, the dye exists as a green fluorescent (530 nm) monomer).

### Example 26. Biodistribution and bioavailability of corroles

Issues regarding biodistribution, transport mechanisms, accumulation in specific organs, and the eventual elimination of administered compounds are among the prime concerns when it comes to real life utilization of potential drugs. This can be addressed by several and quite different means, including in vitro, in vivo and ex-vivo examinations. One way to predict distribution is via examination of the binding of the corroles to circulating proteins that might act as transporters, similar to experiment performed previously by the inventors with metal complexes of corrole **1C** (Scheme 1), which spontaneously form tightly bound non-covalent bioconjugates with HDL (Haber et al., 2007), albumin (Mahammed et al., 2004), and transferrin (Haber and Gross. 2007) (listed in increasing order of affinity), as well as with semi-synthetic proteins (Agadjanian et al., 2006). Preliminary experiments revealed that the metal complexes of corrole **2C** (such as **2C-Mn**) do not bind to albumin, but still do so for transferrin, one of the very few proteins that may carry drugs to the brain. A more direct approach relies on the strong fluorescence of corroles that are not chelated by transition metals (no metal such as in corroles 1C and 2C, or using corrole Ga/Al complexes such as **1C-Ga**). This feature is used for testing cell entry via confocal microscopy (Agadjanian et al., 2006), without the need of attaching a fluorescent tag. Furthermore, it may even be used for whole animal imaging purposes, as shown in Example 4 hereinabove. Positron Emission Tomography (PET) may also be used relying on copper corroles such as corrole **1C-Cu**. The half-life of ⁶⁴Cu is 12.7 hours, copper may be inserted into corroles within minutes at room temperature and the metal does not leach from the corrole once inserted.

Tissue distribution of the corroles is determined in various brain areas including the cortex, hippocampus and brain stem, as well as their levels in the periphery and in plasma at different time intervals (1-96 h), by means of HPLC and chemiluminescence. Importantly, we have already confirmed that nanomolar concentrations of complex **1C-Fe** can be determined in plasma samples via the luminol/H₂O₂ method (Motsenbocker et al., 1999).

### Example 27. The capacity of corroles to prevent development of streptozotocin (STZ)-induced diabetes in mice

Following the initial studies using the compounds for in-vitro protection of insulin-producing cells exposed to hydrogen peroxide or SIN-1 (converts into PN at physiological pH), the selected corroles are administered i.p. to mice (the doses are determined from the MPTP model results) for different time periods (1, 3 and 6 weeks) before, during and one week after administration of the diabetogenic-inducing toxin, STZ, which selectively kills the insulin-producing beta-cells in the pancreas. The following experimental groups are studied.

### Diabetes induction and side effects monitoring

Diabetes in Balb/c mice (Harlan Laboratories, Jerusalem) is induced by a single injection of STZ solution (Sigma, St.Louis, MO) at a dose of 200 mg/kg body mass or by low multiple doses injections (40 mg/kg x 5 days). STZ is injected i.p. after dissolving in citric buffer (pH 4.5). Diabetes severity is estimated by blood and urine glucose monitoring, body weight, as well as by the glucose tolerance test. All animals treated with the corroles are occasionally monitored for possible side effects by blood sampling for biochemical, haematological profiles. These studies include two different groups: mice with high dose STZ diabetes and mice with multiple low doses STZ diabetes, for examining the effects of the corroles on direct toxic beta cell destruction and on autoimmune-like beta cell destruction, respectively.

### Example 28. Potential therapeutic utility of corroles in APP/presenilin-1 (PSI), double transgenic mice model of Alzheimer's disease (AD)

To address the possible neuroprotective role of the corroles on the pathogenesis of AD, their effects are examined on the regulation/processing mechanisms of amyloid precursor protein (APP) (e.g. APP mRNA/ protein, soluble α/βAPP, fibrillar Aβ peptides/plaque deposits) in the cerebral cortex and hippocampus of the doubly transgenic AD, APP/PS1 mice. Daily gavage dosage is given for 4 consecutive weeks before symptoms appearance (∼6 months of age). After drug administration is completed, 5-6 animals from each group are sacrificed; their brains are dissected and stored at -70 °C for further analysis.

### Example 29. Potential therapeutic utility of corroles in ALS transgenic mice:

Hemizygous SOD transgenic mice (carrying a high copy number of a mutant allele human SOD1 containing the Gly93 -->Ala (G93A) substitution) become paralyzed in one or more limbs at late disease stage, with a lifespan of ∼130 days. Selected corroles are administered by daily gavage beginning at an asymptomatic state (day 70) until death. Motor function, lifespan and post-mortem histopathological analysis are assessed.

### Example 30. Bifunctional propargyl-corroles: MAO-A/B inhibition and antidepressant potential

Several hybrid N-propargyl-corroles (Scheme 2) are initially examined in-vitro for their properties as MAO inhibitors. The compounds exhibiting highest potencies are selected for further investigation. Mice are administered with the propargyl-corroles (1-10 mg/kg, i.p.) and sacrificed 1h later to assess *ex-vivo* inhibition of MAO A/B activity in various brain regions and systemic organs. In addition to evaluation of MAO inhibitory potency, these findings provide an indication regarding brain permeability of the examined drugs. Assessment of the levels of striatal amines (DA, DOPAC, HVA, NE, 5-HT, and 5-HIAA) are performed as well, to corroborate findings from the MAO inhibition assay (Gal et al., 2005). Propargyl-corroles displaying ability to inhibit MAO-A in the brain are examined for their potency in ameliorating depression. This is performed using the forced swim test model, a specific model of behavioral. despair and depression, as previously described (Weinstock et al., 2002). Rats are administered propargyl-corroles (5 or 10 mg/kg) and assessed for behavior characteristics typical of depression.

Non-selective MAO inhibitors combined with diet-derived tyramine ingestion may lead to what is commonly termed "the cheese reaction", characterized by life-threatening hypertension, resulting from elevated synapse norepinephrin levels (Youdim and Weinstock, 2004). Due to this side effect, the usage of MAO inhibitors has been limited. Since the predominant MAO isoenzyme in the small intestine is MAO-A, the development of novel MAO-inhibitors has hitherto focused on selective MAO-B inhibition. The propargyl-corroles are examined whether they produce or not such hypertensive effect. This assumption is based on our findings showing that the neuroprotective iron chelator-propargyl containing, M-30, potently inhibits both MAO-A and -B in the brain, but poorly in peripheral organs (Gal et al., 2005). To this end, rats are administered propargyl-corroles (5 and 10 mg/kg, i.p.), prior to oral tyramine administration (Weinstock et al., 2002), and then blood pressure and heart rate is determined. MAO-A and MAO-B inhibition in the brain, liver and intestine is determined as well.

Finally, based on the positive outcome of the above studies, the selected bifunctional corroles are tested for their cyto/neuroprotective properties in the various disease models.

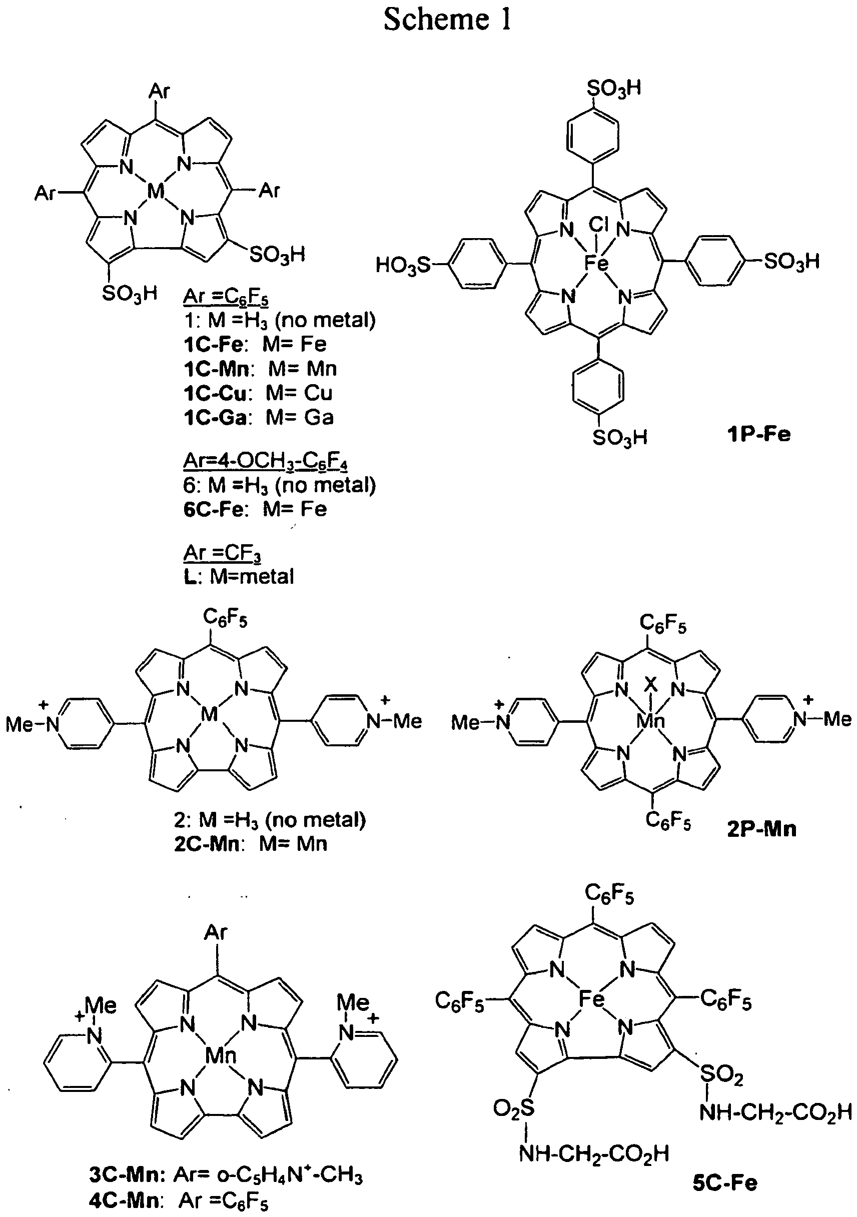

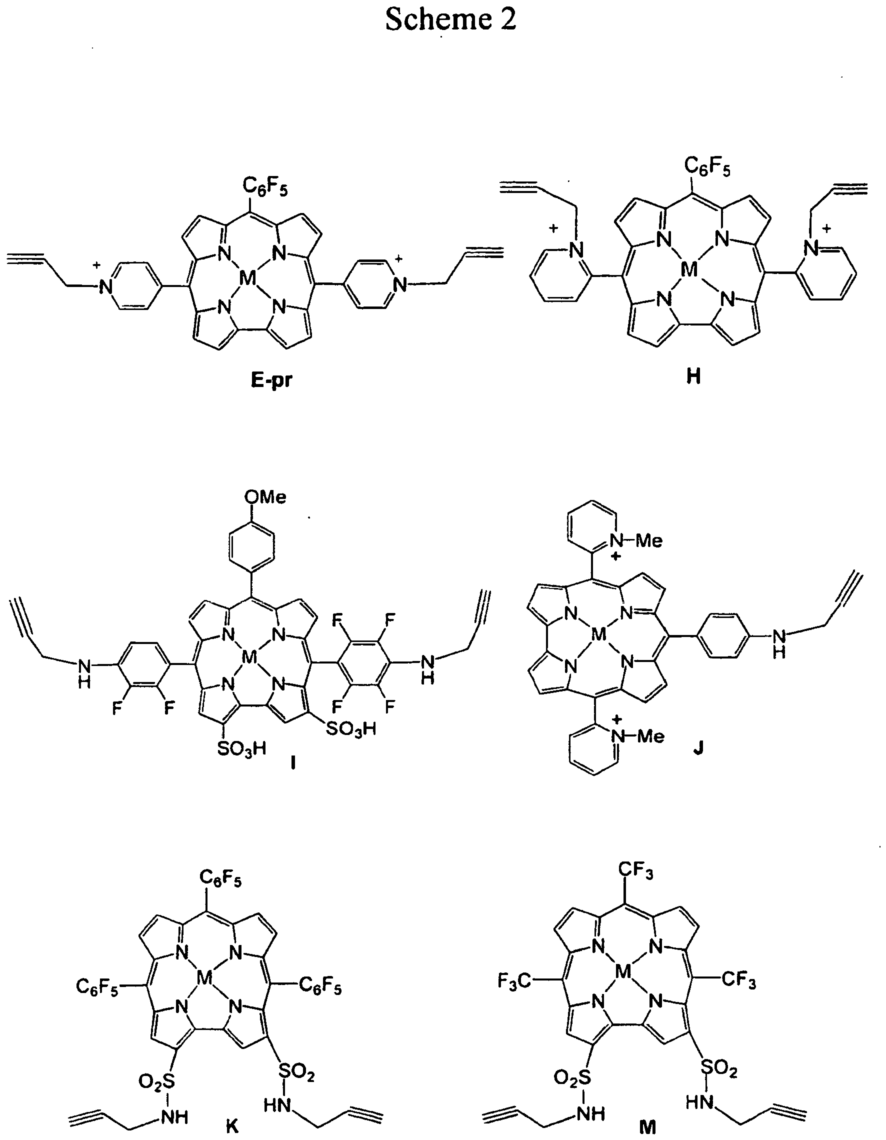

### REFERENCES

Agadjanian H, Weaver J J, Mahammed A, Rentsendorj A, Bass S, Kim J, Dmochowski I J, Margalit R, Gray H B, Gross Z and L. K. Medina-Kauwe. (2006). Specific Delivery of Corroles to Cells via Noncovalent Conjugates with Viral Proteins. Pharmaceutical Res. 23:367-377.
Aviv I, Gross Z. (2007). Corrole-based applications. Chem Commun (Camb).(20):1987-99.
Avramovich-Tirosh Y, Amit T, Bar-Am O, Zheng H, Fridkin M, Youdim MB (2007). Therapeutic targets and potential of the novel brain- permeable multifunctional iron chelator monoamine oxidase inhibitor drug, M-30, for the treatment of Alzheimer's disease. J Neurochem. 100: 490-502.
Bar-Am O, Weinreb O, Amit T and Youdim M B (2005). Regulation of Bcl-2 family proteins, neurotrophic factors, and APP processing in the neurorescue activity of propargylamine. FASEB J. 19:1899-901.
Barbe, J.M., Canard, G., Brandes, S. & Guilrad, R (2005). Synthesis and physicochemical characterization of meso-functionalized corroles: Precursors of organic-inorganic hybrid materials. Eur J Org Chem. 21:4601-4611.
Bamham KJ, Masters CL & Bush AI (2004). Neurodegenerative diseases and oxidative stress. Nat Rev Drug Discov. 3:205-214
Batini'c-Haberle I, Spasojevi'c I, Stevens RD, Bondurant B, Okado-Matsumoto A, Fridovich I, Vujas 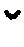 kovic Z. and Dewhirst MW (2006). New PEG-ylated Mn(III) porphyrins approaching catalytic activity of SOD enzyme. Dalton Trans. 617-624.
Chen J, Couto FM, Minn AH, Shalev A (2006). Exenatide inhibits beta-cell apoptosis by decreasing thioredoxin-interacting protein. Biochem. Biophys. Res. Commun 346:1067-74.
Fisher AEO, Hague TA, Clarke CL and Naughton DP. (2004). Catalytic superoxide scavenging by metal complexes of the calcium chelator EGTA and contrast agent EHPG. Biochem. Biophys. Res. Commun. 323:163-167.
Gal S, Zheng H, Fridkin M, Youdim MB (2005). Novel multifunctional neuroprotective iron chelator-monoamine oxidase inhibitor drugs for neurodegenerative diseases. In vivo selective brain monoamine oxidase inhibition and prevention of MPTP-induced striatal dopamine depletion. J. Neurochem. 95:79-88.
Gershman, Z., Goldberg, I. & Gross, Z (2007). DNA binding and catalytic properties of positively-charged corroles. Angew. Chem Int Ed Engl 46:4320-4324.
Goldschmidt R, Goldberg I, Balazs Y and Gross Z (2006). Synthesis of a Corrole with Small and Electron-withdrawing Substitutents, 5,15-bistrifluromethyl-10-pentafluorophenylcorrole. J Porphyrins Phthalocyanines 10:76-86.
Goldstein S., Lind J. & Merenyi G (2005). Chemistry of Peroxynitrites as Compared to Peroxynitrates. Chem Rev 105:2457-2470.
the mouse, Biochem J. 199: 393-398.
Gross Z, Galili N, and Saltsman I (1999). The First Direct Synthesis of Corroles from Pyrrole, Angew. Chem. Int. Ed. Eng. 38:1427-9.
Gryko DT. (2002) Recent advances in the synthesis of corroles and core-modified corroles Eur J Org Chem 11, 1735-1743.
GrykoD.T. and Piechota K.E.(2002) Straightforward route to trans-A2B-corroles bearing substituents with basic nitrogen atoms. J. Porphyrins Phthalocyanines, 6:81.
Gryko DT, Fox JP & Goldberg DP (2004). Recent advances in the chemistry of corroles and core-modified corroles. J. Porph. Phthal. 8:1091-1105.
Haber A., Agadjanian h, Medina-Kauwe LK, and Zeev Gross (2007).. Corroles Bind with High Affinity to both Apo and Holo Transferrin. J Inorg Biochem, 102:446.
Haber A, Mahammed A, Fuhrman B, Volkova N, Coleman R, Hayek T, Aviram M and Zeev Gross (2007). Amphiphilic corrole metal complexes as multifunctional agents for attenuation of atherosclerosis. Angew. Chem. Int. Ed. 47:7896.
Han J, Cheng F C, Yang Z, Dryhurst G (1999). Inhibitors of mitochondrial respiration, iron (II), and hydroxyl radical evoke release and extracellular hydrolysis of glutathione in rat striatum and substantia nigra: potential implications to Parkinson's disease. J Neurochem. 73(4): 1683-95.
Liang, L.P., Huang, J., Fulton, R., Day, B.J. & Patel, M (2007). An orally active catalytic metalloporphyrin protects against 1-methyl-4-phenyl-1,2,3,6-tetrahydropyridine neurotoxicity in vivo. J. Neurosci. 27, 4326-4333.
Mahammed A, Goldberg I & Gross, Z (2001). Highly selective chlorosulfonation of tris(pentafluorophenyl)corrole as a synthetic tool for the preparation of amphiphilic corroles and metal complexes of chiral planarity, Org. Lett. 3: 3443-3446.
Mahammed A, Gray H B, Weaver J J, Sorasaenee K, and Gross Z. (2004). Amphiphilic Corroles Bind Tightly to Human Serum Albumin" Bioconj Chem. 15:738-746.
Mahammed, A. and Gross, Z. (2005) Albumin-conjugated corrole metal complexes: extremely simple yet very efficient biomimetic oxidation systems. J Am Chem Soc 127, 2883-7.
Mahammed, A. & Gross, Z (2006). Iron and manganese corroles are potent catalysts for the decomposition of peroxynitrite. Angew. Chem In. Ed Engl 45:6544-6547.
Mandel S, Weinreb O, Youdim M B H (2003). Using cDNA microarray to assess Parkinson's disease models and the effects of neuroprotective drugs. Trends Pharmacol Sci.; 24(4): 184-91.
Mandel S, Amit T, Kalfon L, Youdim MB (2007a). Applying transcriptomic and proteomic knowledge to Parkinson's disease drug discovery. Expert opinion drug discov. 2:1225-40.
Mandel S, Amit T, Bar-Am O, Youdim MB (2007b). Iron disregulation in Alzheimer's disease: multimodal brain permeable iron chelating drugs, possessing neuroprotective-neurorescue and amyloid precursor protein-processing regulatory activities as therapeutic agents. Prog Neurobiol. 82:348-60.
Mohiuddin I, Chai H, Lin PH, Lumsden AB, Yao QZ, Chen CY (2006). Nitrotyrosine and chlorotyrosine: Clinical significance and biological functions in the vascular system J Surg Res. 133:143-149.
Motsenbocker M, Ichimori Y and Kondo K (1999). Metal Porphyrin Chemiluminescence Reaction and Application to Immunoassay. Anal. Chem. 65:397-402.
Olmos A, Giner RM, Manez S (2007). Drugs modulating the biological effects of peroxynitrite and related nitrogen species. Med. Res. Rev. 27:1-64.
Peng J., Peng L., Stevenson F. F., Doctrow S. R., and. Andersen J. K (2007). Iron and Paraquat as Synergistic Environmental Risk Factors in Sporadic Parkinson's Disease Accelerate Age-Related Neurodegeneration. J. Neurosc., 27: 6914-6922.
Rha SY, Izbicka E, Lawrence R, Davidson K, Sun D, Moyer MP, Roodman GD, Hurley L, Von Hoff D. (2000). Effect of telomere and telomerase interactive agents on human tumor and normal cell lines. Clin. Cancer. Res. 6:987-993.
Rohrer L, Hersberger M, von Eckardstein A (2004). High density lipoproteins in the intersection of diabetes mellitus, inflammation and cardiovascular disease. Curr. Opinion Lipidology 15 (3): 269-278.
Sagi, Y. Mandel, S. Amit, T. and Youdim (2007). Activation of tyrosine kinase receptor signaling pathway by rasagiline facilitates neurorescue and restoration of nigrostriatal dopamine neurons in post-MPTP-induced parkinsonism. Neurobiol Dis. 25:35-44.
Saltsman I, Goldberg I & Gross Z (2002) One-step conversions of a simple corrole into chiral and amphiphilic derivatives", Tetrahedron Lett. 44, 5669-5673.
Saltsman I, Mahammed A, Goldberg I, Tkachenko E, Botoshansky M, Gross Z (2003). Selective substitution of corroles: Nitration, hydroformylation, and chlorosulfonation. J. Am. Chem. Soc. 124:7411-7420.
Saltsman I, Botoshansky M. and Gross Z. (2008) Facile synthesis of ortho-pyridyl-substituted corroles and molecular structures of analogous porphyrins. Tet. Lett, 49:4163
Schlieve, C.R., Lieven, C.J. & Levin, L.A (2006). Biochemical activity of reactive oxygen species scavengers do not predict retinal ganglion cell survival. Invest. Ophthalmol. Vis. Sci. 47:3878-3886.
Shah S. Iqbal M, Karam J, Salifu M, McFarlane SI (2007). Oxidative stress, glucose metabolism, and prevention of type 2 diabetes: pathophysiological insights. Antioxid. Redox Signal. 9:911-929 and references therein.
Szabó C, Ischiropoulos H & Radi R (2007). Peroxynitrite: biochemistry, pathophysiology and development of therapeutics. Nat. Rev. Drug Discov. 6:662-680.
Van der Schyf, C. J., Geldenhuys, W. J. and Youdim, M. B. (2006) Multifunctional drugs with different CNS targets for neuropsychiatric disorders. J. Neurochem. 99, 1033-48.
Weinreb O, Bar-Am O, Amit T, Chillag-Talmor O and Youdim MBH. (2004) Neuroprotection via pro-survival protein kinase C isoforms associated with Bcl-2 family members. FASEB J. 18:1471-3.
Weinstock M, Gorodetsky E, Wang RH, Gross A, Weinreb O, Youdim MB (2002) Limited potentiation of blood pressure response to oral tyramine by brain-selective monoamine oxidase A-B inhibitor, TV-3326 in conscious rabbits. Neuropharmacology 43(6):999-1005.
Wu AS, Kiaei M, Aguirre N, Crow JP, Calingasan NY, Browne SE, Beal MF (2003). Iron porphyrin treatment extends survival in a transgenic animal model of amyotrophic lateral sclerosis. J Neurochem 85: 142-150.
Yogev-Falach M, Amit T, Bar-AM O, Youdim M B H (2003). The importance of propargylamine moiety in the anti-Parkinson drug rasagiline and its derivatives for MAPK-dependent amyloid precursor protein processing. FASEB J. 17:(15): 2325-7.
Youdim M B H, Grunblatt E, Mandel S (1999). The pivotal role of iron in NF-kappa B activation and nigrostriatal dopaminergic neurodegeneration. Prospects for neuroprotection in Parkinson's disease with iron chelators. Ann N Y Acad Sci.; 890:7-25.
Youdim M B (2003). Rasagiline: an anti-Parkinson drug with neuroprotective activity. Expert Rev Neurotherapeutics.; 3:(6): 737-749.
Youdim MB, Weinstock M (2004). Therapeutic applications of selective and non-selective inhibitors of monoamine oxidase A and B that do not cause significant tyramine potentiation. Neurotoxicology 25, 243-50.
Youdim MB, Buccafusco JJ (2005). Multi-functional drugs for various CNS targets in the treatment of neurodegenerative disorders. Trends Pharmacol. Sci.; 26: 27-35.

## Claims

1. A transition metal complex of an amphiphilic corrole, an optical isomer or a pharmaceutically acceptable salt thereof for use in neuroprotection and neurorescue.

2. The complex according to claim 1, for the treatment of diabetes or of a neurodegenerative disease, disorder or condition.

3. The complex according to claim 1 or 2, wherein said amphiphilic corrole is a 5,10,15-tris-aryl- or 5,10,15- tris-CF₃-corrole.

4. The complex according to claim 3, wherein said transition metal complex of the amphiphilic corrole has the formula I: 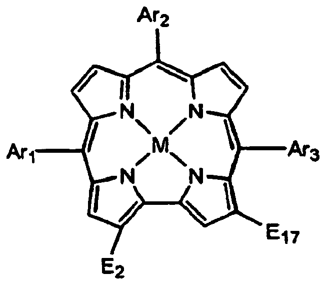 wherein:
Ar₁, Ar₂ and Ar₃, the same or different, each is selected from CF₃, carboaryl, heteroaryl or mixed carboaryl-heteroaryl;
M is a transition metal selected from Mn, Fe, Ru, Co, V, Cr, or Cu; and
E₂ and E₁₇, the same or different, each is H, SO₃H, SO₂N-R₁R₂, CO₂H, CO₂R or CON-R₁R₂; R is C₁-C₈ alkyl or aryl; and R₁ and R₂, the same or different, each is H, C₁-C₈ alkyl optionally substituted by -COOH, C₂-C₈ alkynyl, C₆-C₁₂ aryl or together with the N atom to which they are attached form a saturated 5-6 membered ring optionally containing a further heteroatom selected from O, S and N.

5. The complex according to claim 4, wherein the carboaryl, by itself or as part of the mixed carboaryl-heteroaryl radical, is a monocyclic or bicyclic C₆-C₁₂ aromatic radical such as phenyl, biphenyl or naphthyl optionally mono- or poly-substituted by one or more halogen atoms, or by C₁-C₈, preferably C₁-C₄, alkyl or alkoxy, nitro, hydroxyl, SO₃H, -NR₁R₂, -N⁺R₁R₂R₃, or -N-R₁-NH₂, wherein R₁, R₂ and R₃, the same or different, each is H, C₁-C₈ alkyl, C₂-C₈, preferably C₃, alkynyl, C₆-C₁₂ aryl, C₆-C₁₂aryl-C₁-C₈ alkyl or R₁ and R₂ together with the N atom to which they are attached form a saturated 5-6 membered ring optionally containing a further heteroatom selected from O, S and N; said heteroaryl, by itself or as part of the mixed carboaryl-heteroaryl radical, is a 5-6 membered aromatic ring containing 1-3 heteroatoms selected from O, S and N such as pyrrolyl, furyl, thienyl, imidazolyl, pirazolyl, oxazolyl, thiazolyl, pyridyl, pirazinyl, pyrimidinyl, 1,3,4-triazinyl, 1,2,3-triazinyl, optionally substituted as defined above for the carboaryl, and when the heteroaryl has a N atom in the ring, it may be substituted at the N atom, preferably by an alkyl or alkynyl group, preferably propargyl; and said mixed carboaryl-heteroaryl is a radical derived from a carboaryl and a heteroaryl radical condensed to each other such as benzofuryl, isobenzofuryl, indolyl, benzimidazolyl, benzthiazolyl, benzoxazolyl, quinoline, or isoquinoline, or covalently linked to each other such as pyridilium-phenyl, optionally substituted as defined above either in the carbocyclic, heterocyclic, or in both rings.

6. The complex according to claim 5, wherein the carboaryl is a phenyl radical monosubstituted by propargylamino or methoxy, preferably at position 4, or it is polysubstituted, by halogen atoms, more preferably chloro or fluoro atoms, sulfo, propargylamino, alkoxy, preferably methoxy, aminoalkylamino, and trialkylammonium, and the heteroaryl is pyridine substituted at the N atom by C₁-C₄ alkyl, preferably methyl, or propargyl.

7. The complex according to claim 6, wherein the carboaryl is 2,6-dichlorophenyl, 2,6-difluorophenyl, pentafluorophenyl, 4-methoxy-2,3,5,6-tetrafluorophenyl, 4-sulfophenyl, 4-methoxyphenyl, 4-N-propargylamino-2,3,5,6-tetrafluorophenyl, or 4-N-propargylamino-phenyl; the heteroaryl is 4-(N-methyl)-pyridylium, 2-(N-methyl)-pyridylium, 4-(N-propargyl)-pyridylium, or 2-(N-propargyl)-pyridylium; and the carboaryl-heteroaryl is 4-(pyridyl)-2,3,5,6-tetrafluorophenyl, 4-(N-methyl-pyridylium)-2,3,5,6-tetrafluorophenyl, 4-(N-propargyl-pyridylium)-2,3,5,6-tetrafluorophenyl, 2-(N-propargyl-pyridylium)-2,3,5,6-tetrafluorophenyl.

8. The complex according to claim 7, wherein:
(i) Ar₁, Ar₂ and Ar₃ are the same and each is CF₃, pentafluorophenyl, 4-methoxy-2,3,5,6-tetrafluorophenyl, 4-sulpho-phenyl, 4-(N-methyl)-pyridylium, 2-(N-methyl)-pyridylium, 4-(N-propargyl)-pyridylium, or 2-(N-propargyl)-pyridylium;
(ii) Ar₁ and Ar₃ each is 4-(N-methyl)-pyridylium and Ar₂ is pentafluorophenyl; or Ar₁ and Ar₃ each is 4-N-propargylamino-2,3,5,6-tetrafluorophenyl and Ar₂ is 4-methoxyphenyl; or Ar₁ and Ar₃ each is 4-(N-propargyl)-pyridylium and Ar₂ is pentafluorophenyl; or Ar₁ and Ar₃ each is 2-(N-propargyl)-pyridylium and Ar₂ is pentafluorophenyl; or Ar₁ and Ar₃ each is 2-(N-methyl)-pyridylium and Ar₂ is 4-N-propargylaminophenyl; or
(iii) E₂ and E₁₇ are the same and each is H, SO₃H, SO₂NH-propargyl or SO₂NH-CH₂-COOH.

9. The complex according to any of claims 1-8, wherein the metal M is Fe or Mn.

10. The complex according to claim 9, wherein said corrole metal complex is selected from:
(i) the corroles in which E₂ and E₁₇ are both SO₃H, Ar₁, Ar₂ and Ar₃ each is pentafluorophenyl, and M is Fe herein designated corrole **1C-Fe**, Mn herein designated corrole **1C- Mn**, or Cu herein designated corrole **1C-Cu**; or Ar₁, Ar₂ and Ar₃ each is CF₃ and M is Fe or Mn;
(ii) the corrole in which E₂ and E₁₇ are both SO₃H, Ar₁, Ar₂ and Ar₃ each is 4-methoxy-2,3,5,6,-tetrafluorophenyl, and M is Fe, herein designated corrole **6C-Fe**,
(iii) the corrole in which E₂ and E₁₇ are both SO₂NH-CH₂-COOH, Ar₁, Ar₂ and Ar₃ each is pentafluorophenyl, and M is Fe, herein designated corrole **5C-Fe**;
(iv) the corrole in which E₂ and E₁₇ are both H, Ar₁ and Ar₃ each is 4-(N-methyl)-pyridylium, Ar₂ is pentafluorophenyl, and M is Mn, herein designated corrole **2C-Mn**;
(v) the corrole in which E₂ and E₁₇ are both H, Ar₁, Ar₂ and Ar₃ each is 2-(N-methyl)-pyridylium, and M is Mn, herein designated corrole **3C-Mn**;
(vi) the corrole in which E₂ and E₁₇ are both H, Ar₁ and Ar₃ each is 2-(N-methyl)-pyridylium, Ar₂ is pentafluorophenyl, and M is Mn, herein designated corrole **4C-Mn**; and
(vii) the corroles in which E₂ and E₁₇ are both SO₃H, Ar₁, Ar₂ and Ar₃ each is CF₃, and M is Mn or Fe, herein designated corrole **M-Mn** or **H-Fe,** respectively,
(viii) the corroles in which E₂ and E₁₇ are both H, Ar₁ and Ar₃ each is 4-(N-propargyl)-pyridylium, Ar₂ is pentafluorophenyl and M is Mn or Fe, herein designated corrole **E-pr-Mn** or **E-pr-Fe,** respectively,
(ix) the corrole in which E₂ and E₁₇ are both H, Ar₁ and Ar₃ each is 2-(N-propargyl)-pyridylium, Ar₂ is pentafluorophenyl, and M is Mn or Fe, herein designated corrole **H-Mn** or **H-Fe,** respectively;
(x) the corroles in which E₂ and E₁₇ are both SO₃H, Ar₁ and Ar₃ each is 4-N-propargylamino-2,3,5,6-tetrafluorophenyl and Ar₂ is 4-methoxyphenyl, and M is Mn or Fe, herein designated corrole **I-Mn** or **I-Fe,** respectively;
(xi) the corrole in which E₂ and E₁₇ are both H, Ar₁ and Ar₃ each is 2-(N-methyl)-pyridylium, Ar₂ is 4-propargylamino-phenyl, and M is Mn or Fe, herein designated corrole **J-Mn** or **J-Fe,** respectively;
(xii) the corroles in which E₂ and E₁₇ are both -SO₂-NH-propargyl, Ar₁, Ar₂ and Ar₃ each is pentafluorophenyl, and M is Mn or Fe, herein designated corrole **K-Mn** or **K-Fe,** respectively, and
(xiii) the corroles in which E₂ and E₁₇ are both -SO₂-NH-propargyl, Ar₁, Ar₂ and Ar₃ each is CF₃, and M is Mn or Fe, herein designated corrole **M-Mn** or **M-Fe,** respectively.

11. A pharmaceutical composition for use in neuroprotection and neurorescue, particularly for treatment of diabetes and neurodegenerative diseases, disorders or conditions, comprising a pharmaceutically acceptable carrier and a metal complex of an amphiphilic corrole, an optical isomer or a pharmaceutically acceptable salt thereof, preferably of formula I shown in claim 4.

12. A propargyl-containing corrole carrying one or more radicals substituted by a propargylamino group or one or more nitrogen-containing heteroaryl radicals substituted by propargyl at the ring N atom.

13. The propargyl-containing corrole according to claim 12, which is a transition metal complex of an amphiphilic corrole of the formula I as defined in claim 4, wherein either: (i) at least one of Ar₁, Ar₂ and Ar₃ is a carboaryl radical substituted by a -NR₁R₂ group, wherein R₁ is H and R₂ is propargyl, or a N-heteroaryl radical substituted at the ring N atom by propargyl; or (ii) at least one of E₂ and E₁₇ is - CONR₁R₂ or -SO₂N-R₁R₂, wherein R₁ is H and R₂ is propargyl.

14. The propargyl-containing corrole according to claim 13, wherein E₂ and E₁₇ each is SO₂N-R₁R₂, wherein R₁ is H and R₂ is propargyl, or at position 5, 10 and/or 15, Ar₁, Ar₂ and/or Ar₃ is a phenyl radical solely substituted by a propargylamino group, preferably at position 4, or by a propargylamino group and other substituents such as halogen, preferably fluoro, or Ar₁, Ar₂ and/or Ar₃ is a pyridyl radical substituted by propargyl at the ring N atom.

15. The propargyl-containing corrole according to claim 14, wherein the propargyl-containing groups are selected from 4-(N-propargyl)-pyridylium, 2-(N-propargyl)-pyridylium, 4-N-propargylaminophenyl and 4-N-propargylamino-2,3,5,6-tetrafluorophenyl at positions 5, 10 and/ or 15 of the corrole or -SO₂-NH-propargyl at positions 2 and 17 of the corrole, and the metal M is Fe or Mn.

16. The propargyl-containing corrole according to claim 15, selected from:
(a) the corroles in which E₂ and E₁₇ are both H, Ar₁ and Ar₃ each is 4-(N-propargyl)-pyridylium, Ar₂ is pentafluorophenyl and M is Mn or Fe, herein designated corrole **E-pr-Mn** or **E-pr-Fe,** respectively;
(b) the corrole in which E₂ and E₁₇ are both H, Ar₁ and Ar₃ each is 2-(N-propargyl)-pyridylium, Ar₂ is pentafluorophenyl, and M is Mn or Fe, herein designated corrole **H-Mn** or **H-Fe,** respectively;
(c) the corroles in which E₂ and E₁₇ are both SO₃H, Ar₁ and Ar₃ each is 4-N-propargylamino-2,3,5,6-tetrafluorophenyl and Ar₂ is 4-methoxyphenyl, and M is Mn or Fe, herein designated corrole **I-Mn** or **I-Fe,** respectively;
(d) the corrole in which E₂ and E₁₇ are both H, Ar₁ and Ar₃ each is 2-(N-methyl)-pyridylium, Ar₂ is 4-propargylamino-phenyl, and M is Mn or Fe, herein designated corrole **J-Mn** or **J-Fe,** respectively;
(e) the corroles in which E₂ and E₁₇ are both -SO₂-NH-propargyl, Ar₁, Ar₂ and Ar₃ each is pentafluorophenyl, and M is Mn or Fe, herein designated corrole **K-Mn** or **K-Fe,** respectively; and
(f) the corroles in which E₂ and E₁₇ are both -SO₂-NH-propargyl, Ar₁, Ar₂ and Ar₃ each is CF₃, and M is Mn or Fe, herein designated corrole **M-Mn** or **M-Fe,** respectively.

17. A pharmaceutical composition comprising a propargyl-containing corrole according to any of claims 12 to 16, a pharmaceutically acceptable salt or an optical isomer thereof, and a pharmaceutically acceptable carrier.

18. The metal complexes of amphiphilic corroles of the formula I as defined in claim 4, wherein:
(i) E₂ and E₁₇ are both H, Ar₁, Ar₂ and Ar₃ each is 2-(N-methyl)-pyridylium, and M is Mn, herein designated corrole **3C-Mn**;
(ii) E₂ and E₁₇ are both H, Ar₁ and Ar₃ each is 2-(N-methyl)-pyridylium, Ar₂ is pentafluorophenyl, and M is Mn, herein designated corrole **4C-M**n;
(iii) E₂ and E₁₇ are both SO₂NH-CH₂-COOH, Ar₁, Ar₂ and Ar₃ each is pentafluorophenyl, and M is Fe, herein designated corrole **5C-Fe**; or
(iv) E₂ and E₁₇ are both SO₃H, Ar₁, Ar₂ and Ar₃ each is 4-methoxy-2,3,5,6,-tetrafluorophenyl, and M is Fe, herein designated corrole **6C-Fe**.

## Patentansprüche

1. Übergangsmetallkomplex mit einem amphiphilen Corrol, einem optischen Isomer oder einem pharmazeutisch anerkannten Salz davon zur Verwendung für Neuroprotektion und Neurorescue (Rettung von Nervenzellen).

2. Komplex nach Anspruch 1 für die Behandlung von Diabetes oder einer neurodegenerativen Erkrankung oder Störung oder eines neurodegenerativen Zustands.

3. Komplex nach Anspruch 1 oder 2, wobei genanntes amphiphiles Corrol ein 5,10,15-Tris-aryl- oder 5,10,15-Tris-CF3-Corrol ist.

4. Komplex nach Anspruch 3, wobei genannter Übergangsmetallkomplex mit amphiphilem Corrol die Formel I aufweist: 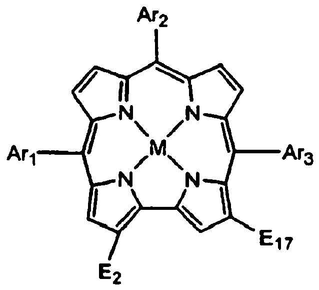 wobei:
Ar₁, Ar₂ und Ar₃ gleich oder verschieden voneinander sind und jeweils aus CF₃, Carboaryl, Heteroaryl oder einem gemischten Carboaryl-Heteroaryl ausgewählt sind;
M ein Übergangsmetall ist, das aus Mn, Fe, Ru, Co, V, Cr oder Cu ausgewählt ist; und
E₂ und E₁₇ gleich oder verschieden voneinander sind und jeweils H, SO₃H, SO₂N-R₁R₂, CO₂H, CO₂R oder CON-R₁R₂ sind; R C₁-C₈-Alkyl oder -Aryl ist und R₁ und R₂ gleich oder verschieden voneinander sind und jeweils H, C₁-C₈-Alkyl optional substituiert durch -COOH, C₂-C₈-Alkynyl, C₆-C₁₂-Aryl sind oder zusammen mit dem N-Atom, an welches sie gebunden sind, einen gesättigten 5-6-gliedrigen Ring bilden, optional enthaltend ein weiteres Heteroatom ausgewählt aus O, S und N.

5. Komplex nach Anspruch 4, wobei das Carboaryl selbst oder als Bestandteil des gemischten Carboaryl-Heteroaryl-Radikals ein monozyklisches oder bizyklisches C₆-C₁₂ aromatisches Radikal, wie beispielsweise Phenyl, Biphenyl oder Naphthyl, optional mono- oder polysubstituiert durch ein oder mehrere Halogenatom(e) oder durch C₁-C₈-, vorzugsweise C₁-C₄-Alkyl oder -Alkoxy, Nitro, Hydroxyl, SO₃H, -NR₁R₂, -N⁺R₁R₂R₃ oder -N-R₁-NH₂ ist, wobei R₁, R₂ und R₃ gleich oder verschieden voneinander sind und jeweils H, C₁-C₈-Alkyl, C₂-C₈-, vorzugsweise C₃-Alkynyl, C₆-C₁₂-Aryl, C₆-C₁₂-Aryl-C₁-C₈-Alkyl sind, oder R₁ und R₂ zusammen mit dem N-Atom, an welches sie gebunden sind, einen gesättigten 5-6-gliedrigen Ring bilden, optional enthaltend ein weiteres Heteroatom ausgewählt aus O, S und N; genanntes Heteroaryl selbst oder als Bestandteil des gemischten Carboaryl-Heteroaryl-Radikals ein 5-6-gliedriger aromatischer Ring ist, enthaltend 1-3 Heteroatome ausgewählt aus O, S und N, wie beispielsweise Pyrrolyl, Furyl, Thienyl, Imidazolyl, Pirazolyl, Oxazolyl, Thiazolyl, Pyridyl, Pirazinyl, Pyrimidinyl, 1,3,4-Triazinyl, 1,2,3-Triazinyl optional substituiert, wie oben für das Carboaryl definiert, und wenn das Heteroaryl ein N-Atom in dem Ring aufweist, dieses an dem N-Atom substituiert sein kann, vorzugsweise durch eine Alkyl- oder Alkynylgruppe, vorzugsweise Propargyl; und genanntes gemischtes Carboaryl-Heteroaryl ein Radikal ist, abgeleitet von einem Carboaryl- und einem Heteroaryl-Radikal, welche miteinander kondensiert sind, wie Benzofuryl, Isobenzofuryl, Indolyl, Benzimidazolyl, Benzthiazolyl, Benzoxazolyl, Chinolin oder Isochinolin, oder kovalent miteinander verknüpft sind, wie beispielsweise Pyridilium-phenyl optional substituiert, wie oben definiert, entweder im carbozyklischen oder heterozyklischen Ring oder in beiden Ringen.

6. Komplex nach Anspruch 5, wobei das Carboaryl ein Phenylradikal ist, welches vorzugsweise an Position 4 durch Propargylamino oder Methoxy monosubstituiert ist, oder durch Halogenatome, besonders bevorzugt Chlor-oder Fluoratome, Sulfo, Propargylamino, Alkoxy, vorzugsweise Methoxy, Aminoalkylamino und Trialkylammonium polysubstituiert ist, und das Heteroaryl Pyridin ist, welches an dem N-Atom durch C₁-C₄-Alkyl, vorzugsweise Methyl oder Propargyl, substituiert ist.

7. Komplex nach Anspruch 6, wobei das Carboaryl 2,6-Dichlorphenyl, 2,6-Difluorphenyl, Pentafluorphenyl, 4-Methoxy-2,3,5,6-Tetrafluorphenyl, 4-Sulfophenyl, 4-Methoxyphenyl, 4-N-Propargylamino-2,3,5,6-tetrafluorphenyl oder 4-N-Propargylamino-phenyl ist; das Heteroaryl 4-(N-Methyl)-pyridylium, 2-(N-Methyl)-pyridylium, 4-(N-Propargyl)-pyridylium oder 2-(N-Propargyl)-pyridylium ist; und das Carboaryl-Heteroaryl 4-(Pyridyl)-2,3,5,6-tetrafluorphenyl, 4-(N-Methyl-pyridylium)-2,3,5,6-tetrafluorphenyl, 4-(N-Propargyl-pyridylium)-2,3,5,6-tetrafluorphenyl, 2-(N-Propargyl-pyridyliuim)-2,3,5,6-tetrafluorphenyl ist.

8. Komplex nach Anspruch 7, wobei:
(i) Ar₁, Ar₂ und Ar₃ gleich sind und jeweils CF₃, Pentafluorphenyl, 4-Methoxy-2,3,5,6-tetrafluorphenyl, 4-Sulfo-phenyl, 4-(N-Methyl)-pyridylium, 2-(N-Methyl)-pyridylium, 4-(N-propargyl)-pyridylium oder 2-(N-Propargyl)-pyridylium sind;
(ii) Ar₁ und Ar₃ jeweils 4-(N-Methyl)-pyridylium sind und Ar₂ Pentafluorphenyl ist; oder Ar₁ und Ar₃ jeweils 4-N-Propargylamino-2,3,5,6-tetrafluorphenyl sind und Ar₂ 4-Methoxyphenyl ist; oder Ar₁ und Ar₃ jeweils 4-(N-Propargyl)-pyridylium sind und Ar₂ Pentafluorphenyl ist; oder Ar₁ und Ar₃ jeweils 2-(N-Propargyl)-pyridylium sind und Ar₂ Pentafluorphenyl ist; oder Ar₁ und Ar₃ jeweils 2-(N-Methyl)-pyridylium sind und Ar₂ 4-N-Propargylaminophenyl ist;oder
(iii) E₂ und E₁₇ gleich sind und jeweils H, SO₃H, SO₂NH-Propargyl oder SO₂NH-CH₂-COOH sind.

9. Komplex nach einem der Ansprüche 1-8, wobei das Metall M Fe oder Mn ist.

10. Komplex nach Anspruch 9, wobei genannter Corrol-Metallkomplex ausgewählt ist unter:
(i) Corrole, in denen E₂ und E₁₇ beide SO₃H sind, Ar₁, Ar₂ und Ar₃ jeweils Pentafluorphenyl sind und M Fe, hierin bezeichnet als Corrol **1C-FE**, Mn, hierin bezeichnet als Corrol **1C-Mn**, oder Cu, hierin bezeichnet als Corrol **1C-Cu**, ist oder Ar₁, Ar₂ und Ar₃ jeweils CF₃ sind und M Fe oder Mn ist.
(ii) Corrol, in dem E₂ und E₁₇ beide SO₃H sind, Ar₁, Ar₂ und Ar₃ jeweils 4-Methoxy-2,3,5,6-tetrafluorphenyl sind und M Fe ist, hierin bezeichnet als Corrol **6C-Fe**.
(iii) Corrol, in dem E₂ und E₁₇ beide SO₂NH-CH₂-COOH sind, Ar₁, Ar₂ und Ar₃ jeweils Pentafluorphenyl sind und M Fe ist, hierin bezeichnet als Corrol **5C-Fe**;
(iv) Corrol, in dem E₂ und E₁₇ beide H sind, Ar₁ und Ar₃ jeweils 4-(N-Methyl)-pyridylium sind und Ar₂ Pentafluorphenyl ist und M Mn ist, hierin bezeichnet als Corrol **2C-Mn**;
(v) Corrol, in dem E₂ und E₁₇ beide H sind, Ar₁, Ar₂ und Ar₃ jeweils 2-(N-Methyl)-pyridylium sind und M Mn ist, hierin bezeichnet als Corrol **3C-Mn**;
(vi) Corrol, in dem E₂ und E₁₇ beide H sind, Ar₁ und Ar₃ jeweils 2-(N-Methyl)-pyridylium sind, Ar₂ Pentafluorphenyl ist und M Mn ist, hierin bezeichnet als Corrol **4C-Mn**, und
(vii) Corrole, in denen E₂ und E₁₇ beide SO₃H sind, Ar₁, Ar₂ und Ar₃ jeweils CF₃ sind und M Mn oder Fe ist, hierin bezeichnet als Corrol **M-Mn** beziehungsweise **H-Fe;**
(viii) Corrole, in denen E₂ und E₁₇ beide H sind, Ar₁ und Ar₃ jeweils 4-(N-Propargyl)-pyridylium sind, Ar₂ Pentafluorphenyl ist und M Mn oder Fe ist, hierin bezeichnet als Corrol **E-pr-Mn** beziehungsweise **E-pr-Fe**;
(ix) Corrol, in dem E₂ und E₁₇ beide H sind, Ar₁ und Ar₃ jeweils 2-(N-Propargyl)-pyridylium sind, Ar₂ Pentafluorphenyl ist und M Mn oder Fe ist, hierin bezeichnet als Corrol **H-Mn** beziehungsweise **H-Fe**;
(x) Corrole, in denen E₂ und E₁₇ beide SO₃H sind, Ar₁ und Ar₃ jeweils 4-N-Propargylamino-2,3,5,6-tetrafluorphenyl sind und Ar₂ 4-Methoxyphenyl ist und M Mn oder Fe ist, hierin bezeichnet als Corrol **I-Mn** beziehungsweise **I-Fe**;
(xi) Corrol, in dem E₂ und E₁₇ beide H sind, Ar₁ und Ar₃ jeweils 2-(N-Methyl)-pyridylium sind, Ar₂ 4-Propargylamino-phenyl ist und M Mn oder Fe ist, hierin bezeichnet als Corrol **J-Mn** beziehungsweise **J-Fe**.
(xii) Corrole, in denen E₂ und E₁₇ beide -SO₂-NH-Propargyl sind, Ar₁, Ar₂ und Ar₃ jeweils Pentafluorphenyl sind und M Mn oder Fe ist, hierin bezeichnet als Corrol **K-Mn** beziehungsweise **K-Fe**, und
(xiii) Corrole, in denen E₂ und E₁₇ beide -SO₂-NH-Propargyl sind, Ar₁, Ar₂ und Ar₃ jeweils CF₃ sind und M Mn oder Fe ist, hierin bezeichnet als Corrol **M-Mn** beziehungsweise **M-Fe**.

11. Pharmazeutische Zusammensetzung zur Verwendung für Neuroprotektion und Neurorescue, insbesondere für die Behandlung von Diabetes und neurodegenerativen Erkrankungen oder Störungen oder neurodegenerativen Zuständen, umfassend einen pharmazeutisch anerkannten Trägerstoff und einen Metallkomplex mit einem amphiphilen Corrol, einem optischen Isomer oder einem pharmazeutisch anerkannten Salz davon, vorzugsweise nach Formel I, wie in Anspruch 4 dargestellt.

12. Propargyl enthaltendes Corrol, welches ein oder mehrere Radikal(e) trägt, substituiert durch eine Propargylaminogruppe oder ein oder mehrere Stickstoff enthaltende Heteroarylradikal(e), substituiert an dem N-Atom des Rings durch Propargyl.

13. Propargyl enthaltendes Corrol nach Anspruch 12, wobei es sich um einen Übergangsmetallkomplex mit einem amphiphilen Corrol mit der Formel I handelt, wie in Anspruch 4 definiert, wobei entweder: (i) mindestens ein Rest unter Ar₁, Ar₂ und Ar₃ ein Carboarylradikal ist, substituiert durch eine -NR₁R₂-Gruppe, wobei R₁ H ist und R₂ Propargyl ist, oder ein N-Heteroarylradikal an dem N-Atom des Rings durch Propargyl substituiert ist, oder (ii) mindestens ein Rest unter E₂ und E₁₇ -CONR₁R₂ oder -SO₂N-R₁R₂ ist, wobei R₁ H ist und R₂ Propargyl ist.

14. Propargyl enthaltendes Corrol nach Anspruch 13, wobei E₂ und E₁₇ jeweils SO₂N-R₁R₂ sind, wobei R₁ H ist und R₂ Propargyl ist, oder an Position 5, 10 und/oder 15 Ar₁, Ar₂ und/oder Ar₃ ein Phenylradikal sind, ausschließlich substituiert durch eine Propargylaminogruppe, vorzugsweise an Position 4, oder durch eine Propargylaminogruppe und andere Substituenten wie Halogen, vorzugsweise Fluor, oder Ar₁, Ar₂ und/oder Ar₃ ein Pyridylradikal sind, substituiert an dem N-Atom des Rings durch Propargyl.

15. Propargyl enthaltendes Corrol nach Anspruch 14, wobei die Propargyl enthaltenden Gruppen ausgewählt sind aus 4-(N-Propargyl)-pyridylium, 2-(N-Propoargyl)-pyridylium, 4-N-Propargylaminophenyl und 4-N-Propargylamino-2,3,5,6-tetrafluorphenyl an Positionen 5, 10 und/oder 15 des Corrols oder -SO₂-NH-Propargyl an Positionen 2 und 17 des Corrols und das Metall M Fe oder Mn ist.

16. Propargyl enthaltendes Corrol nach Anspruch 15, ausgewählt aus:
(a) Corrolen, in denen E₂ und E₁₇ beide H sind, Ar₁ und Ar₃ jeweils 4-(N-Propargyl)-pyridylium sind, Ar₂ Pentafluorphenyl ist und M Mn oder Fe ist, hierin bezeichnet als Corrol **E-pr-Mn** beziehungsweise **E-pr-Fe**.
(b) einem Corrol, in dem E₂ und E₁₇ beide H sind, Ar₁ und Ar₃ jeweils 2-(N-Propargyl)-pyridylium sind, Ar₂ Pentafluorphenyl ist und M Mn oder Fe ist, hierin bezeichnet als Corrol **H-Mn** beziehungsweise **H-Fe**.
(c) Corrolen, in denen E₂ und E₁₇ beide SO₃H sind, Ar₁ und Ar₃ jeweils 4-(N-Propargylamino-2,3,5,6-tetrafluorphenyl sind und Ar₂ 4-Methoxyphenyl ist und M Mn oder Fe ist, hierin bezeichnet als Corrol **I-Mn** beziehungsweise **I-Fe**.
(d) einem Corrol, in dem E₂ und E₁₇ beide H sind, Ar₁ und Ar₃ jeweils 2-(N-Methyl)-pyridylium sind, Ar₂ 4-Propargylamino-phenyl ist und M Mn oder Fe ist, hierin bezeichnet als Corrol **J-Mn** beziehungsweise **J-Fe**.
(e) Corrolen, in denen E₂ und E₁₇ beide -SO₂-NH-Propargyl sind, Ar₁, Ar₂ und Ar₃ jeweils Pentafluorphenyl sind, und M Mn oder Fe ist, hierin bezeichnet als Corrol **K-Mn** beziehungsweise **K-Fe**.
(f) Corrolen, in denen E₂ und E₁₇ beide -SO₂-NH-Propargyl sind, Ar₁, Ar₂ und Ar₃ jeweils CF₃ sind, und M Mn oder Fe ist, hierin bezeichnet als Corrol **M-Mn** beziehungsweise **M-Fe**.

17. Pharmazeutische Zusammensetzung, umfassend ein Propargyl enthaltendes Corrol nach einem der Ansprüche 12 bis 16, ein pharmazeutisch anerkanntes Salz oder ein optisches Isomer davon sowie einen pharmazeutisch anerkannten Trägerstoff.

18. Metallkomplexe mit amphiphilen Corrolen mit der Formel I, wie in Anspruch 4 definiert, wobei:
(i) E₂ und E₁₇ beide H sind, Ar₁, Ar₂ und Ar₃ jeweils 2-(N-Methyl)-pyridylium sind und M Mn ist, hierin bezeichnet als Corrol **3C-Mn**;
(ii) E₂ und E₁₇ beide H sind, Ar₁ und Ar₃ jeweils 2-(N-Methyl)-pyridylium sind, Ar₂ Pentafluorphenyl ist und M Mn ist, hierin bezeichnet als Corrol **4C-Mn**;
(iii) E₂ und E₁₇ beide SO₂NH-CH₂-COOH sind, Ar₁, Ar₂ und Ar₃ jeweils Pentafluorphenyl sind und M Fe ist, hierin bezeichnet als Corrol **5C-Fe**; oder
(iv) E₂ und E₁₇ beide SO₃H sind, Ar₁, Ar₂ und Ar₃ jeweils 4-Methoxy-2,3,5,6-tetrafluorphenyl sind und M Fe ist, hierin bezeichnet als Corrol **6C-Fe**.

## Revendications

1. Complexe de métal de transition d'une corolle amphiphile, isomère optique ou sel acceptable au plan pharmaceutique de celui-ci, pour une utilisation en neuroprotection et en sauvetage neuronal.

2. Complexe selon la revendication 1, pour le traitement du diabète ou d'une maladie, d'un trouble ou d'un état neurodégénératif.

3. Complexe selon la revendication 1 ou 2, dans lequel ladite corolle amphiphile est une 5,10,15-tris-aryl-corolle ou une 5,10,15-tris-CF₃-corolle.

4. Complexe selon la revendication 3, dans lequel ledit complexe de métal de transition de la corolle amphiphile présente la formule I : 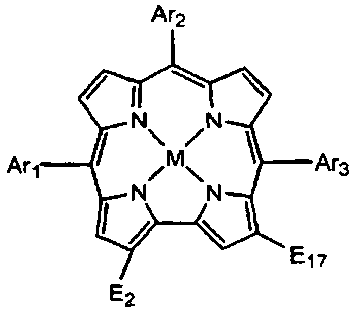 dans laquelle :
Ar₁, Ar₂ et Ar₃, identiques ou différents, sont chacun sélectionnés parmi les groupements CF₃, carboaryle, hétéroaryle ou carboaryle-hétéroaryle mixte ;
M est un métal de transition sélectionné parmi les éléments Mn, Fe, Ru, Co, V, Cr ou Cu ; et
E₂ et E₁₇, identiques ou différents, représentent chacun H, SO₃H, SO₂N-R₁R₂, CO₂H, CO₂R ou CON-R₁R₂ ; R représente un alkyle ou un aryle en C₁-C₈ ; et R₁ et R₂, identiques ou différents, représentent chacun H, un alkyle en C₁-C₈ éventuellement substitué par -COOH, un alcynyle en C₂-C₈, un aryle en C₆-C₁₂ ou forment, conjointement avec l'atome N auquel ils sont fixés, un cycle saturé de 5 à 6 éléments contenant éventuellement un autre hétéroatome sélectionné parmi O, S et N.

5. Complexe selon la revendication 4, dans lequel le carboaryle, en lui-même ou comme partie du radical de carboaryle-hétéroaryle mixte, est un radical aromatique en C₆-C₁₂ monocyclique ou bicyclique, tel qu'un groupement phényle, biphényle ou naphtyle éventuellement mono- ou poly-substitué par un ou plusieurs atomes d'halogène, ou par un alkyle ou un alcoxy en C₁-C₈, de préférence en C₁-C₄, par un nitro, un hydroxyle, un groupement SO₃H, -NR₁R₂, -N⁺R₁R₂R₃ ou -N-R₁-NH₂, où R₁, R₂ et R₃, identiques ou différents, représentent chacun H, un alkyle en C₁-C₈, un alcynyle en C₂-C₈, de préférence en C₃, un aryle en C₆-C₁₂, un aryle(C₆-C₁₂)-alkyle (C₁-C₈), ou R₁ et R₂ forment conjointement avec l'atome N auquel ils sont fixés, un cycle saturé de 5 à 6 éléments contenant éventuellement un autre hétéroatome sélectionné parmi O, S et N ; ledit hétéroaryle, en lui-même ou comme partie du radical de carboaryle-hétéroaryle mixte, est un cycle aromatique de 5 à 6 éléments contenant 1 à 3 hétéroatomes sélectionnés parmi 0, S et N, tel qu'un groupement pyrrolyle, furyle, thiényle, imidazolyle, pirazolyle, oxazolyle, thiazolyle, pyridyle, pirazinyle, pyrimidinyle, 1,3,4-triazinyle, 1,2,3-triazinyle, éventuellement substitué comme défini ci-dessus pour le carboaryle et, lorsque l'hétéroaryle présente un atome de N dans le cycle, il peut être substitué sur l'atome de N, de préférence, par un groupement alkyle ou alcynyle, de préférence un groupement propargyle ; et ledit carboaryle-hétéroaryle mixte est un radical dérivé d'un carboaryle et d'un radical hétéroaryle condensés l'un avec l'autre, tel qu'un groupement benzofuryle, isobenzofuryle, indolyle, benzimidazolyle, benzthiazolyle, benzoxazolyle, quinoléine ou isoquinoléine, ou sont liés mutuellement de manière covalente, tel qu'un groupement pyridilium-phényle, éventuellement substitué comme défini ci-dessus dans le cycle carboxylique ou hétérocyclique ou dans les deux.

6. Complexe selon la revendication 5, dans lequel le carboaryle est un radical de phényle monosubstitué par un groupement propargylamino ou méthoxy, de préférence sur la position 4, ou il est polysubstitué, par des atomes d'halogène, mieux encore par des atomes de chlore ou de fluor, par un groupement sulfo, propargylamino, alcoxy, de préférence, un groupement méthoxy, aminoalkylamino et trialkylammonium, et l'hétéroaryle est une pyridine substituée sur l'atome de N par un alkyle en C₁-C₄, de préférence un groupement méthyle ou propargyle.

7. Complexe selon la revendication 6, dans lequel le carboaryle est un groupement 2,6-dichlorophényle, 2,6-difluorophényle, pentafluorophényle, 4-méthoxy-2,3,5,6-tétrafluorophényle, 4-sulfo-phényle, 4-méthoxyphényle, 4-N-propargylamino-2,3,5,6-tétrafluorophényle ou 4-N-propargylaminophényle ; l'hétéroaryle est un groupement 4-(N-méthyl)-pyridylium, 2-(N-méthyl)-pyridylium, 4-(N-propargyl)-pyridylium ou 2-(N-propargyl)-pyridylium ; et le carboaryle-hétéroaryle est un groupement 4-(pyridyl)-2,3,5,6-tétrafluorophényle, 4-(N-méthyl-pyridylium)-2,3,5,6-tétrafluorophényle, 4-(N-propargyl-pyridylium)-2,3,5,6-tétrafluorophényle ou 2-(N-propargyl-pyridylium)-2,3,5,6-tétrafluorophényle.

8. Complexe selon la revendication 7, dans lequel :
(i) Ar₁, Ar₂ et Ar₃ sont identiques ou différents et représentent chacun un groupement CF₃, pentafluorophényle, 4-méthoxy-2,3,5,6-tétrafluorophényle, 4-sulfo-phényle, 4-(N-méthyl)-pyridylium, 2-(N-méthyl)-pyridylium, 4-(N-propargyl)-pyridylium ou 2-(N-propargyl)-pyridylium ;
(ii) Ar₁ et Ar₃ représentent chacun un groupement 4-(N-méthyl)-pyridylium et Ar₂ est un pentafluorophényle ; ou Ar₁ et Ar₃ représentent chacun un 4-N-propargylamino-2,3,5,6- tétrafluorophényle et Ar₂ est un 4-méthoxyphényle ; ou Ar₁ et Ar₃ représentent chacun un 4-(N-propargyl)-pyridylium et Ar₂ et un pentafluorophényle ; ou Ar₁ et Ar₃ représentent chacun un 2-(N-propargyl)-pyridylium et Ar₂ est un pentafluorophényle ; ou Ar₁ et Ar₃ représentent chacun un 2-(N-méthyl)-pyridylium et Ar₂ est un 4-N-propargylaminophényle ; ou
(iii) E₂ et E₁₇ sont identiques et représentent chacun H, un groupement SO₃H, SO₂NH-propargyle ou SO₂NH-CH₂-COOH.

9. Complexe selon l'une quelconque des revendications 1 à 8, dans lequel le métal M est l'élément Fe ou Mn.

10. Complexe selon la revendication 9, dans lequel ledit complexe de métal de corolle est sélectionné parmi :
(i) les corolles dans lesquelles E₂ et E₁₇ représentent tous deux SO₃H, Ar₁, Ar₂ et Ar₃ représentent chacun un pentafluorophényle et M est l'élément Fe, désignées ici par corolle 1C-Fe, Mn, désignées ici par corolle 1C-Mn, ou Cu, désignées ici par corolle 1C-Cu ; ou Ar₁, Ar₂ et Ar₃ représentent chacun CF₃ et M est l'élément Fe ou Mn ;
(ii) la corolle dans laquelle E₂ et E₁₇ représentent tous deux SO₃H, Ar₁, Ar₂ et Ar₃ représentent un 4-méthoxy-2,3,5,6,-tétrafluorophényle, et M représente l'élément Fe, désignée ici par corolle 6C-Fe ;
(iii) la corolle dans laquelle E₂ et E₁₇ représentent tous deux SO₂NH-CH₂-COOH, Ar₁, Ar₂ et Ar₃ représentent chacun un pentafluorophényle et M est l'élément Fe, désignée ici par corolle 5C-Fe ;
(iv) la corolle dans laquelle E₂ et E₁₇ représentent tous deux H, Ar₁ et Ar₃ représentent chacun un 4-(N-méthyl)-pyridylium, Ar₂ représente un pentafluorophényle et M représente l'élément Mn, désignée ici par corolle 2C-Mn ;
(v) la corolle dans laquelle E₂ et E₁₇ représentent tous deux H, Ar₁, Ar₂ et Ar₃ représentent chacun un 2-(N-méthyl)-pyridylium et M représente l'élément Mn, désignée ici par corolle 3C-Mn ;
(vi) la corolle dans laquelle E₂ et E₁₇ représentent tous deux H, Ar₁ et Ar₃ représentent chacun un 2-(N-méthyl)-pyridylium, Ar₂ représente un pentafluorophényle et M représente l'élément Mn, désignée ici par corolle 4C-Mn ; et
(vii) les corolles dans lesquelles E₂ et E₁₇ représentent tous deux SO₃H, Ar₁, Ar₂ et Ar₃ représentent chacun CF₃ et M représente les éléments Mn ou Fe, désignées ici par corolle M-Mn ou H-Fe, respectivement,
(viii) les corolles dans lesquelles E₂ et E₁₇ représentent tous deux H, Ar₁ et Ar₃ représentent chacun un 4-(N-propargyl)-pyridylium, Ar₂ représente un pentafluorophényle et M représente l'élément Mn ou Fe, désignées ici par corolle E-pr-Mn ou E-pr-Fe, respectivement ;
(ix) les corolles dans lesquelles E₂ et E₁₇ représentent tous deux H, Ar₁ et Ar₃ représentent chacun un 2-(N-propargyl)-pyridylium, Ar₂ représente un pentafluorophényle et M représente l'élément Mn ou Fe, désignées ici par corolle H-Mn ou H-Fe, respectivement ;
(x) les corolles dans lesquelles E₂ et E₁₇ représentent tous deux SO₃H, Ar₁ et Ar₃ représentent chacun un 4-N-propargylamino-2,3,5,6-tétrafluorophényle et Ar₂ représente un 4-méthoxyphényle, et M représente l'élément Mn ou Fe, désignées ici par corolle I-Mn ou I-Fe, respectivement ;
(xi) les corolles dans lesquelles E₂ et E₁₇ représentent tous deux H, Ar₁ et Ar₃ représentent chacun un 2-(N-méthyl)-pyridylium, Ar₂ représente un 4-propargylamino-phényle et M représente l'élément Mn ou Fe, désignées ici par corolle J-Mn ou J-Fe, respectivement ;
(xii) les corolles dans lesquelles E₂ et E₁₇ représentent tous deux un groupement -SO₂-NH-propargyle, Ar₁, Ar₂ et Ar₃ représentent chacun un pentafluorophényle et M représente l'élément Mn ou Fe, désignées ici par corolle K-Mn ou K-Fe, respectivement ; et
(xiii) les corolles dans lesquelles E₂ et E₁₇ représentent tous deux un groupement -SO₂-NH-propargyle, Ar₁, Ar₂ et Ar₃ représentent chacun CF₃, et M représente l'élément Mn ou Fe, désignées ici par corolle M-Mn ou M-Fe, respectivement.

11. Composition pharmaceutique pour une utilisation en neuroprotection et en sauvetage neuronal, en particulier pour le traitement du diabète et de maladies, de troubles et d'états neurodégénératifs, comprenant un véhicule acceptable au plan pharmaceutique et un complexe de métal d'une corolle amphiphile, un isomère optique ou un de ses sels acceptables au plan pharmaceutique, de préférence répondant à la formule I présentée dans la revendication 4.

12. Corolle contenant un groupement propargyle, portant un ou plusieurs radicaux substitués par un groupement propargylamino ou un ou plusieurs radicaux d'hétéroaryle contenant de l'azote substitués par un groupement propargyle sur l'atome N du cycle.

13. Corolle contenant un groupement propargyle selon la revendication 12, qui est un complexe de métal de transition d'une corolle amphiphile de formule I selon la revendication 4, dans laquelle : (i) au moins un des substituants Ar₁, Ar₂ et Ar₃ représente un radical de carboaryle substitué par un groupement -NR₁R₂, où R₁ représente H et R₂ représente un propargyle, ou un radical de N-hétéroaryle substitué sur l'atome N du cycle par un propargyle ; ou (ii) au moins un des substituants E₂ et E₁₇ représente un groupement -CONR₁R₂ ou -SO₂N-R₁R₂, où R₁ représente H et R₂ représente un propargyle.

14. Corolle contenant un groupement propargyle selon la revendication 13, dans laquelle E₂ et E₁₇ représentent chacun SO₂N-R₁R₂, où R₁ représente H et R₂ représente un propargyle, ou, sur les position 5, 10 et/ou 15, Ar₁, Ar₂ et/ou Ar₃ représentent un radical de phényle substitué seulement par un groupement propargylamino, de préférence sur la position 4, ou par un groupement propargylamino et par d'autres substituants, tels qu'un halogène, de préférence le fluor, ou Ar₁, Ar₂ et/ou Ar₃ représentent un radical de pyridyle substitué par un propargyle sur l'atome N du cycle.

15. Corolle contenant un groupement propargyle selon la revendication 14, dans laquelle les groupements contenant un propargyle sont sélectionnés parmi les groupements 4-(N-propargyl)-pyridylium, 2-(N-propargyl)-pyridylium, 4-N-propargylaminophényle et 4-N-propargylamino-2,3,5,6-tétrafluorophényle sur les positions 5, 10 et/ou 15 de la corolle ou -SO₂-NH-propargyle sur les positions 2 et 17 de la corolle, et le métal M représente l'élément Fe ou Mn.

16. Corolle contenant un groupement propargyle selon la revendication 15, sélectionnée parmi :
(a) les corolles dans lesquelles E₂ et E₁₇ représentent tous deux H, Ar₁ et Ar₃ représentent chacun un 4-(N-propargyl)-pyridylium, Ar₂ représente un pentafluorophényle et M représente l'élément Mn ou Fe, désignées ici par corolle E-pr-Mn ou E-pr-Fe, respectivement ;
(b) les corolles dans lesquelles E₂ et E₁₇ représentent tous deux H, Ar₁ et Ar₃ représentent chacun un 2-(N-propargyl)-pyridylium, Ar₂ représente un pentafluorophényle et M représente l'élément Mn ou Fe, désignées ici par corolle H-Mn ou H-Fe, respectivement ;
(c) les corolles dans lesquelles E₂ et E₁₇ représentent tous deux SO₃H, Ar₁ et Ar₃ représentent chacun un 4-N-propargylamino-2,3,5,6-tétrafluorophényle et Ar₂ représente un 4-méthoxyphényle, et M représente l'élément Mn ou Fe, désignées ici par corolle I-Mn ou I-Fe, respectivement ;
(d) les corolles dans lesquelles E₂ et E₁₇ représentent tous deux H, Ar₁ et Ar₃ représentent chacun un 2-(N-méthyle)-pyridylium, Ar₂ représente un 4-propargylamino-phényle et M représente l'élément Mn ou Fe, désignées ici par corolle J-Mn ou J-Fe, respectivement ;
(e) les corolles dans lesquelles E₂ et E₁₇ représentent tous deux un groupement -SO₂-NH-propargyle, Ar₁, Ar₂ et Ar₃ représentent chacun un pentafluorophényle et M représente l'élément Mn ou Fe, désignées ici par corolle K-Mn ou K-Fe, respectivement ; et
(f) les corolles dans lesquelles E₂ et E₁₇ représentent tous deux un groupement -SO₂-NH-propargyle, Ar₁, Ar₂ et Ar₃ représentent chacun CF₃, et M représente l'élément Mn ou Fe, désignées ici par corolle M-Mn ou M-Fe, respectivement.

17. Composition pharmaceutique comprenant une corolle contenant un groupement propargyle selon l'une quelconque des revendications 12 à 16, un sel acceptable au plan pharmaceutique ou l'un de ses isomères optiques et un véhicule acceptable au plan pharmaceutique.

18. Complexes de métaux de corolles amphiphiles de formule I, tels que définis dans la revendication 4, dans lesquels :
(i) E₂ et E₁₇ représentent tous deux H, Ar₁, Ar₂ et Ar₃ représentent chacun un 2-(N-méthyl)-pyridylium et M représente l'élément Mn, désignée ici par corolle 3C-Mn ;
(ii) E₂ et E₁₇ représentent tous deux H, Ar₁ et Ar₃ représentent chacun un 2-(N-méthyl)-pyridylium, Ar₂ représente un pentafluorophényle et M représente l'élément Mn, désignée ici par corolle 4C-Mn ;
(iii) E₂ et E₁₇ représentent tous deux un groupement SO₂NH-CH₂-COOH, Ar₁, Ar₂ et Ar₃ représentent chacun un pentafluorophényle et M est l'élément Fe, désignée ici par corolle 5C-Fe ; ou
(iv) E₂ et E₁₇ représentent tous deux un groupement SO₃H, Ar₁, Ar₂ et Ar₃ représentent un 4-méthoxy-2,3,5,6,-tétrafluorophényle, et M représente l'élément Fe, désignée ici par corolle 6C-Fe.
